# EUROPEAN PATENT SPECIFICATION

(11) **EP 1 644 050 B1**
(45) Date of publication and mention of the grant of the patent: **09.06.2010**
(21) Application number: 04759566.5
(22) Date of filing: 15.04.2004
(51) Int. Cl.: A61K 51/04, C07D 231/12, C07D 231/14, A61P 35/00

(54) **BIFUNCTIONAL TRIDENTATE PYRAZOLYL CONTAINING LIGANDS FOR RE, TC AND MN TRICARBONYL COMPLEXES**
BIFUNKTIONALE TRIDENTATE PYRAZOLYL-ENTHALTENDE LIGANDEN FÜR RE-, TC- UND MN- TRICARBONYL-KOMPLEXE
TRIDENTATE PYRAZOLYLE BIFONCTIONNEL CONTENANT DES LIGANDS POUR LES COMPLEXES RE, TC ET MN TRICARBONYLE

(30) Priority: 15.04.2003 EP 03076106; 10.10.2003 EP 03078217
(43) Date of publication of application: 12.04.2006
(73) Proprietor: MALLINCKRODT INC., St. Louis, Missouri 63134 (US)
(72) Inventor: SANTOS, Isabel, R., P-1800 Lisboa (PT); GALAMBA CORREIA, Joao, D., P-1070-373 Lisboa (PT); ROCHA PAULO, Antonio, M., P-1900 Lisboa (PT); ALVES, Susana, P-2800-310 Almada (PT); VITOR, Rute, P-2685 Santa Iria Da Azoia (PT)
(74) Representative: Jones, Nicholas Andrew
(86) International application number: PCT/US2004/011685
(87) International publication number: WO 2004/091669

(56) References cited:
- ALVES S ET AL: "COORDINATION CAPABILITES OF PYRAZOLYL CONTAINING LIGANDS TOWARDS THE FAC-ÄRE(CO)3Ü+ MOIETY" JOURNAL OF THE CHEMICAL SOCIETY, DALTON TRANSACTIONS, CHEMICAL SOCIETY. LETCHWORTH, GB, no. 24, 2002, pages 4714-4719, XP001179527 ISSN: 1472-7773
- VAN BERKEL P M ET AL: "Highly copper(II)-selective chelating ion-exchange resins based on bis(imidazole)-modified glycidyl methacrylate copolymers" REACTIVE & FUNCTIONAL POLYMERS, ELSEVIER SCIENCE PUBLISHERS BV, NL, vol. 32, no. 2, 1 February 1997 (1997-02-01), pages 139-151, XP004054255 ISSN: 1381-5148
- TANDON S S ET AL: "COPPER(II) COORDINATION CHEMISTRY OF POTENTIALLY OCTADENTATE (N8) TETRAPYRIDYL AND TETRAPYRAZOLYL-PYRIDAZINE LIGANDS. X-RAY CRYSTAL STRUCTURES OF ÄCU2(PTAPY)BR4Ü.2DMF AND ÄCU2(PTAPY)(NO3)2(N3)(H2O)Ü2(N O3)2.1.2CH3OH" INORGANICA CHIMICA ACTA, LAUSANNE, CH, vol. 213, no. 1/2, 1993, pages 289-300, XP001155491 ISSN: 0020-1693
- PAAP F ET AL: "TRANSITION METAL COMPLEXES OF LIGANDS CONTAINING 3,5-DIMETHYLPYRAZOLYL GROUPS AND THIOETHER FUNCTIONS. THE CRYSTAL AND MOLECULAR STRUCTURE OF (1,12-BIS(3,5-DIMETHYLPYRAZOL-1-YL)-2,11-D IAZA-5,8-DITHIADODECANE)NIC KEL(II)-BIS(TETRAFLUOROBORATE" INORGANICA CHIMICA ACTA, LAUSANNE, CH, vol. 150, no. 1, 1988, pages 57-64, XP001155492 ISSN: 0020-1693
- DATABASE CHEMABS [Online] CHEMICAL ABSTRACTS SERVICE, COLUMBUS, OHIO, US; 19 April 1986 (1986-04-19), DI VAIRA, MASSIMO ET AL: "Copper complexes with quadridentate bis(pyrazolyl)thioether amine and tris(pyrazolyl)amine ligands. Structural characterization of the complexes isothiocyanato[tris(2-pyrazolylethyl)amine - NN2N2'N2'']copper(II) diisothiocyanatocuprate(I) and {bis[2-(3',5'-dimethylpyrazolyl)ethyl](2-m ethylthioethyl)a" XP002271976 accession no. STN Database accession no. 104:140923 -& JOURNAL OF THE CHEMICAL SOCIETY, DALTON TRANSACTIONS: INORGANIC CHEMISTRY (1972-1999) , (11), 2327-32 CODEN: JCDTBI; ISSN: 0300-9246, 1985, XP009026349
- GARCIA-ANTON J ET AL: "SYNTHESIS OF NEW PDII COMPLEXES CONTAINING THIOETER-PYRAZOLE HEMILABILE LIGANDS - STRUCTURAL ANALYSIS BY 1H AND 13C NMR SPECTROSCOPY AND CRYSTAL STRUCTURES OF ÄPDCL2(BDDO)Ü AND ÄPD(BDDO)Ü(BF4)2 ÄBDDO = 1,8-BIS(3,5-DIMETHYL-1-PYRAZOLYL)-3,6-DITH IA OCTANEÜ" EUROPEAN JOURNAL OF INORGANIC CHEMISTRY, WILEY-VCH VERLAG, WEINHEIM, DE, 2002, pages 3319-3327, XP001155500 ISSN: 1434-1948
- ADHIKARY B ET AL: "COPPER(II) COMPLEXES OF N2S3 LIGANDS INVOLVING AROMATIC NITROGEN AND THIOETHER DONORS AND HAVING HIGH REDOX POTENTIALS" INORGANIC CHEMISTRY, AMERICAN CHEMICAL SOCIETY. EASTON, US, vol. 33, no. 7, 1994, pages 1376-1381, XP001155495 ISSN: 0020-1669
- ADDISON A W ET AL: "COPPER COMPLEXES OF SOME TETRADENTATE PYRAZOLYL AMINES" INORGANICA CHIMICA ACTA, LAUSANNE, CH, vol. 142, no. 1, 1988, pages 95-100, XP001155490 ISSN: 0020-1693

## Description

This invention lies in the field of radiopharmaceuticals and provides new chelating agents to link biomolecules and carbonyl moieties for labeling with technetium and rhenium. In particular the invention relates to bifunctional tridentate pyrazolyl-polyamines, pyrazolyl-aminothioethers, and pyrazolyl-aminophosphines which stabilize the moieties [M(CO)₃]⁺ (M = Re, Tc, Mn) and bind to biomolecules which accumulate in diseased tissues. The invention relates to the chelators as such, to chelators coupled to a biomolecule and to either of these complexed with carbonyl. In addition the invention relates to a kit for providing radiolabeled biomolecules and to the use of such radiolabeled molecules in diagnosis and therapy.

The diagnosis and therapy of cancer still needs a significant input from the chemical, radiochemical and pharmaceutical point of view. Tumour seeking compounds stable *in vitro* and *in vivo*, with high specific activity and specificity are still an important issue in the radiopharmaceutical field. Since the publication of international patents on [Re(CO)₃]⁺ and [Tc(CO)₃]⁺ [1] a significant interest has appeared in this oxidation state, which opens new perspectives on pharmaceutical and Nuclear Medicine fields. The search for new chelating agents is essential as they are determinant for the uptake of biological vectors. Several chelating agents have been described in patents [1, 2] and publications [3, 4, 5].

Alves et al. (J. Chem. Soc., (2002) 24, 4714) discloses a chelating agent of the pyrazolyl-thioetheramine type. Chelating agents of this type are also disclosed in Van Berkel et al. (Reactive & Functional Polymers, (1997) 32, 139). Chelating agents of the pyrazolyl-polythioether type are disclosed in Garcia-Anton et al. (Eur. J. Inorg. Chem., (2002) 3319) and Adhikary et al. (Inorg. Chem., (1994) 33, 1376). Chelating agents of the pyrazolyl-aminothioether type are described in Tandon et al. (Inorg. Chim. Acta, (1993) 213, 289), Paap et al. (Inorg. Chim. Acta, (1988) 150, 57) and Di Vaira et al. (Database Chemabs, 1986, STN no. 104:140923). In each of the latter three disclosures, however, the compounds have a group attached to the amino moiety which is unsuitable for attachment of a biomolecule. Addison et al. (Inorg. Chim. Acta, (1988) 142, 95) discloses a chelating agent of the pyrazolyl-polyamine type, but the amino moiety does not bear a spaced functional group suitable for modification, e.g. with a biomolecule.

It is the object of the present invention to enlarge the family of bifunctional chelating agents.

This is achieved by the invention by chelating agents of the general formula: 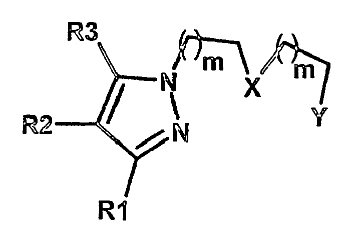 wherein m is 0 or 1;
X is NR₄ ;
Y is SR₅, NHR₅ or P(R₅)₂;
R₁ and R₃ are the same or different and are selected from H, alkyl or aryl;
R₂ is H, COOH, NHR₆ or (CH₂)ₙCOOR₆ ;
R₄ is (CH₂)ₙCOOR₆ or (CH₂)ₙOR₆ ;
R₅ is H, alkyl, aryl, (CH₂)ₙCOOR₆ or (CH₂)ₙOR₆
R₆ is H, alkyl or aryl, or a biomolecule; and n is 1, 2, 3, 4, 5, 6, 7, 8, 9 or 10.

These molecules combine two functions. One is for the stabilization of metal centers, including radioactive metals, and comprises different donor atom sets, and the other is a functional group for binding to the molecule of interest.

The alkyl may be a C₁ alkyl, C₂ alkyl, C₃ alkyl, C₄ alkyl, C₅ alkyl or C₆ alkyl, in particular selected from methyl, ethyl, *n*-propyl, isopropyl, *n*-butyl, isobutyl, *s-*butyl, *t*-butyl, *n*-pentyl, isopentyl, neopentyl, *n*-hexyl, isohexyl (2-methylpentyl), neohexyl (2,2-dimethylbutyl), 3-methylpentyl, 2,3- dimethylbutyl.

The aryls may be monocyclic, C₅-C₈, or polycyclic C₁₀-C₁₈, and are optionally substituted with alkyl, carboxy, oxo, amino, alkoxy or aldehyde groups.

n may be 2, 3, 4, 5 or 6 and preferably 2, 3 or 4.

The chelating agent is for example a pyrazolyl-polyamine of the general formula: 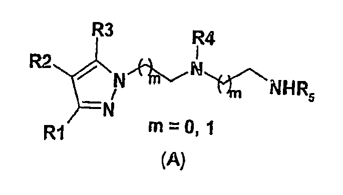 wherein R₁, R₂, R₃, R₄ and R₅ are as defined above.

Alternatively, the chelating agent is a pyrazolyl-aminothioether of the general formula: 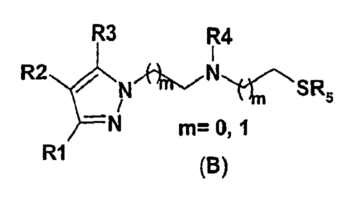 wherein R₁, R₂, R₃, R₄ and R₅ are as defined above.

In yet another embodiment the chelating agent is a pyrazolyl-aminophosphine of the general formula : 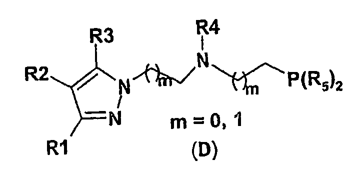 wherein R₁, R₂, R₃, R₄ and R₅ are as defined above.

The invention provides more particularly chelating agents of formula I, wherein X and Y are N, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₃, R₃, R₄ and R₅ are as listed in Table 1.

In yet another embodiment chelating agents of formula I are provided, wherein X is N and Y is S, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

According to another aspect of the invention, chelating agents of formula I are provided, wherein X is N and Y is P, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

The chelating agents of the invention are particularly suited to link biomolecules with carbonyl moieties in order to arrive at labeled biomolecules having a high specificity for the target. In formula I R₆ can thus be a biomolecule.

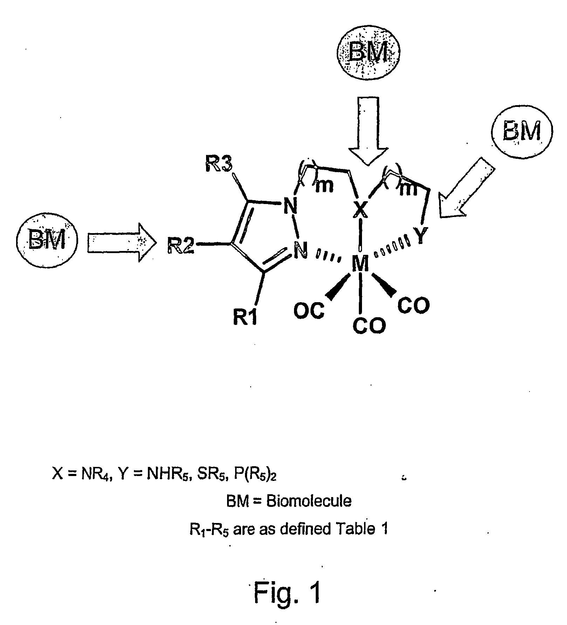

The possible positions of the biomolecules (BM) are shown in **Fig. 1****.**

The biomolecule can be anything that is useful in the treatment and diagnosis of tumors and can be coupled to the chelators of the invention. The skilled person will be able to establish for which biomolecules the chelators of the invention can be used. In particular the biomolecule is selected from amino acids, peptides, proteins, oligonucleotides, polynucleotides, sugars.

More specifically, the biomolecule is selected from the group consisting of antibodies, ligands of tumor receptors, such as CCK, thioglucose, glucosamine, somatostatin, neurotensin, bombesin, CCK, annexin, interleukins, growth factors, steroid hormones and molecules binding to GPIIb/IIIa receptors. Other biomolecules can be glucose, thioglucose, neurotransmitters, inhibitors of the tyrosine kinase activity such as benzothiopyranones, anilinophthalimides, quinazolines, pyridopyrimidines and pyrrolopyrimidines.

A particular agent of the invention is the following:

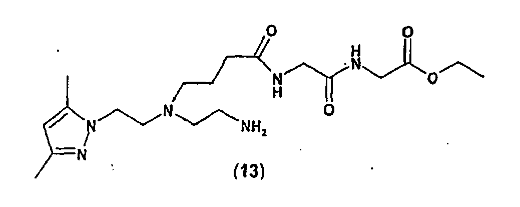

All of the chelating agents, either with or without a biomolecule coupled thereto can be complexed with a carbonyl moiety of the formula [M(CO)₃]⁺, wherein M is rhenium (Re), technetium (Tc) or Manganese (Mn).

The chelating agents of the invention are molecules according to formula I wherein X and Y can be either N and N, N and S, or N and P. Each of these combinations can be combined with various combinations of R₁, R₂, R₃, R₄ and R₅. All possible combinations of R₁, R₂, R₃, R₄ and R₅ are listed in **Table 1.** In **Table 1** alkyl is a C₁ alkyl, C₂ alkyl, C₃ alkyl, C₄ alkyl, C₅ alkyl or C₆ alkyl, in particular selected from methyl, ethyl, *n*-propyl, isopropyl, *n*-butyl, isobutyl, *s*-butyl, *t*-butyl, *n*-pentyl, isopentyl, neopentyl, *n*-hexyl, isohexyl (2-methylpentyl), neohexyl (2,2-dimethylbutyl), 3-methylpentyl, 2,3-dimethylbutyl; the aryl is monocyclic, C₅-C₈, or polycyclic, C₁₀-C₁₈, and optionally substituted with alkyl, carboxy, oxo, amino, alkoxy or aldehyde groups and is in particular phenyl or benzyl, and n is 1, 2, 3, 4, 5, 6, 7, 8, 9 or 10. R₆ is H, alkyl, aryl or a biomolecule as defined above. Substituting each of the above variables into the table will give all compounds of claim 1 that are herewith disclosed.

**Table 1**

| **R1** | **R2** | **R3** | **R4** | **R5** |
|---|---|---|---|---|
| H | H | H | (CH₂)ₙCOOR₆ | H |
| H | H | H | (CH₂)ₙCOOR₆ | alkyl |
| H | H | H | (CH₂)ₙCOOR₆ | aryl |
| H | H | H | (CH₂)ₙCOOR₆ | (CH₂)ₙCOOR₆ |
| H | H | H | (CH₂)ₙCOOR₆ | (CH₂)ₙOR₆ |
| H | H | H | (CH₂)ₙOR₆ | H |
| H | H | H | (CH₂)ₙOR₆ | alkyl |
| H | H | H | (CH₂)ₙOR₆ | aryl |
| H | H | H | (CH₂)ₙOR₆ | (CH₂)ₙCOOR₆ |
| H | H | H | (CH₂)ₙOR₆ | (CH₂)ₙOR₆ |
| H | H | alkyl | (CH₂)ₙCOOR₆ | H |
| H | H | alkyl | (CH₂)ₙCOOR₆ | alkyl |
| H | H | alkyl | (CH₂)ₙCOOR₆ | aryl |
| H | H | alkyl | (CH₂)ₙCOOR₆ | (CH₂)ₙCOOR₆ |
| H | H | alkyl | (CH₂)ₙCOOR₆ | (CH₂)ₙOR₆ |
| H | H | alkyl | (CH₂)ₙOR₆ | H |
| H | H | alkyl | (CH₂)ₙOR₆ | alkyl |
| H | H | alkyl | (CH₂)ₙOR₆ | aryl |
| H | H | alkyl | (CH₂)ₙOR₆ | (CH₂)ₙCOOR₆ |
| H | H | alkyl | (CH₂)ₙOR₆ | (CH₂)ₙOR₆ |
| H | H | aryl | (CH₂)ₙCOOR₆ | H |
| H | H | aryl | (CH₂)ₙCOOR₆ | alkyl |
| H | H | aryl | (CH₂)ₙCOOR₆ | aryl |
| H | H | aryl | (CH₂)ₙCOOR₆ | (CH₂)ₙCOOR₆ |
| H | H | aryl | (CH₂)ₙCOOR₆ | (CH₂)ₙOR₆ |
| H | H | aryl | (CH₂)ₙOR₆ | H |
| H | H | aryl | (CH₂)ₙOR₆ | alkyl |
| H | H | aryl | (CH₂)ₙOR₆ | aryl |
| H | H | aryl | (CH₂)ₙOR₆ | (CH₂)ₙCOOR₆ |
| H | H | aryl | (CH₂)ₙOR₆ | (CH₂)ₙOR₆ |
| H | COOH | H | (CH₂)ₙCOOR₆ | H |
| H | COOH | H | (CH₂)ₙCOOR₆ | alkyl |
| H | COOH | H | (CH₂)ₙCOOR₆ | aryl |
| H | COOH | H | (CH₂)ₙCOOR₆ | (CH₂)ₙCOOR₆ |
| H | COOH | H | (CH₂)ₙCOOR₆ | (CH₂)ₙOR₆ |
| H | COOH | H | (CH₂)ₙOR₆ | H |
| H | COOH | H | (CH₂)ₙOR₆ | alkyl |
| H | COOH | H | (CH₂)ₙOR₆ | aryl |
| H | COOH | H | (CH₂)ₙOR₆ | (CH₂)ₙCOOR₆ |
| H | COOH | H | (CH₂)ₙOR₆ | (CH₂)ₙOR₆ |
| H | COOH | alkyl | (CH₂)ₙCOOR₆ | H |
| H | COOH | alkyl | (CH₂)ₙCOOR₆ | alkyl |
| H | COOH | alkyl | (CH₂)ₙCOOR₆ | aryl |
| H | COOH | alkyl | (CH₂)ₙCOOR₆ | (CH₂)ₙCOOR₆ |
| H | COOH | alkyl | (CH₂)ₙCOOR₆ | (CH₂)ₙOR₆ |
| H | COOH | alkyl | (CH₂)ₙOR₆ | H |
| H | COOH | alkyl | (CH₂)ₙOR₆ | alkyl |
| H | COOH | alkyl | (CH₂)ₙOR₆ | aryl |
| H | COOH | alkyl | (CH₂)ₙOR₆ | (CH₂)ₙCOOR₆ |
| H | COOH | alkyl | (CH₂)ₙOR₆ | (CH₂)ₙOR₆ |
| H | COOH | aryl | (CH₂)ₙCOOR₆ | H |
| H | COOH | aryl | (CH₂)ₙCOOR₆ | alkyl |
| H | COOH | aryl | (CH₂)ₙCOOR₆ | aryl |
| H | COOH | aryl | (CH₂)ₙCOOR₆ | (CH₂)ₙCOOR₆ |
| H | COOH | aryl | (CH₂)ₙCOOR₆ | (CH₂)ₙOR₆ |
| H | COOH | aryl | (CH₂)ₙOR₆ | H |
| H | COOH | aryl | (CH₂)ₙOR₆ | alkyl |
| H | COOH | aryl | (CH₂)ₙOR₆ | aryl |
| H | COOH | aryl | (CH₂)ₙOR₆ | (CH₂)ₙCOOR₆ |
| H | COOH | aryl | (CH₂)ₙOR₆ | (CH₂)ₙOR₆ |
| H | NHR₆ | H | (CH₂)ₙCOOR₆ | H |
| H | NHR₆ | H | (CH₂)ₙCOOR₆ | alkyl |
| H | NHR₆ | H | (CH₂)ₙCOOR₆ | aryl |
| H | NHR₆ | H | (CH₂)ₙCOOR₆ | (CH₂)ₙCOOR₆ |
| H | NHR₆ | H | (CH₂)ₙCOOR₆ | (CH₂)ₙOR₆ |
| H | NHR₆ | H | (CH₂)ₙOR₆ | H |
| H | NHR₆ | H | (CH₂)ₙOR₆ | alkyl |
| H | NHR₆ | H | (CH₂)ₙOR₆ | aryl |
| H | NHR₆ | H | (CH₂)ₙOR₆ | (CH₂)ₙCOOR₆ |
| H | NHR₆ | H | (CH₂)ₙOR₆ | (CH₂)ₙOR₆ |
| H | NHR₆ | alkyl | (CH₂)ₙCOOR₆ | H |
| H | NHR₆ | alkyl | (CH₂)ₙCOOR₆ | alkyl |
| H | NHR₆ | alkyl | (CH₂)ₙCOOR₆ | aryl |
| H | NHR₆ | alkyl | (CH₂)ₙCOOR₆ | (CH₂)ₙCOOR₆ |
| H | NHR₆ | alkyl | (CH₂)ₙCOOR₆ | (CH₂)ₙOR₆ |
| H | NHR₆ | alkyl | (CH₂)ₙOR₆ | H |
| H | NHR₆ | alkyl | (CH₂)ₙOR₆ | alkyl |
| H | NHR₆ | alkyl | (CH₂)ₙOR₆ | aryl |
| H | NHR₆ | alkyl | (CH₂)ₙOR₆ | (CH₂)ₙCOOR₆ |
| H | NHR₆ | alkyl | (CH₂)ₙOR₆ | (CH₂)ₙOR₆ |
| H | NHR₆ | aryl | (CH₂)ₙCOOR₆ | H |
| H | NHR₆ | aryl | (CH₂)ₙCOOR₆ | alkyl |
| H | NHR₆ | aryl | (CH₂)ₙCOOR₆ | aryl |
| H | NHR₆ | aryl | (CH₂)ₙCOOR₆ | (CH₂)ₙCOOR₆ |
| H | NHR₆ | aryl | (CH₂)ₙCOOR₆ | (CH₂)ₙOR₆ |
| H | NHR₆ | aryl | (CH₂)ₙOR₆ | H |
| H | NHR₆ | aryl | (CH₂)ₙOR₆ | alkyl |
| H | NHR₆ | aryl | (CH₂)ₙOR₆ | aryl |
| H | NHR₆ | aryl | (CH₂)ₙOR₆ | (CH₂)ₙCOOR₆ |
| H | NHR₆ | aryl | (CH₂)ₙOR₆ | (CH₂)ₙOR₆ |
| H | (CH₂)ₙCOOR₆ | H | (CH₂)ₙCOOR₆ | H |
| H | (CH₂)ₙCOOR₆ | H | (CH₂)ₙCOOR₆ | alkyl |
| H | (CH₂)ₙCOOR₆ | H | (CH₂)ₙCOOR₆ | aryl |
| H | (CH₂) ₙCOOR₆ | H | (CH₂)ₙCOOR₆ | (CH₂) ₙCOOR₆ |
| H | (CH₂)ₙCOOR₆ | H | (CH₂)ₙCOOR₆ | (CH₂)ₙOR₆ |
| H | (CH₂)ₙCOOR₆ | H | (CH₂)ₙOR₆ | H |
| H | (CH₂)ₙCOOR₆ | H | (CH₂)ₙOR₆ | alkyl |
| H | (CH₂)ₙCOOR₆ | H | (CH₂)ₙOR₆ | aryl |
| H | (CH₂)ₙCOOR₆ | H | (CH₂)ₙOR₆ | (CH₂)ₙCOOR₆ |
| H | (CH₂)ₙCOOR₆ | H | (CH₂)ₙOR₆ | (CH₂)ₙOR₆ |
| H | (CH₂)ₙCOOR₆ | alkyl | (CH₂)ₙCOOR₆ | H |
| H | (CH₂) ₙCOOR₆ | alkyl | (CH₂)ₙCOOR₆ | alkyl |
| H | (CH₂)ₙCOOR₆ | alkyl | (CH₂)ₙCOOR₆ | aryl |
| H | (CH₂)ₙCOOR₆ | alkyl | (CH₂)ₙCOOR₆ | (CH₂)ₙCOOR₆ |
| H | (CH₂)ₙCOOR₆ | alkyl | (CH₂)ₙCOOR₆ | (CH₂)ₙOR₆ |
| H | (CH₂)ₙCOOR₆ | alkyl | (CH₂)ₙOR₆ | H |
| H | (CH₂)ₙCOOR₆ | alkyl | (CH₂)ₙOR₆ | alkyl |
| H | (CH₂)ₙCOOR₆ | alkyl | (CH₂)ₙOR₆ | aryl |
| H | (CH₂)ₙCOOR₆ | alkyl | (CH₂)ₙOR_{b} | (CH₂)ₙCOOR₆ |
| H | (CH₂)ₙCOOR₆ | alkyl | (CH₂)ₙOR₆ | (CH₂)ₙOR₆ |
| H | (CH₂)ₙCOOR₆ | aryl | (CH₂)ₙCOOR₆ | H |
| H | (CH₂)ₙCOOR₆ | aryl | (CH₂)ₙCOOR₆ | alkyl |
| H | (CH₂)ₙCOOR₆ | aryl | (CH₂)ₙCOOR₆ | aryl |
| H | (CH₂)ₙCOOR₆ | aryl | (CH₂)ₙCOOR₆ | (CH₂)ₙCOOR₆ |
| H | (CH₂)ₙCOOR₆ | aryl | (CH₂)ₙCOOR₆ | (CH₂)ₙOR₆ |
| H | (CH₂)ₙCOOR₆ | aryl | (CH₂)ₙOR₆ | H |
| H | (CH₂)ₙCOOR₆ | aryl | (CH₂)ₙOR₆ | alkyl |
| H | (CH₂)ₙCOOR₆ | aryl | (CH₂)ₙOR₆ | aryl |
| H | (CH₂)ₙCOOR₆ | aryl | (CH₂)ₙOR₆ | (CH₂)ₙCOOR₆ |
| H | (CH₂)ₙCOOR₆ | aryl | (CH₂)ₙOR₆ | (CH₂)ₙOR₆ |
| alkyl | H | H | (CH₂)ₙCOOR₆ | H |
| alkyl | H | H | (CH₂)ₙCOOR₆ | alkyl |
| alkyl | H | H | (CH₂)ₙCOOR₆ | aryl |
| alkyl | H | H | (CH₂)ₙCOOR₆ | (CH₂)ₙCOOR₆ |
| alkyl | H | H | (CH₂)ₙCOOR₆ | (CH₂)ₙOR₆ |
| alkyl | H | H | (CH₂)ₙOR₆ | H |
| alkyl | H | H | (CH₂)ₙOR₆ | alkyl |
| alkyl | H | H | (CH₂)ₙOR₆ | aryl |
| alkyl | H | H | (CH₂)ₙOR₆ | (CH₂)ₙCOOR₆ |
| alkyl | H | H | (CH₂)ₙOR₆ | (CH₂)ₙOR₆ |
| alkyl | H | alkyl | (CH₂)ₙCOOR₆ | H |
| alkyl | H | alkyl | (CH₂)ₙCOOR₆ | alkyl |
| alkyl | H | alkyl | (CH₂)ₙCOOR₆ | aryl |
| alkyl | H | alkyl | (CH₂)ₙCOOR₆ | (CH₂)ₙCOOR₆ |
| alkyl | H | alkyl | (CH₂)ₙCOOR₆ | (CH₂)ₙOR₆ |
| alkyl | H | alkyl | (CH₂)ₙOR₆ | H |
| alkyl | H | alkyl | (CH₂)ₙOR₆ | alkyl |
| alkyl | H | alkyl | (CH₂)ₙOR₆ | aryl |
| alkyl | H | alkyl | (CH₂) ₙOR₆ | (CH₂) ₙCOOR₆ |
| alkyl | H | alkyl | (CH₂)ₙOR₆ | (CH₂)ₙOR₆ |
| alkyl | H | aryl | (CH₂)ₙCOOR₆ | H |
| alkyl | H | aryl | (CH₂)ₙCOOR₆ | alkyl |
| alkyl | H | aryl | (CH₂)ₙCOOR₆ | aryl |
| alkyl | H | aryl | (CH₂)ₙCOOR₆ | (CH₂)ₙCOOR₆ |
| alkyl | H | aryl | (CH₂)ₙCOOR₆ | (CH₂)ₙOR₆ |
| alkyl | H | aryl | (CH₂)ₙOR₆ | H |
| alkyl | H | aryl | (CH₂)ₙOR₆ | alkyl |
| alkyl | H | aryl | (CH₂)ₙOR₆ | aryl |
| alkyl | H | aryl | (CH₂)ₙOR₆ | (CH₂)ₙCOOR₆ |
| alkyl | H | aryl | (CH₂)ₙOR₆ | (CH₂)ₙOR₆ |
| alkyl | COOH | H | (CH₂)ₙCOOR₆ | H |
| alkyl | COOH | H | (CH₂)ₙCOOR₆ | alkyl |
| alkyl | COOH | H | (CH₂)ₙCOOR₆ | aryl |
| alkyl | COOH | H | (CH₂)ₙCOOR₆ | (CH₂)ₙCOOR₆ |
| alkyl | COOH | H | (CH₂)ₙCOOR₆ | (CH₂)ₙOR₆ |
| alkyl | COOH | H | (CH₂)ₙOR₆ | H |
| alkyl | COOH | H | (CH₂)ₙOR₆ | alkyl |
| alkyl | COOH | H | (CH₂)ₙOR₆ | aryl |
| alkyl | COOH | H | (CH₂)ₙOR₆ | (CH₂)ₙCOOR₆ |
| alkyl | COOH | H | (CH₂)ₙOR₆ | (CH₂)ₙOR₆ |
| alkyl | COOH | alkyl | (CH₂)ₙCOOR₆ | H |
| alkyl | COOH | alkyl | (CH₂)ₙCOOR₆ | alkyl |
| alkyl | COOH | alkyl | (CH₂)ₙCOOR₆ | aryl |
| alkyl | COOH | alkyl | (CH₂)ₙCOOR₆ | (CH₂)ₙCOOR₆ |
| alkyl | COOH | alkyl | (CH₂)ₙCOOR₆ | (CH₂)ₙOR₆ |
| alkyl | COOH | alkyl | (CH₂)ₙOR₆ | H |
| alkyl | COOH | alkyl | (CH₂)ₙOR₆ | alkyl |
| alkyl | COOH | alkyl | (CH₂)ₙOR₆ | aryl |
| alkyl | COOH | alkyl | (CH₂)ₙOR₆ | (CH₂)ₙCOOR₆ |
| alkyl | COOH | alkyl | (CH₂)ₙOR₆ | (CH₂)ₙOR₆ |
| alkyl | COOH | aryl | (CH₂)ₙCOOR₆ | H |
| alkyl | COOH | aryl | (CH₂)ₙCOOR₆ | alkyl |
| alkyl | COOH | aryl | (CH₂)ₙCOOR₆ | aryl |
| alkyl | COOH | aryl | (CH₂)ₙCOOR₆ | (CH₂)ₙCOOR₆ |
| alkyl | COOH | aryl | (CH₂)ₙCOOR₆ | (CH₂)ₙOR₆ |
| alkyl | COOH | aryl | (CH₂)ₙOR₆ | H |
| alkyl | COOH | aryl | (CH₂)ₙOR₆ | alkyl |
| alkyl | COOH | aryl | (CH₂)ₙOR₆ | aryl |
| alkyl | COOH | aryl | (CH₂)ₙOR₆ | (CH₂)ₙCOOR₆ |
| alkyl | COOH | aryl | (CH₂)ₙOR₆ | (CH₂)ₙOR₆ |
| alkyl | NHR₆ | H | (CH₂)ₙCOOR₆ | H |
| alkyl | NHR₆ | H | (CH₂)ₙCOOR₆ | alkyl |
| alkyl | NHR₆ | H | (CH₂)ₙCOOR₆ | aryl |
| alkyl | NHR₆ | H | (CH₂)ₙCOOR₆ | (CH₂)ₙCOOR₆ |
| alkyl | NHR₆ | H | (CH₂)ₙCOOR₆ | (CH₂)ₙOR₆ |
| alkyl | NHR₆ | H | (CH₂)ₙOR₆ | H |
| alkyl | NHR₆ | H | (CH₂)ₙOR₆ | alkyl |
| alkyl | NHR₆ | H | (CH₂)ₙOR₆ | aryl |
| alkyl | NHR₆ | H | (CH₂)ₙOR₆ | (CH₂)ₙCOOR₆ |
| alkyl | NHR₆ | H | (CH₂)ₙOR₆ | (CH₂)ₙOR₆ |
| alkyl | NHR₆ | alkyl | (CH₂)ₙCOOR₆ | H |
| alkyl | NHR₆ | alkyl | (CH₂)ₙCOOR₆ | alkyl |
| alkyl | NHR₆ | alkyl | (CH₂)ₙCOOR₆ | aryl |
| alkyl | NHR₆ | alkyl | (CH₂)ₙCOOR₆ | (CH₂)ₙCOOR₆ |
| alkyl | NHR₆ | alkyl | (CH₂)ₙCOOR₆ | (CH₂)ₙOR₆ |
| alkyl | NHR₆ | alkyl | (CH₂)ₙOR₆ | H |
| alkyl | NHR₆ | alkyl | (CH₂)ₙOR₆ | alkyl |
| alkyl | NHR₆ | alkyl | (CH₂)ₙOR₆ | aryl |
| alkyl | NHR₆ | alkyl | (CH₂)ₙOR₆ | (CH₂)ₙCOOR₆ |
| alkyl | NHR₆ | alkyl | (CH₂)ₙOR₆ | (CH₂)ₙOR₆ |
| alkyl | NHR₆ | aryl | (CH₂)ₙCOOR₆ | H |
| alkyl | NHR₆ | aryl | (CH₂)ₙCOOR₆ | alkyl |
| alkyl | NHR₆ | aryl | (CH₂)ₙCOOR₆ | aryl |
| alkyl | NHR₆ | aryl | (CH₂)ₙCOOR₆ | (CH₂)ₙCOOR₆ |
| alkyl | NHR₆ | aryl | (CH₂)ₙCOOR₆ | (CH₂)ₙOR₆ |
| alkyl | NHR₆ | aryl | (CH₂)ₙOR₆ | H |
| alkyl | NHR₆ | aryl | (CH₂)ₙOR₆ | alkyl |
| alkyl | NHR₆ | aryl | (CH₂)ₙOR₆ | aryl |
| alkyl | NHR₆ | aryl | (CH₂)ₙOR₆ | (CH₂)ₙCOOR₆ |
| alkyl | NHR₆ | aryl | (CH₂)ₙOR₆ | (CH₂)ₙOR₆ |
| alkyl | (CH₂) ₙCOOR₆ | H | (CH₂)ₙCOOR₆ | H |
| alkyl | (CH₂)ₙCOOR₆ | H | (CH₂)ₙCOOR₆ | alkyl |
| alkyl | (CH₂)ₙCOOR₆ | H | (CH₂)ₙCOOR₆ | aryl |
| alkyl | (CH₂)ₙCOOR₆ | H | (CH₂)ₙCOOR₆ | (CH₂)ₙCOOR₆ |
| alkyl | (CH₂)ₙCOOR₆ | H | (CH₂)ₙCOOR₆ | (CH₂)ₙOR₆ |
| alkyl | (CH₂)ₙCOOR₆ | H | (CH₂)ₙOR₆ | H |
| alkyl | (CH₂)ₙCOOR₆ | H | (CH₂)ₙOR₆ | alkyl |
| alkyl | (CH₂)ₙCOOR₆ | H | (CH₂)ₙOR₆ | aryl |
| alkyl | (CH₂)ₙCOOR₆ | H | (CH₂)ₙOR₆ | (CH₂)ₙCOOR₆ |
| alkyl | (CH₂)ₙCOOR₆ | H | (CH₂)ₙOR₆ | (CH₂)ₙOR₆ |
| alkyl | (CH₂)ₙCOOR₆ | alkyl | (CH₂)ₙCOOR₆ | H |
| alkyl | (CH₂)ₙCOOR₆ | alkyl | (CH₂)ₙCOOR₆ | alkyl |
| alkyl | (CH₂)ₙCOOR₆ | alkyl | (CH₂)ₙCOOR₆ | aryl |
| alkyl | (CH₂)ₙCOOR₆ | alkyl | (CH₂)ₙCOOR₆ | (CH₂)ₙCOOR₆ |
| alkyl | (CH₂)ₙCOOR₆ | alkyl | (CH₂)ₙCOOR₆ | (CH₂)ₙOR₆ |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | alkyl |
| alkyl | (CH₂)ₙCOOR₆ | alkyl | (CH₂)ₙOR₆ | aryl |
| alkyl | (CH₂)ₙCOOR₆ | alkyl | (CH₂)ₙOR₆ | (CH₂)ₙCOOR₆ |
| alkyl | (CH₂) ₙCOOR₆ | alkyl | (CH₂)ₙCOR₆ | (CH₂)ₙOR₆ |
| alkyl | (CH₂)ₙCOOR₆ | aryl | (CH₂)ₙCOOR₆ | H |
| alkyl | (CH₂)ₙCOOR₆ | aryl | (CH₂)ₙCOOR₆ | alkyl |
| alkyl | (CH₂)ₙCOOR₆ | aryl | (CH₂)ₙCOOR₆ | aryl |
| alkyl | (CH₂)ₙCOOR₆ | aryl | (CH₂)ₙCOOR₆ | (CH₂)ₙCOOR₆ |
| alkyl | (CH₂)ₙCOOR₆ | aryl | (CH₂)ₙCOOR₆ | (CH₂)ₙOR₆ |
| alkyl | (CH₂)ₙCOOR₆ | aryl | (CH₂)ₙOR₆ | H |
| alkyl | (CH₂)ₙCOOR₆ | aryl | (CH₂)ₙOR₆ | alkyl |
| alkyl | (CH₂) ₙCOOR₆ | aryl | (CH₂)ₙOR₆ | aryl |
| alkyl | (CH₂)ₙCOOR₆ | aryl | (CH₂)ₙOR₆ | (CH₂)ₙCOOR₆ |
| alkyl | (CH₂)ₙCOOR₆ | aryl | (CH₂)ₙOR₆ | (CH₂)ₙOR₆ |
| aryl | H | H | (CH₂)ₙCOOR₆ | H |
| aryl | H | H | (CH₂)ₙCOOR₆ | alkyl |
| aryl | H | H | (CH₂) ₙCOOR₆ | aryl |
| aryl | H | H | (CH₂)ₙCOOR₆ | (CH₂)ₙCOOR₆ |
| aryl | H | H | (CH₂)ₙCOOR₆ | (CH₂)ₙOR₆ |
| aryl | H | H | (CH₂)ₙOR₆ | H |
| aryl | H | H | (CH₂)ₙOR₆ | alkyl |
| aryl | H | H | (CH₂)ₙOR₆ | aryl |
| aryl | H | H | (CH₂)ₙOR₆ | (CH₂)ₙCOOR₆ |
| aryl | H | H | (CH₂)ₙOR₆ | (CH₂)ₙOR₆ |
| aryl | H | alkyl | (CH₂)ₙCOOR₆ | H |
| aryl | H | alkyl | (CH₂)ₙCOOR₆ | alkyl |
| aryl | H | alkyl | (CH₂)ₙCOOR₆ | aryl |
| aryl | H | alkyl | (CH₂)ₙCOOR₆ | (CH₂)ₙCOOR₆ |
| aryl | H | alkyl | (CH₂)ₙCOOR₆ | (CH₂)ₙOR₆ |
| aryl | H | alkyl | (CH₂)ₙOR₆ | H |
| aryl | H | alkyl | (CH₂)ₙOR₆ | alkyl |
| aryl | H | alkyl | (CH₂)ₙOR₆ | aryl |
| aryl | H | alkyl | (CH₂)ₙOR₆ | (CH₂)ₙCOOR₆ |
| aryl | H | alkyl | (CH₂)ₙOR₆ | (CH₂)ₙOR₆ |
| aryl | H | aryl | (CH₂)ₙCOOR₆ | H |
| aryl | H | aryl | (CH₂) ₙCOOR₆ | alkyl |
| aryl | H | aryl | (CH₂)ₙCOOR₆ | aryl |
| aryl | H | aryl | (CH₂)ₙCOOR₆ | (CH₂)ₙCOOR₆ |
| aryl | H | aryl | (CH₂)ₙCOOR₆ | (CH₂)ₙOR₆ |
| aryl | H | aryl | (CH₂)ₙOR₆ | H |
| aryl | H | aryl | (CH₂)ₙOR₆ | alkyl |
| aryl | H | aryl | (CH₂)ₙOR₆ | aryl |
| aryl | H | aryl | (CH₂)ₙOR₆ | (CH₂)ₙCOOR₆ |
| aryl | H | aryl | (CH₂)ₙOR₆ | (CH₂)ₙOR₆ |
| aryl | COOH | H | (CH₂)ₙCOOR₆ | H |
| aryl | COOH | H | (CH₂)ₙCOOR₆ | alkyl |
| aryl | COOH | H | (CH₂)ₙCOOR₆ | aryl |
| aryl | COOH | H | (CH₂)ₙCOOR₆ | (CH₂)ₙCOOR₆ |
| aryl | COOH | H | (CH₂)ₙCOOR₆ | (CH₂)ₙOR₆ |
| aryl | COOH | H | (CH₂)ₙOR₆ | H |
| aryl | COOH | H | (CH₂)ₙOR₆ | alkyl |
| aryl | COOH | H | (CH₂)ₙOR₆ | aryl |
| aryl | COOH | H | (CH₂)ₙOR₆ | (CH₂)ₙCOOR₆ |
| aryl | COOH | H | (CH₂)ₙOR₆ | (CH₂)ₙOR₆ |
| aryl | COOH | alkyl | (CH₂)ₙCOOR₆ | H |
| aryl | COOH | alkyl | (CH₂)ₙCOOR₆ | alkyl |
| aryl | COOH | alkyl | (CH₂)ₙCOOR₆ | aryl |
| aryl | COOH | alkyl | (CH₂)ₙCOOR₆ | (CH₂)ₙCOOR₆ |
| aryl | COOH | alkyl | (CH₂)ₙCOOR₆ | (CH₂) ₙOR₆ |
| aryl | COOH | alkyl | (CH₂)ₙOR₆ | H |
| aryl | COOH | alkyl | (CH₂)ₙOR₆ | alkyl |
| aryl | COOH | alkyl | (CH₂)ₙOR₆ | aryl |
| aryl | COOH | alkyl | (CH₂)ₙOR₆ | (CH₂)ₙCOOR₆ |
| aryl | COOH | alkyl | (CH₂)ₙOR₆ | (CH₂)ₙOR₆ |
| aryl | COOH | aryl | (CH₂)ₙCOOR₆ | H |
| aryl | COOH | aryl | (CH₂)ₙCOOR₆ | alkyl |
| aryl | COOH | aryl | (CH₂)ₙCOOR₆ | aryl |
| aryl | COOH | aryl | (CH₂)ₙCOOR₆ | (CH₂)ₙCOOR₆ |
| aryl | COOH | aryl | (CH₂)ₙCOOR₆ | (CH₂)ₙOR₆ |
| aryl | COOH | aryl | (CH₂)ₙOR₆ | H |
| aryl | COOH | aryl | (CH₂)ₙOR₆ | alkyl |
| aryl | COOH | aryl | (CH₂)ₙOR₆ | aryl |
| aryl | COOH | aryl | (CH₂)ₙOR₆ | (CH₂)ₙCOOR₆ |
| aryl | COOH | aryl | (CH₂)ₙOR₆ | (CH₂)ₙOR₆ |
| aryl | NHR₆ | H | (CH₂)ₙCOOR₆ | H |
| aryl | NHR₆ | H | (CH₂)ₙCOOR₆ | alkyl |
| aryl | NHR₆ | H | (CH₂)ₙCOOR₆ | aryl |
| aryl | NHR₆ | H | (CH₂)ₙCOOR₆ | (CH₂)ₙCOOR₆ |
| aryl | NHR₆ | H | (CH₂)ₙCOOR₆ | (CH₂)ₙOR₆ |
| aryl | NHR₆ | H | (CH₂)ₙOR₆ | H |
| aryl | NHR₆ | H | (CH₂)ₙOR₆ | alkyl |
| aryl | NHR₆ | H | (CH₂)ₙOR₆ | aryl |
| aryl | NHR₆ | H | (CH₂)ₙOR₆ | (CH₂)ₙ COOR₆ |
| aryl | NHR₆ | H | (CH₂)ₙOR₆ | (CH₂)ₙOR₆ |
| aryl | NHR₆ | alkyl | (CH₂)ₙCOOR₆ | H |
| aryl | NHR₆ | alkyl | (CH₂)ₙCOOR₆ | alkyl |
| aryl | NHR₆ | alkyl | (CH₂)ₙCOOR₆ | aryl |
| aryl | NHR₆ | alkyl | (CH₂)ₙCOOR₆ | (CH₂)ₙCOOR₆ |
| aryl | NHR₆ | alkyl | (CH₂)ₙCOOR₆ | (CH₂)ₙOR₆ |
| aryl | NHR₆ | alkyl | (CH₂)ₙOR₆ | H |
| aryl | NHR₆ | alkyl | (CH₂)ₙOR₆ | alkyl |
| aryl | NHR₆ | alkyl | (CH₂)ₙOR₆ | aryl |
| aryl | NHR₆ | alkyl | (CH₂)ₙOR₆ | (CH₂)ₙCOOR₆ |
| aryl | NHR₆ | alkyl | (CH₂)ₙOR₆ | (CH₂)ₙOR₆ |
| aryl | NHR₆ | aryl | (CH₂)ₙCOOR₆ | H |
| aryl | NHR₆ | aryl | (CH₂)ₙCOOR₆ | alkyl |
| aryl | NHR₆ | aryl | (CH₂)ₙCOOR₆ | aryl |
| aryl | NHR₆ | aryl | (CH₂)ₙCOOR₆ | (CH₂)ₙCOOR₆ |
| aryl | NHR₆ | aryl | (CH₂)ₙCOOR₆ | (CH₂)ₙOR₆ |
| aryl | NHR₆ | aryl | (CH₂)ₙOR₆ | H |
| aryl | NHR₆ | aryl | (CH₂)ₙOR₆ | alkyl |
| aryl | NHR₆ | aryl | (CH₂)ₙOR₆ | aryl |
| aryl | NHR₆ | aryl | (CH₂)ₙOR₆ | (CH₂)ₙCOOR₆ |
| aryl | NHR₆ | aryl | (CH₂)ₙOR₆ | (CH₂)ₙOR₆ |
| aryl | (CH₂)ₙCOOR₆ | H | (CH₂)ₙCOOR₆ | H |
| aryl | (CH₂)ₙCOOR₆ | H | (CH₂)ₙCOOR₆ | alkyl |
| aryl | (CH₂)ₙCOOR₆ | H | (CH₂)ₙCOOR₆ | aryl |
| aryl | (CH₂)ₙCOOR₆ | H | (CH₂)ₙCOOR₆ | (CH₂)ₙCOOR₆ |
| aryl | (CH₂)ₙCOOR₆ | H | (CH₂)ₙCOOR₆ | (CH₂)ₙOR₆ |
| aryl | (CH₂)ₙCOOR₆ | H | (CH₂)ₙOR₆ | H |
| aryl | (CH₂)ₙCOOR₆ | H | (CH₂)ₙOR₆ | alkyl |
| aryl | (CH₂)ₙCOOR₆ | H | (CH₂)ₙOR₆ | aryl |
| aryl | (CH₂)ₙCOOR₆ | H | (CH₂)ₙOR₆ | (CH₂)ₙCOOR₆ |
| aryl | (CH₂)ₙCOOR₆ | H | (CH₂)ₙOR₆ | (CH₂)ₙOR₆ |
| aryl | (CH₂)ₙCOOR₆ | alkyl | (CH₂)ₙCOOR₆ | H |
| aryl | (CH₂)ₙCOOR₆ | alkyl | (CH₂)ₙCOOR₆ | alkyl |
| aryl | (CH₂)ₙCOOR₆ | alkyl | (CH₂)ₙCOOR₆ | aryl |
| aryl | (CH₂) ₙCOOR₆ | alkyl | (CH₂)ₙCOOR₆ | (CH₂)ₙCOOR₆ |
| aryl | (CH₂)ₙCOOR₆ | alkyl | (CH₂)ₙCOOR₆ | (CH₂)ₙOR₆ |
| aryl | (CH₂) ₙCOOR₆ | alkyl | (CH₂)ₙOR₆ | H |
| aryl | (CH₂)ₙCOOR₆ | alkyl | (CH₂)ₙOR₆ | alkyl |
| aryl | (CH₂)ₙCOOR₆ | alkyl | (CH₂)ₙOR₆ | aryl |
| aryl | (CH₂)ₙCOOR₆ | alkyl | (CH₂)ₙOR₆ | (CH₂)ₙCOOR₆ |
| aryl | (CH₂)ₙCOOR₆ | alkyl | (CH₂)ₙOR₆ | (CH₂)ₙOR₆ |
| aryl | (CH₂)ₙCOOR₆ | aryl | (CH₂)ₙCOOR₆ | H |
| aryl | (CH₂)ₙCOOR₆ | aryl | (CH₂)ₙCOOR₆ | alkyl |
| aryl | (CH₂)ₙCOOR₆ | aryl | (CH₂)ₙCOOR₆ | aryl |
| aryl | (CH₂)ₙCOOR₆ | aryl | (CH₂)ₙCOOR₆ | (CH₂)ₙCOOR₆ |
| aryl | (CH₂)ₙCOOR₆ | aryl | (CH₂)ₙCOOR₆ | (CH₂)ₙOR₆ |
| aryl | (CH₂)ₙCOOR₆ | aryl | (CH₂)ₙOR₆ | H |
| aryl | (CH₂)ₙCOOR₆ | aryl | (CH₂)ₙOR₆ | alkyl |
| aryl | (CH₂)ₙCOOR₆ | aryl | (CH₂)ₙOR₆ | aryl |
| aryl | (CH₂)ₙCOOR₆ | aryl | (CH₂)ₙOR₆ | (CH₂)ₙCOOR₆ |
| aryl | (CH₂)ₙCOOR₆ | aryl | (CH₂)ₙOR₆ | (CH₂)ₙOR₆ |

The invention also relates to a method for the preparation of radiolabeled biomolecules comprising:
a) contacting a chelating agent of the invention with a carbonyl moiety of the formula [M(CO)₃]⁺, wherein M is rhenium (Re) or technetium (Tc), under conditions for forming a chelator-carbonyl complex; and
b) contacting the complex with a biomolecule for obtaining a radiolabeled biomolecule. This method is in particular useful for labeling biomolecules that are sensitive to temperature and extreme pH.

This method can for example be performed with a kit, comprising a first vial with the chelating agent of the invention, optionally a first reaction vial for contacting the chelating agent with the carbonyl moiety, a second vial with the biomolecule and optionally a second reaction vial for reacting the biomolecule with the chelator-carbonyl complex obtained in the first step of the reaction.

In an alternative embodiment the invention provides a method for the preparation of radiolabeled biomolecules comprising:
a) contacting a chelating agent of the invention with a biomolecule for obtaining a chelator-biomolecule.; and
b) contacting the chelator-biomolecule with a carbonyl moiety of the formula [M(CO)₃]⁺, wherein M is rhenium (Re) or technetium (Tc) under conditions for forming a radiolabeled biomolecule.

A kit for performing this method comprises for example a first vial with the chelating agent of the invention, optionally a first reaction vial for reacting the chelating agent with the biomolecule, a second vial with the carbonyl moiety and optionally a second reaction vial for reacting the chelator-biomolecule obtained in the first step of the reaction with the carbonyl.

The invention will be further illustrated in the example that follows.

### EXAMPLE

### Introduction

The bifunctional pyrazolyl-polyamines, pyrazolyl amino-thioether ligands, and pyrazolyl-aminophosphines contain different donor atom sets to stabilize the metal and have different functional groups in different positions to which seeking molecules such as, for example, monoclonal antibodies, peptides, oligonucleotides and glycoproteins, can be coupled. They can also have different substituents and alkyl chains in different positions of the backbone for tuning the physico-chemical properties of the molecules.

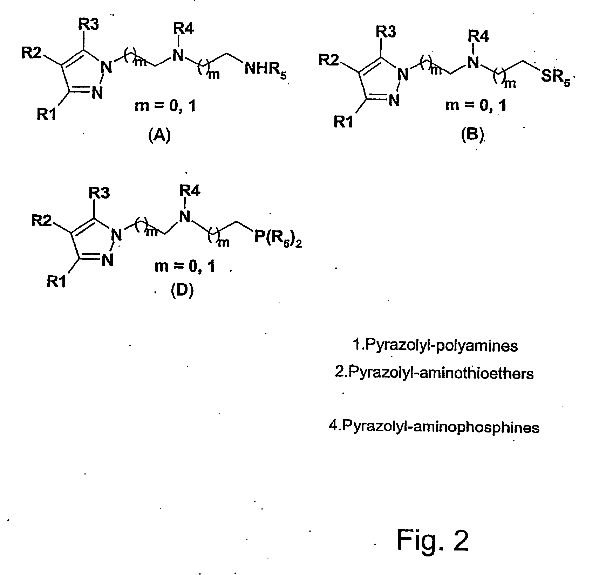

A general overview is given in Figure 1, showing possible combinations for metal fragments of the type [M(CO)₃]⁺ (M = Re, Tc, Mn). The three different types of bifunctional tridentate pyrazolyl-containing ligands, which are subject of this invention are depicted schematically in Figure 2.

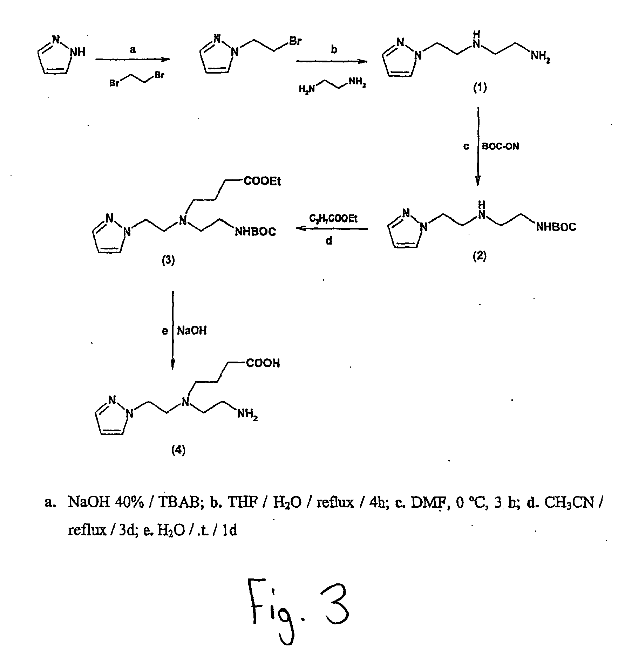

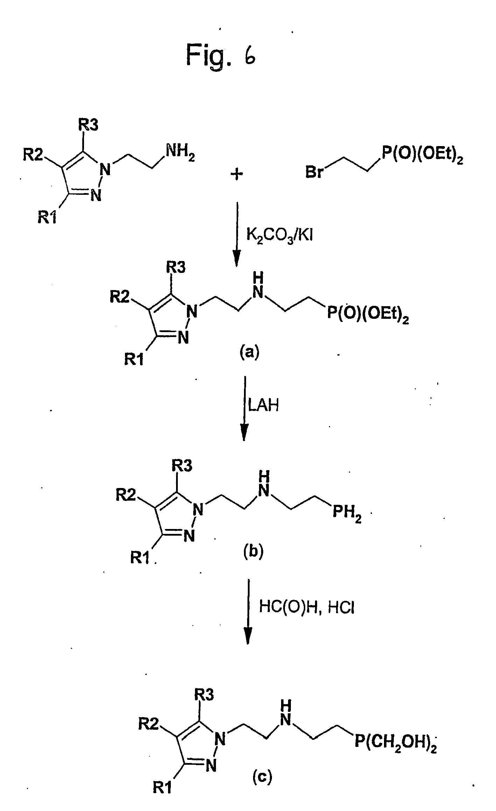

The present invention will be further illustrated in the Figures 3, 4 and 6, which are solely intended to clarify the invention. This family of ligands led to thermodynamically stable complexes and the versatility of the backbone is an important factor for tuning the physico-chemical properties of the compounds and obviously its pharmacokinetics. In Figure 6, some of the Re and Tc complexes referred as examples are schematically represented.

### Materials and methods

### 1. Synthesis of 2-[2-(pyrazol-1-yl)ethylimino]ethylamine (pz (CH₂)₂NH(CH₂)₂NH₂) (1) (see Figure 3)

A solution of **1-(2-bromoethyl)pyrazole** [6d] (12 mmol) in tetrahydrofuran was added dropwise to a solution of ethylenediamine (0.24 mol) in water. The mixture was refluxed for 4 hours. The THF was removed under vacuum and the water phase was washed with dichloromethane. After drying under vacuum resulted a yellow oil which was formulated as pz (CH₂)₂NH(CH₂)₂NH₂ (1).
Yield: 50%
¹H-NMR (D₂O) : 7.53 (d, H(3)pz, 1H) ; 7.45 (d, H(5)pz, 1H) ; 6.23 (t, H(4)pz, 1H); 4.14 (t, CH2, 2H); 2.91 (t, CH₂, 2H); 2.77 (t, CH₂, 2H); 2.62 (t, CH₂, 2H).

### 2. Synthesis pz(CH₂)₂N[(CH₂)₃COOH](CH₂)₂NH₂ (4) (see Figure 3)

### 2.1. BOC-ON protection

A solution of 1 (1.1 g; 7 mmol) in DMF (20 ml) was cooled to 0°C and a solution of BOC-ON (1,7 g; 7 mmol) in DMF (20 ml) was added dropwise. The reaction mixture was stirred for 3 hours at 0°C. The solvent was removed under vacuum and the solid residue was dissolved in water and washed with chloroform 3 times, yielding 2 as an oil. Yield: 68%. ¹H-NMR (D₂O) : 7.55 (d, H(3)pz, 1H) ; 7.47 (d, H(5)pz, 1H) ; 6.24 (t, H(4)pz, 1H); 4.38 (t, CH₂, 2H); 3.40 (t, CH₂, 2H); 3.20 (t, CH₂, 2H) ; 2.99 (t, CH₂, 2H) ; 1.25 (s, CH₃, 9H).

### 2.2. Alkylation with ethyl 4-bromobutyrate, hydrolysis and deprotection

Compound 2 (757 mg; 3 mmol) was dissolved in 10 ml of acetonitrile. Potassium carbonate (829 mg; 6 mmol) and a catalytic amount of potassium iodide were added to the solution, and ethyl 4-bromobutyrate (858 ml, 16 mmol) was added dropwise. After refluxing for 3 days, the supernatant was separated by filtration and vacuum dried leading to 3. This compound (733 mg, 2 mmol) was dissolved in an aqueous solution of NaOH (800 mg, 20 mmol) and reacted for one day at room temperature. The solution was then neutralized with HCl 1N and vacuum dried. The solid residue was dissolved in methanol, the precipitating salts were filtered off, and the solvent was removed under vacuum, yielding a yellow/brown oil formulated as 4. Yield: (50%).
¹H-NMR (D₂O): 7.78 (d, H(3)pz, 1H); 7.64 (d, H(5)pz, 1H); 6.42 (t, H(4)pz, 1H) ; 4.36 (t, CH₂, 2H) ; 3.10 (t, CH₂, 2H) ; 3.02 (t, CH₂, 2H) ; 2.86 (t, CH₂, 2H); 2.64 (t, CH₂, 2H) ; 2.15 (t, CH₂, 2H); 1.68 (q, CH₂, 2H).

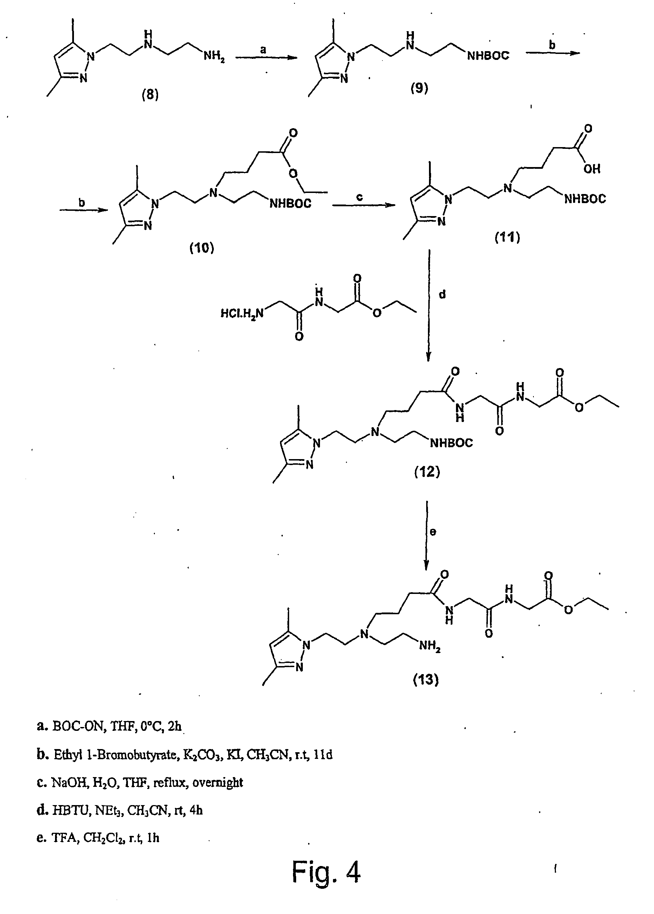

### 3. Synthesis of 3,5-Mepz (CH₂)₂N[(CH₂)₃GlyGlyOEt)](CH₂)₂NH₂ (13) (Figure 4)

### 3.1 BOC-ON protection (9)

Compound **8** (3.41 g, 18.71 mmol) [4c] was dissolved in THF (25 mL) and cooled to a temperature between -10°C and 0°C. BOC-ON (4.60 g, 18.71 mmol) in THF (20 ml) was added dropwise and the reaction mixture was stirred for 2 h at 0°C, resulting in the complete conversion of **8** as monitored by TLC (R_{f} = 0.5, 100% MeOH). The reaction mixture was then warmed to room temperature and partitioned between a saturated aqueous Na₂CO₃ solution and dichloromethane. The organic layer was separated, dried over anhydrous MgSO₄, filtered, and concentrated under reduced pressure to afford the product **9** in quantitative yield (by ¹H-NMR), as a highly viscous colorless oil. This product was used in the next step without further purification.
¹H-NMR (CDCl₃) : δ 5.76 (s, pyrazol, 1H) , 5.08 (s br., NH, 1H), 4.04 (t, CH₂, 2H), 3.18 (m, CH₂, 2H), 2.99 (t, CH₂, 2H), 2.72 (t, CH₂, 2H), 2.18 (s, CH₃, 3H), 2.20 (s, CH₃, 3H), 1.40 (s, C(CH₃)₃, 9H).

### 3.2 Synthesis of 3,5-Me₂pz(CH₂)₂N[(CH₂)₃COOH](CH₂)₂NHBOC (11)

To a stirred solution of the crude product **9** (1.02 g) in CH₃CN (15 mL), ethyl 4-bromobutyrate (1.4 g, 7.20 mmol), K₂CO₃ (1.00 g, 7.20 mmol) and a catalytic amount of KI were added. The obtained suspension was allowed to react under vigorous stirring for 11 days, being the reaction monitored by TLC (*R_{f}* product = 0.4, 10% MeOH/CH₂Cl₂). After elimination of the white solids in suspension by filtration, the solvent was evaporated in vacuum and a pale-yellow viscous oil was obtained. The crude product was chromatographed on an appropriate column of silica gel with 75-100% ethyl acetate/hexane (gradient) to afford **10** as a pale-yellow viscous oil, which solidifies on standing for several days at room temperature. Yield: 0.73 g (51% yield).

A solution of **10** (4.6 g, 11.60 mmol) in THF (190 mL) and aqueous NaOH (8.3 mL of a 14 N NaOH solution, 116.0 mmol) was refluxed for 8 h. The reaction was monitored by TLC (R_{f} product = 0.2, 10% MeOH/CH₂Cl₂). After neutralization with HCl 4N (pH 6-7), the THF/H₂O solution was evaporated to dryness under reduced pressure. The crude product was chromatographed on an appropriate column of silica gel with 10-50% MeOH/CHCl₃ (gradient) to afford 11 as an highly viscous colorless oil, which crystallizes on standing after several days. Yield: 2.82 g (66%).

Compound **10**: ¹H-NMR (CDCl₃): δ 5.75 (s, pyrazol, 1H), 4.09 (q, CH₂, 2H), 3.98 (s br., CH₂, 2H), 3.08 (s br., CH₂, 2H), 2.78 (s br., CH₂, 2H), 2.45-2.51 (m, CH₂, 4H), 2.23 (s, CH₃, 3H), 2.18 (m, CH₃, CH₂, 5H), 1.63 (s br., CH₂, 2H), 1.41 (s, C(CH₃)₃, 9H), 1.23 (t, CH₃, 3H).

Compound 11: ¹H-NMR (CDCl₃): δ 5.81 (s, pyrazol, 1H), 4.93(s br., NH, 1H) 4.12 (t br., CH₂, 2H), 3.04 (q br., CH₂, 2H), 2.86 (t br., CH₂, 2H), 2.58-2.64 (m, CH₂, 4H), 2.42 (t, CH₂, 2H), 2.24 (s, CH₃, 3H), 2.19 (s, CH₃, 3H), 1.79 (m, CH₂, 2H), 1.40 (s, C(CH₃)₃, 9H).

Compound 3,5-Me₂pz(CH₂)₂N[(CH₂)₃CONHGlyGlyOEt](CH₂)₂NH₂ (13) was prepared as follows (see Figure 4).

To a solution of **11** (1.51 g, 4.09 mmol) in CH₃CN (48 mL) were added GlyGly ethyl ester hydrochloride (0.57 g, 4.09 mmol), triethylamine (1.24g, 12.27 mmol), and HBTU (1.55 g, 4.09 mmol). The reaction mixture was stirred 20 h at room temperature under nitrogen. The reaction was monitored by TLC (R_{f} product = 0.8, 20% MeOH/CH₂Cl₂). The solvent was evaporated and the crude product obtained was purified by chromatography on an appropriate silica gel column with 3-5% MeOH/CHCl₃ (gradient) to afford **12** as a viscous colorless oil. Yield: 1.23 g (59%).

A solution of 3 , 5-Me₂pz(CH₂)₂N[(CH₂)₃CONHGlyGlyOEt] (CH₂)₂NHBOC (12) (1.23 g, 2.41 mmol) in CH₂Cl₂/TFA (25 mL / 4.1 mL) was allowed to react for 2 h. The reaction was monitored by TLC (R_{f} = 0.4, 20% MeOH/CH₂Cl₂). The solvent and the TFA were evaporated under reduced pressure and a highly viscous pale-yellow oil was obtained. This oil was dissolved in water, neutralized with NaOH 1N (pH 7-8) and the solvent evaporated to dryness. TLC: *R_{f}* = 0.2, 20% MeOH/CH₂Cl₂ The compound was further purified by chromatography on an appropriate silica gel column with 20-40% MeOH/CHCl₃ (gradient) to afford 13 as a viscous colorless oil. Yield: 0.97 g (98 %).

Compound **12**: ¹H-NMR (CDCl₃) : δ 8.66 (s br., NH, 1H), 7.00 (s br., NH, 1H), 5.80 (s, pyrazol, 1H), 4.91 (s br., NH, 1H) 4.15 (q., CH₂, 2H), 4.04 (s br., CH₂, 2H), 3.97 (d, CH₂, 2H), 3.90 (d, CH₂, 2H), 2.89 (s br., CH₂, 2H), 2.69 (s br., CH₂, 2H), 2.51 (s br., CH₂, 2H), 2.39 (s br., CH₂, 2H), 2.30 (s br., CH₂, 2H), 2.20 (s, CH₃, 3H), 2.18 (s, CH₃, 3H), 1.74 (s br., CH₂, 2H), 1.38 (s, C(CH₃)₃, 9H), 1.23 (t, CH₃, 3H).

Compound **13**: ¹H-NMR (CD3OD): δ 5.84 (s, pyrazol, 1H), 4.17 (q, CH₂, 2H), 4.06 (t, CH₂, 2H), 3.91 (d, CH₂, 4H), 2.97 (t, CH₂, 2H), 2.71-2.80 (m, CH₂, 4H), 2.51 (t, CH₂, 2H), 2.25 (s, CH₃, 3H), 2.15 (s, CH₃, 3H), 2.12 (t, CH₂, 2H), 1.66 (m, CH₂, 2H), 1.25 (t, CH₃, 3H).

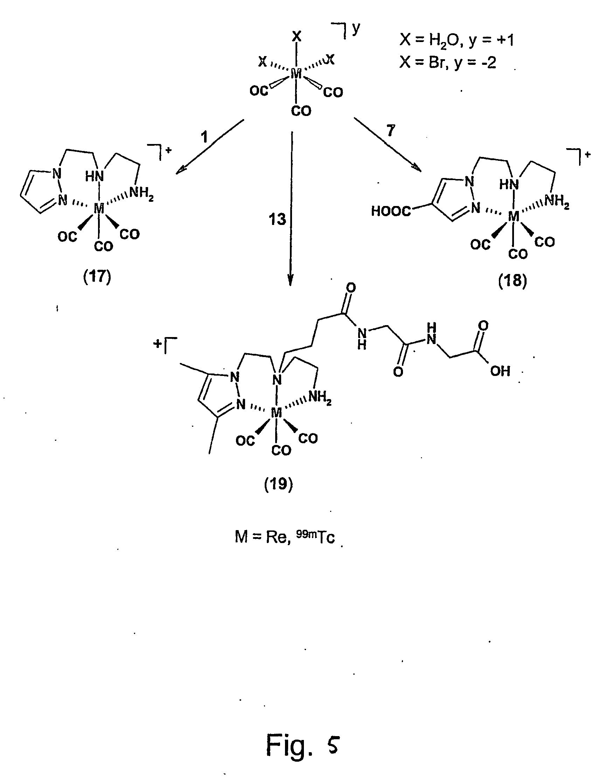

### 4. Re and Tc compounds (see Figure 5)

### 4.1. Synthesis of [Re(CO)₃(κ³-pz(CH₂)₂NH(CH₂)₂NH₂)] Br (17a)

### (Reference Example)

100 mg (0.130 mmol) of (NEt₄)₂[ReBr₃(CO)₃] were mixed with 20 mg (0.130 mmol) of the compound **1**
(pz(CH₂)₂NH(CH₂)₂NH₂) in water, and the solution was refluxed for 2 hours. The volume was then reduced under vacuum, and the mixture was left at 4°C until a white solid precipitated. Yield: >90% by ¹H-NMR
¹H-NMR (D₂O): 7.82 (d, H(3)pz, 1H); 7.7.6 (d, H(5)pz, 1H); 6.54 (s br, NH, 1H); 6.39 (t, H(4)pz, 1H); 4.86 (s, largo, NH₂, 1H) ; 4.43 (m, CH₂, 1H) ; 4.16 (m, CH₂, 1H) ; 3.94 (s, largo, NH₂, 1H) ; 3.50 (m, CH₂, 1H) ; 2.87 (m, CH₂, 1H) ; 2.71 (m, CH₂, 2H); 2.48 (m, CH₂, 1H) ; 2.08 (m, CH₂, 1H).

### 4.2. Synthesis of [^{99m}Tc (CO)₃(κ³-pz(CH₂)₂NH(CH₂)₂NH₂)]+

### (17b)(Reference Example)

100 µl of a solution of compound **1 (pz(CH₂)₂NH(CH₂)₂NH₂)** 10⁻⁴ M was added to 1 ml of a solution of [^{99m}TC(OH)₃(CO)₃] + (1-2 mCi) in phosphate buffer. The solution was incubated for 30 min at 100°C and then analyzed by HPLC. The radiochemical purity was >90%.

### 4.3 Synthesis of [Re(CO)₃(κ³-(4-carboxylic acid)pz(CH₂)₂NH (CH₂)₂NH₂)]Br (18a) (Reference Example)

100 mg (0.130 mmol) of (NEt₄)₂[ReBr3(CO)₃] were mixed with 26 mg (0.130 mmol) of compound **7**, in water, and the solution was refluxed for 2 hours. The volume was then reduced under vacuum, and the mixture was left at 4°C until a white solid precipitated.
Yield: >90% by ¹H-NMR
¹H-NMR (D₂O) : δ 8.22 (s, H(3)pz, 1H); 8.20 (s, H(5)pz, 1H); 6.62 (s, largo, NH, 1H); 4.94 (s, largo, NH₂, 1H); 4.43 (m, CH₂, 1H) ; 4.25 (m, CH₂, 1H) ; 4.05 (s, largo, NH₂, 1H) ; 3.52 (m, CH₂, 1H) ; 2.92 (m, CH₂, 1H) ; 2.76 (m, CH₂, 2H); 2.53 (m, CH₂, 1H) ; 2.14 (m, CH₂, 1H).
IV (KBr) (ν/cm⁻¹): 2010 (C=O); 1885 (C=O); 1690 (C=O ligando)

### 4.4. Synthesis of [Re(CO)₃(κ³-3,5-Me₂pz(CH₂)₂N(CH₂)₂ (glygly) NH₂) Br (19a)

100 mg (0.130 mmol) of (NEt₄)₂[ReBr₃(CO)₃] were mixed with 53 mg (0.130 mmol) of the ligand **13**, in water, and the solution was refluxed overnight.
Yield: 100% by ¹H-NMR
¹H-NMR (D₂O): δ 6.04 (s, H(4)pz, 1H); 5.05 (s, br, NH₂, 1H); 4.36-4.31 (m, CH₂, 1H); 4.16-4.04 (m, CH₂, 1H); 3.88 (s, NHCH₂CO, 2H); 3.84 (s, NHCH₂CO, 2H); 3.65 (s, br, NH₂, 1H); 3.53 (m, CH₂, 1H) ; 3.30 (m, CH₂, 2H); 2.86 (m, CH₂, 1H) ; 2.74 (m, CH₂, 2H); 2.57 (m, CH₂, 1H) ; 2.40 (m, CH₂, 1H) ; 2.31 (m, CH₂, 1H) ; 2.73 (s, CH₃, 3H) ; 2.16 (s, CH₃, 3H); 2.10 (m, CH₂, 1H) ; 1.95 (m, CH₂, 1H).

### 4.5.Synthesis of [^{99m}TC(CO)₃ (κ³-3,5-Me₂pz(CH₂)₂N(CH₂)₂ (glygly)NH₂)]⁺ (19b)

100 ml of a solution of **13** (10⁻³ M) was added to 1 ml of a solution of [^{99m}Tc (OH)₃(CO)₃]⁺ (1-2 mCi) in phosphate buffer. The solution was incubated for 1h at 100°C and then analysed by HPLC. The radiochemical purity was >90%.

### 5. Synthesis of pyrazolyl-aminophosphines (figure 6)

The preparation of the pyrazolyl-aminophosphines of the invention involves alkylation of 1-(2-aminoethyl)pyrazoles with (2-bromoethyl)phosphonic acid diethyl esther, yielding a pyrazole-amino-phosphonate derivative (compound a). Reduction of compound a with lithium aluminium hydride (LAH) affords a primary phosphine (compound b) which is then converted to the final chelator (compound c) by treatment with formaldehyde in acidic medium (Katti et al., J. Am. Chem. Soc. 122, 1554 (2000). The scheme shown in Figure 7 is illustrative of an approach which can be used to prepare compounds according to the present invention.

### REFERENCES

[1]
   a) Alberto et al WO 98/48848,
   b) Alberto et al., WO 00/50086,
   c) Alberto et al., WO 01/00637
   d) Alberto et al, US 6, 344,178 B1
[2] a) Hilger et al, US 6, 488,909 B1.
[3]
   a) Alberto et al, Polyhedron 17 (1998) 1303
   b) Alberto et al., J. Med. Chem. 41 (1998) 4429
   c) Alberto et al, J. Am. Chem. Soc. 121 (1999) 6076
   d) Alberto et al, J. Nucl. Med. 40 (1999) 1913
   e) Schibli et al, Nucl. Med. and Biol. 26 (1999) 711
   f) Alberto et al, Bioconjugate Chem. 11 (2000) 414
   g) Alberto et al, Bioconjugate Chem. 11 (2000) 345
   h) Alberto et al., Chem. Eur. Journal 7 (2001) 1868
   i) Alberto et al, Angew. Chem. Int. Ed. 40 (2001) 3062
   j) Alberto et al, Bioconjugate Chem. 13 (2002) 750.
[4]
   a) Santos et al, J. Am. Chem. Soc. 122 (2000) 11240
   b) Santos et al., Inorg. Chem. 40 (2001) 5147
   c) Santos et al, J. Chem. Soc. Dalton Trans.(2002) 4714.
[5]
   a) Valliant et al, Inorg Chem. Commun. 41 (2002) 628
   b) Valliant et al, Inorg Chem. 41 (2002) 6417
[6]
   a) Sorrell et al, Inorg. Chem. 22 (1983) 1883
   b) Driessen et al. J. Chem. Soc. Dalton Trans. (1992) 481
   c) Parkin et al., Inorg. Chem. 35 (1996) 2415
   d) Ballesteros et al., Bioorganic and Medicinal Chem. 7 (1999) 517.
   e) Bouwman et al., Inorg. Chim. Acta (2000) 183
[7] Holzer et al, J. Heterocyclic Chem. 130 (1993) 865

## Claims

1. Chelating agent of the general formula: 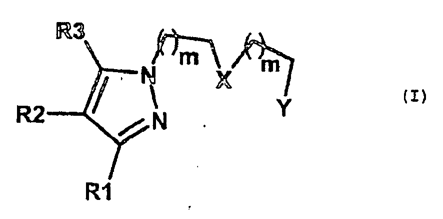 wherein m is 0 or 1
X is NR₄;
Y is NHR₅, SR₅ or P(R₅)₂;
R₁ and R₃ are the same or different and are selected from H, alkyl or aryl;
R₂ is H, COOH, NHR₆ or (CH₂)ₙCOOR₆;
R₄ is (CH₂)ₙCOOR₆, or (CH₂)ₙOR₆;
R₅ is H, alkyl, aryl, (CH₂) ₙCOOR₆, or (CH₂)ₙOR₆;
R₆ is H, alkyl or aryl, or a biomolecule; and
n is 1, 2, 3, 4, 5, 6, 7, 8, 9 or 10.

2. Chelating agent as claimed in claim 1, wherein the alkyl is a C₁ alkyl, C₂ alkyl, C₃ alkyl, C₄ alkyl, C₅ alkyl or C₆ alkyl.

3. Chelating agent as claimed in claim 2, wherein the alkyl is methyl, ethyl, *n-*propyl, isopropyl, *n-*butyl, isobutyl, *s*-butyl, *t*-butyl, *n-*pentyl, isopentyl, neopentyl, *n-*hexyl, isohexyl (2-methylpentyl), neohexyl (2, 2-dimethylbutyl), 3-methylpentyl, 2,3-dimethylbutyl.

4. Chelating agent as claimed in claim 1, wherein the aryl is monocyclic, preferably phenyl or benzyl, or polycyclic, C₁₀-C₁₈, and optionally substituted with alkyl, carboxy, oxo, amino, alkoxy or aldehyde groups.

5. Chelating agent as claimed in claim 4, wherein aryl is phenyl or benzyl.

6. Chelating agent as claimed in claim 1, wherein n is 2, 3, 4, 5 or 6 and preferably 2, 3 or 4.

7. Chelating agent as claimed in claim 1, which agent is a pyrazolyl-polyamine of the general formula: 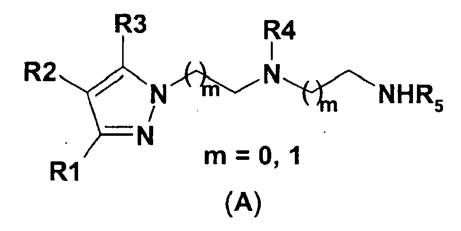 wherein R₁, R₂, R₃, R₄ and R₅ are as defined in claim 1.

8. Chelating agent as claimed in claim 1, which agent is a pyrazolyl-aminophosphine of the general formula: 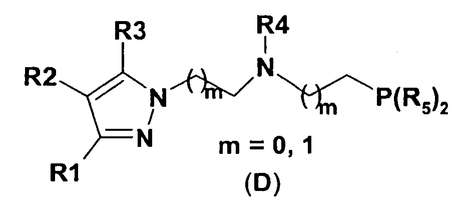 wherein R₁, R₂, R₃, R₄ and R₅ are as defined in claim 1.

9. Chelating agent as claimed in claim 1, wherein X is NR₄ and Y is NHR₅, R₆ is H, C₁ alkyl, C₂ alkyl C₃ alkyl C₄ alkyl C₅ alkyl or C₆ alkyl, phenyl, benzyl or a biomolecule and R₁, R₂, R₃, R₄ and R₅ are as listed in the following combinations:
| **R1** | **R2** | **R3** | **R4** | **R5** |
|---|---|---|---|---|
| H | H | H | (CH₂) ₙCOOR₆ | H |
| H | H | H | (CH₂)ₙCOOR₆ | alkyl |
| H | H | H | (CH₂)ₙCOOR₆ | aryl |
| H | H | H | (CH₂)ₙCOOR₆ | (CH₂)ₙCOOR₆ |
| H | H | H | (CH₂)ₙCOOR₆ | (CH₂)ₙOR₆ |
| H | H | H | (CH₂)ₙOR₆ | H |
| H | H | H | (CH₂)ₙOR₆ | alkyl |
| H | H | H | (CH₂)ₙOR₆ | aryl |
| H | H | H | (CH₂)ₙOR₆ | (CH₂)ₙCOOR₆ |
| H | H | H | (CH₂)ₙOR₆ | (CH₂)ₙOR₆ |
| H | H | alkyl | (CH₂)ₙCOOR₆ | H |
| H | H | alkyl | (CH₂)ₙCOOR₆ | alkyl |
| H | H | alkyl | (CH₂)ₙCOOR₆ | aryl |
| H | H | alkyl | (CH₂) ₙCOOR₆ | (CH₂)ₙCOOR₆ |
| H | H | alkyl | (CH₂)ₙCOOR₆ | (CH₂)ₙOR₆ |
| H | H | alkyl | (CH₂)ₙOR₆ | H |
| H | H | alkyl | (CH₂)ₙOR₆ | alkyl |
| H | H | alkyl | (CH₂)ₙOR₆ | aryl |
| H | H | alkyl | (CH₂)ₙOR₆ | (CH₂)ₙCOOR₆ |
| H | H | alkyl | (CH₂)ₙOR₆ | (CH₂)ₙOR₆ |
| H | H | aryl | (CH₂)ₙCOOR₆ | H |
| H | H | aryl | (CH₂)ₙCOOR₆ | alkyl |
| H | H | aryl | (CH₂)ₙCOOR₆ | aryl |
| H | H | aryl | (CH₂)ₙCOOR₆ | (CH₂)ₙCOOR₆ |
| H | H | aryl | (CH₂)ₙCOOR₆ | (CH₂)ₙOR₆ |
| H | H | aryl | (CH₂)ₙOR₆ | H |
| H | H | aryl | (CH₂)ₙOR₆ | alkyl |
| H | H | aryl | (CH₂)ₙOR₆ | aryl |
| H | H | aryl | (CH₂)ₙOR₆ | (CH₂)ₙCOOR₆ |
| H | H | aryl | (CH₂) ₙOR₆ | (CH₂)ₙOR₆ |
| H | COOH | H | (CH₂)ₙCOOR₆ | H |
| H | COOH | H | (CH₂)ₙCOOR₆ | alkyl |
| H | COOH | H | (CH₂)ₙCOOR₆ | aryl |
| H | COOH | H | (CH₂)ₙCOOR₆ | (CH₂)ₙCOOR₆ |
| H | COOH | H | (CH₂)ₙCOOR₆ | (CH₂)ₙOR₆ |
| H | COOH | H | (CH₂)ₙOR₆ | H |
| H | COOH | H | (CH₂)ₙOR₆ | alkyl |
| H | COOH | H | (CH₂)ₙOR₆ | aryl |
| H | COOH | H | (CH₂)ₙOR₆ | (CH₂)ₙCOOR₆ |
| H | COOH | H | (CH₂)ₙOR₆ | (CH₂)ₙOR₆ |
| H | COOH | alkyl | (CH₂)ₙCOOR₆ | H |
| H | COOH | alkyl | (CH₂)ₙCOOR₆ | alkyl |
| H | COOH | alkyl | (CH₂)ₙCOOR₆ | aryl |
| H | COOH | alkyl | (CH₂)ₙCOOR₆ | (CH₂)ₙCOOR₆ |
| H | COOH | alkyl | (CH₂)ₙCOOR₆ | (CH₂)ₙOR₆ |
| H | COOH | alkyl | (CH₂)ₙOR₆ | H |
| H | COOH | alkyl | (CH₂)ₙOR₆ | alkyl |
| H | COOH | alkyl | (CH₂)ₙOR₆ | aryl |
| H | COOH | alkyl | (CH₂)ₙOR₆ | (CH₂)ₙCOOR₆ |
| H | COOH | alkyl | (CH₂)ₙOR₆ | (CH₂) ₙOR₆ |
| H | COOH | aryl | (CH₂)ₙCOOR₆ | H |
| H | COOH | aryl | (CH₂)ₙCOOR₆ | alkyl |
| H | COOH | aryl | (CH₂)ₙCOOR₆ | aryl |
| H | COOH | aryl | (CH₂)ₙCOOR₆ | (CH₂)ₙCOOR₆ |
| H | COOH | aryl | (CH₂)ₙCOOR₆ | (CH₂)ₙOR₆ |
| H | COOH | aryl | (CH₂)ₙOR₆ | H |
| H | COOH | aryl | (CH₂)ₙOR₆ | alkyl |
| H | COOH | aryl | (CH₂)ₙOR₆ | aryl |
| H | COOH | aryl | (CH₂)ₙOR₆ | (CH₂)ₙCOOR₆ |
| H | COOH | aryl | (CH₂)ₙOR₆ | (CH₂)ₙOR₆ |
| H | NHR₆ | H | (CH₂) COOR₆ | H |
| H | NHR₆ | H | (CH₂)ₙCOOR₆ | alkyl |
| H | NHR₆ | H | (CH₂)ₙCOOR₆ | aryl |
| H | NHR₆ | H | (CH₂)ₙCOOR₆ | (CH₂)ₙCOOR₆ |
| H | NHR₆ | H | (CH₂)ₙCOOR₆ | (CH₂)ₙOR₆ |
| H | NHR₆ | H | (CH₂)ₙOR₆ | H |
| H | NHR₆ | H | (CH₂)ₙOR₆ | alkyl |
| H | NHR₆ | H | (CH₂) ₙOR₆ | aryl |
| H | NHR₆ | H | (CH₂)ₙOR₆ | (CH₂)ₙCOOR₆ |
| H | NHR₆ | H | (CH₂)ₙOR₆ | (CH₂)ₙOR₆ |
| H | NHR₆ | alkyl | (CH₂)ₙCOOR₆ | H |
| H | NHR₆ | alkyl | (CH₂)ₙCOOR₆ | alkyl |
| H | NHR₆ | alkyl | (CH₂)ₙCOOR₆ | aryl |
| H | NHR₆ | alkyl | (CH₂)ₙCOOR₆ | (CH₂)ₙCOOR₆ |
| H | NHR₆ | alkyl | (CH₂)ₙCOOR₆ | (CH₂)ₙOR₆ |
| H | NHR₆ | alkyl | (CH₂)ₙOR₆ | H |
| H | NHR₆ | alkyl | (CH₂)ₙOR₆ | alkyl |
| H | NHR₆ | alkyl | (CH₂)ₙOR₆ | aryl |
| H | NHR₆ | alkyl | (CH₂)ₙOR₆ | (CH₂)ₙCOOR₆ |
| H | NHR₆ | alkyl | (CH₂)ₙOR₆ | (CH₂)ₙOR₆ |
| H | NHR₆ | aryl | (CH₂)ₙCOOR₆ | H |
| H | NHR₆ | aryl | (CH₂)ₙCOOR₆ | alkyl |
| H | NHR₆ | aryl | (CH₂)COOR₆ | aryl |
| H | NHR₆ | aryl | (CH₂)ₙCOOR₆ | (CH₂)ₙCOOR₆ |
| H | NHR₆ | aryl | (CH₂)ₙCOOR₆ | (CH₂)ₙOR₆ |
| H | NHR₆ | aryl | (CH₂)ₙOR₆ | H |
| H | NHR₆ | aryl | (CH₂)ₙOR₆ | alkyl |
| H | NHR₆ | aryl | (CH₂)ₙOR₆ | aryl |
| H | NHR₆ | aryl | (CH₂)ₙOR₆ | (CH₂)ₙCOOR₆ |
| H | NHR₆ | aryl | (CH₂)ₙOR₆ | (CH₂)ₙOR₆ |
| H | (CH₂)ₙCOOR₆ | H | (CH₂)ₙCOOR₆ | H |
| H | (CH₂)ₙCOOR₆ | H | (CH₂)ₙCOOR₆ | alkyl |
| H | (CH₂)ₙCOOR₆ | H | (CH₂)ₙCOOR₆ | aryl |
| H | (CH₂)ₙCOOR₆ | H | (CH₂)ₙCOOR₆ | (CH₂)ₙCOOR_{b} |
| H | (CH₂)ₙCOOR₆ | H | (CH₂)ₙCOOR₆ | (CH₂)ₙOR₆ |
| H | (CH₂)ₙCOOR₆ | H | (CH₂)ₙOR₆ | H |
| H | (CH₂)ₙCOOR₆ | H | (CH₂)ₙOR₆ | alkyl |
| H | (CH₂)ₙCOOR₆ | H | (CH₂)ₙOR₆ | aryl |
| H | (CH₂)ₙCOOR₆ | H | (CH₂)ₙOR₆ | (CH₂)ₙCOOR₆ |
| H | (CH₂)ₙCOOR₆ | H | (CH₂)ₙOR₆ | (CH₂)ₙOR₆ |
| H | (CH₂)ₙCOOR₆ | alkyl | (CH₂)ₙCOOR₆ | H |
| H | (CH₂)ₙCOOR₆ | alkyl | (CH₂)ₙCOOR₆ | alkyl |
| H | (CH₂)ₙCOOR₆ | alkyl | (CH₂)ₙCOOR₆ | aryl |
| H | (CH₂)ₙCOOR₆ | alkyl | (CH₂)ₙCOOR₆ | (CH₂)ₙCOOR₆ |
| H | (CH₂)ₙCOOR₆ | alkyl | (CH₂)ₙCOOR₆ | (CH₂)ₙOR₆ |
| H | (CH₂)ₙCOOR₆ | alkyl | (CH₂)ₙOR₆ | H |
| H | (CH₂)ₙCOOR₆ | alkyl | (CH₂)ₙOR₆ | alkyl |
| H | (CH₂)ₙCOOR₆ | alkyl | (CH₂)ₙOR₆ | aryl |
| H | (CH₂)ₙCOOR₆ | alkyl | (CH₂)ₙOR₆ | (CH₂)ₙCOOR₆ |
| H | (CH₂)ₙCOOR₆ | alkyl | (CH₂)ₙOR₆ | (CH₂)ₙOR₆ |
| H | (CH₂)ₙCOOR₆ | aryl | (CH₂)ₙCOOR₆ | H |
| H | (CH₂)ₙCOOR₆ | aryl | (CH₂)ₙCOOR₆ | alkyl |
| H | (CH₂)ₙCOOR₆ | aryl | (CH₂)ₙCOOR₆ | aryl |
| H | (CH₂)ₙCOOR₆ | aryl | (CH₂)ₙCOOR₆ | (CH₂) ₙCOOR₆ |
| H | (CH₂)ₙCOOR₆ | aryl | (CH₂)ₙCOOR₆ | (CH₂)ₙOR₆ |
| H | (CH₂)ₙCOOR₆ | aryl | (CH₂)ₙOR₆ | H |
| H | (CH₂)ₙCOOR₆ | aryl | (CH₂)ₙOR₆ | alkyl |
| H | (CH₂)ₙCOOR₆ | aryl | (CH₂)ₙOR₆ | aryl |
| H | (CH₂)ₙCOOR₆ | aryl | (CH₂)ₙOR₆ | (CH₂)ₙCOOR₆ |
| H | (CH₂)ₙCOOR₆ | aryl | (CH₂)ₙOR₆ | (CH₂)ₙOR₆ |
| alkyl | H | H | (CH₂)ₙCOOR₆ | H |
| alkyl | H | H | (CH₂)ₙCOOR₆ | alkyl |
| alkyl | H | H | (CH₂)ₙCOOR₆ | aryl |
| alkyl | H | H | (CH₂)ₙCOOR₆ | (CH₂)ₙCOOR₆ |
| alkyl | H | H | (CH₂)ₙCOOR₆ | (CH₂)ₙOR₆ |
| alkyl | H | H | (CH₂)ₙOR₆ | H |
| alkyl | H | H | (CH₂)ₙOR₆ | alkyl |
| alkyl | H | H | (CH₂)ₙOR₆ | aryl |
| alkyl | H | H | (CH₂)ₙOR₆ | (CH₂)ₙCOOR₆ |
| alkyl | H | H | (CH₂)ₙOR₆ | (CH₂)ₙOR₆ |
| alkyl | H | alkyl | (CH₂)ₙCOOR₆ | H |
| alkyl | H | alkyl | (CH₂)ₙCOOR₆ | alkyl |
| alkyl | H | alkyl | (CH₂)ₙCOOR₆ | aryl |
| alkyl | H | alkyl | (CH₂)ₙCOOR₆ | (CH₂)ₙCOOR₆ |
| alkyl | H | alkyl | (CH₂)ₙCOOR₆ | (CH₂)ₙOR₆ |
| alkyl | H | alkyl | (CH₂)ₙOR₆ | H |
| alkyl | H | alkyl | (CH₂)ₙOR₆ | alkyl |
| alkyl | H | alkyl | (CH₂)ₙOR₆ | aryl |
| alkyl | H | alkyl | (CH₂)ₙOR₆ | (CH₂)ₙCOOR₆ |
| alkyl | H | alkyl | (CH₂)ₙOR₆ | (CH₂)ₙOR₆ |
| alkyl | H | aryl | (CH₂)ₙCOOR₆ | H |
| alkyl | H | aryl | (CH₂)ₙCOOR₆ | alkyl |
| alkyl | H | aryl | (CH₂)ₙCOOR₆ | aryl |
| alkyl | H | aryl | (CH₂)ₙCOOR₆ | (CH₂)ₙCOOR₆ |
| alkyl | H | aryl | (CH₂)ₙCOOR₆ | (CH₂)ₙOR₆ |
| alkyl | H | aryl | (CH₂)ₙOR₆ | H |
| alkyl | H | aryl | (CH₂)ₙOR₆ | alkyl |
| alkyl | H | aryl | (CH₂)ₙOR₆ | aryl |
| alkyl | H | aryl | (CH₂)ₙOR₆ | (CH₂)ₙCOOR₆ |
| alkyl | H | aryl | (CH₂)ₙOR₆ | (CH₂)ₙOR₆ |
| alkyl | COOH | H | (CH₂)ₙCOOR₆ | H |
| alkyl | COOH | H | (CH₂)ₙCOOR₆ | alkyl |
| alkyl | COOH | H | (CH₂)ₙCOOR₆ | aryl |
| alkyl | COOH | H | (CH₂)ₙCOOR₆ | (CH₂)ₙCOOR₆ |
| alkyl | COOH | H | (CH₂)ₙCOOR₆ | (CH₂)ₙOR₆ |
| alkyl | COOH | H | (CH₂)ₙOR₆ | H |
| alkyl | COOH | H | (CH₂)ₙOR₆ | alkyl |
| alkyl | COOH | H | (CH₂)ₙOR₆ | aryl |
| alkyl | COOH | H | (CH₂)ₙOR₆ | (CH₂)ₙCOOR₆ |
| alkyl | COOH | H | (CH₂)ₙOR₆ | (CH₂)ₙOR₆ |
| alkyl | COOH | alkyl | (CH₂)ₙCOOR₆ | H |
| alkyl | COOH | alkyl | (CH₂)ₙCOOR₆ | alkyl |
| alkyl | COOH | alkyl | (CH₂)ₙCOOR₆ | aryl |
| alkyl | COOH | alkyl | (CH₂)ₙCOOR₆ | (CH₂)ₙCOOR₆ |
| alkyl | COOH | alkyl | (CH₂)ₙCOOR₆ | (CH₂)ₙOR₆ |
| alkyl | COOH | alkyl | (CH₂)ₙOR₆ | H |
| alkyl | COOH | alkyl | (CH₂)ₙOR₆ | alkyl |
| alkyl | COOH | alkyl | (CH₂)ₙOR₆ | aryl |
| alkyl | COOH | alkyl | (CH₂)ₙOR₆ | (CH₂)ₙCOOR₆ |
| alkyl | COOH | alkyl | (CH₂)ₙOR₆ | (CH₂)ₙOR₆ |
| alkyl | COOH | aryl | (CH₂)ₙCOOR₆ | H |
| alkyl | COOH | aryl | (CH₂)ₙCOOR₆ | alkyl |
| alkyl | COOH | aryl | (CH₂)ₙCOOR₆ | aryl |
| alkyl | COOH | aryl | (CH₂)ₙCOOR₆ | (CH₂)ₙCOOR₆ |
| alkyl | COOH | aryl | (CH₂)ₙCOOR₆ | (CH₂)ₙOR₆ |
| alkyl | COOH | aryl | (CH₂)ₙOR₆ | H |
| alkyl | COOH | aryl | (CH₂)ₙOR₆ | alkyl |
| alkyl | COOH | aryl | (CH₂)ₙOR₆ | aryl |
| alkyl | COOH | aryl | (CH₂)ₙOR₆ | (CH₂)ₙCOOR₆ |
| alkyl | COOH | aryl | (CH₂)ₙOR₆ | (CH₂)ₙOR₆ |
| alkyl | NHR₆ | H | (CH₂)ₙCOOR₆ | H |
| alkyl | NHR₆ | H | (CH₂)ₙCOOR₆ | alkyl |
| alkyl | NHR₆ | H | (CH₂)ₙCOOR₆ | aryl |
| alkyl | NHR₆ | H | (CH₂)ₙCOOR₆ | (CH₂)ₙCOOR₆ |
| alkyl | NHR₆ | H | (CH₂)ₙCOOR₆ | (CH₂)ₙOR₆ |
| alkyl | NHR₆ | H | (CH₂)ₙOR₆ | H |
| alkyl | NHR₆ | H | (CH₂)ₙOR₆ | alkyl |
| alkyl | NHR₆ | H | (CH₂)ₙOR₆ | aryl |
| alkyl | NHR₆ | H | (CH₂)ₙOR₆ | (CH₂)ₙCOOR₆ |
| alkyl | NHR₆ | H | (CH₂)ₙOR₆ | (CH₂)ₙOR₆ |
| alkyl | NHR₆ | alkyl | (CH₂)ₙCOOR₆ | H |
| alkyl | NHR₆ | alkyl | (CH₂)ₙCOOR₆ | alkyl |
| alkyl | NHR₆ | alkyl | (CH₂)ₙCOOR₆ | aryl |
| alkyl | NHR₆ | alkyl | (CH₂)ₙCOOR₆ | (CH₂)ₙCOOR₆ |
| alkyl | NHR₆ | alkyl | (CH₂)ₙCOOR₆ | (CH₂)ₙOR₆ |
| alkyl | NHR₆ | alkyl | (CH₂)ₙOR₆ | H |
| alkyl | NHR₆ | alkyl | (CH₂)ₙOR₆ | alkyl |
| alkyl | NHR₆ | alkyl | (CH₂)ₙOR₆ | aryl |
| alkyl | NHR₆ | alkyl | (CH₂)ₙOR₆ | (CH₂)ₙCOOR₆ |
| alkyl | NHR₆ | alkyl | (CH₂)ₙOR₆ | (CH₂)ₙOR₆ |
| alkyl | NHR₆ | aryl | (CH₂)ₙCOOR₆ | H |
| alkyl | NHR₆ | aryl | (CH₂)ₙCOOR₆ | alkyl |
| alkyl | NHR₆ | aryl | (CH₂)ₙCOOR₆ | aryl |
| alkyl | NHR₆ | aryl | (CH₂)ₙCOOR₆ | (CH₂)ₙCOOR₆ |
| alkyl | NHR₆ | aryl | (CH₂)ₙCOOR₆ | (CH₂)ₙOR₆ |
| alkyl | NHR₆ | aryl | (CH₂)ₙOR₆ | H |
| alkyl | NHR₆ | aryl | (CH₂)ₙOR₆ | alkyl |
| alkyl | NHR₆ | aryl | (CH₂)ₙOR₆ | aryl |
| alkyl | NHR₆ | aryl | (CH2)ₙOR₆ | (CH₂)ₙCOOR₆ |
| alkyl | NHR₆ | aryl | (CH₂)ₙOR₆ | (CH₂)ₙOR₆ |
| alkyl | (CH₂)ₙCOOR₆ | H | (CH₂)ₙCOOR₆ | H |
| alkyl | (CH₂)ₙCOOR₆ | H | (CH₂)ₙCOOR₆ | alkyl |
| alkyl | (CH₂)ₙCOOR₆ | H | (CH₂)ₙCOOR₆ | aryl |
| alkyl | (CH₂)ₙCOOR₆ | H | (CH₂)ₙCOOR₆ | (CH₂)ₙCOOR₆ |
| alkyl | (CH₂)ₙCOOR₆ | H | (CH₂)ₙCOOR₆ | (CH₂)ₙOR₆ |
| alkyl | (CH₂)ₙCOOR₆ | H | (CH₂)ₙOR₆ | H |
| alkyl | (CH₂)ₙCOOR₆ | H | (CH₂)ₙOR₆ | alkyl |
| alkyl | (CH₂)ₙCOOR₆ | H | (CH₂)ₙOR₆ | aryl |
| alkyl | (CH₂)ₙCOOR₆ | H | (CH₂)ₙOR₆ | (CH₂)ₙCOOR₆ |
| alkyl | (CH₂)ₙCOOR₆ | H | (CH₂)ₙOR₆ | (CH₂)ₙOR₆ |
| alkyl | (CH₂)ₙCOOR₆ | alkyl | (CH₂)ₙCOOR₆ | H |
| alkyl | (CH₂)ₙCOOR₆ | alkyl | (CH₂)ₙCOOR₆ | alkyl |
| alkyl | (CH₂)ₙCOOR₆ | alkyl | (CH₂)ₙCOOR₆ | aryl |
| alkyl | (CH₂)ₙCOOR₆ | alkyl | (CH₂)ₙCOOR₆ | (CH₂)ₙCOOR₆ |
| alkyl | (CH₂)ₙCOOR₆ | alkyl | (CH₂)ₙCOOR₆ | (CH₂)ₙOR₆ |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | alkyl |
| alkyl | (CH₂)ₙCOOR₆ | alkyl | (CH₂)ₙOR₆ | aryl |
| alkyl | (CH₂)ₙCOOR₆ | alkyl | (CH₂)ₙOR₆ | (CH₂)ₙCOOR₆ |
| alkyl | (CH₂)ₙCOOR₆ | alkyl | (CH₂)ₙCOR₆ | (CH₂)ₙOR₆ |
| alkyl | (CH₂)ₙCOOR₆ | aryl | (CH₂)ₙCOOR₆ | H |
| alkyl | (CH₂)ₙCOOR₆ | aryl | (CH₂)ₙCOOR₆ | alkyl |
| alkyl | (CH₂)ₙCOOR₆ | aryl | (CH₂)ₙCOOR₆ | aryl |
| alkyl | (CH₂)ₙCOOR₆ | aryl | (CH₂)ₙCOOR₆ | (CH₂)ₙCOOR₆ |
| alkyl | (CH₂)ₙCOOR₆ | aryl | (CH₂)ₙCOOR₆ | (CH₂)ₙOR₆ |
| alkyl | (CH₂)ₙCOOR₆ | aryl | (CH₂)ₙOR₆ | H |
| alkyl | (CH₂)ₙCOOR₆ | aryl | (CH₂)ₙOR₆ | alkyl |
| alkyl | (CH₂)ₙCOOR₆ | aryl | (CH₂)ₙOR₆ | aryl |
| alkyl | (CH₂)ₙCOOR₆ | aryl | (CH₂)ₙOR₆ | (CH₂)ₙCOOR₆ |
| alkyl | (CH₂)ₙCOOR₆ | aryl | (CH₂)ₙOR₆ | (CH₂)ₙOR₆ |
| aryl | H | H | (CH₂)ₙCOOR₆ | H |
| aryl | H | H | (CH₂)ₙCOOR₆ | alkyl |
| aryl | H | H | (CH₂)ₙCOOR₆ | aryl |
| aryl | H | H | (CH₂)ₙCOOR₆ | (CH₂)ₙCOOR₆ |
| aryl | H | H | (CH₂)ₙCOOR₆ | (CH₂)ₙOR₆ |
| aryl | H | H | (CH₂)ₙOR₆ | H |
| aryl | H | H | (CH₂)ₙOR₆ | alkyl |
| aryl | H | H | (CH₂)ₙOR₆ | aryl |
| aryl | H | H | (CH₂)ₙOR₆ | (CH₂)ₙCOOR₆ |
| aryl | H | H | (CH₂)ₙOR₆ | (CH₂)ₙOR₆ |
| aryl | H | alkyl | (CH₂)ₙCOOR₆ | H |
| aryl | H | alkyl | (CH₂)ₙCOOR₆ | alkyl |
| aryl | H | alkyl | (CH₂)ₙCOOR₆ | aryl |
| aryl | H | alkyl | (CH₂)ₙCOOR₆ | (CH₂)ₙCOOR₆ |
| aryl | H | alkyl | (CH₂)ₙCOOR₆ | (CH₂)ₙOR₆ |
| aryl | H | alkyl | (CH₂)ₙOR₆ | H |
| aryl | H | alkyl | (CH₂)ₙOR₆ | alkyl |
| aryl | H | alkyl | (CH₂)ₙOR₆ | aryl |
| aryl | H | alkyl | (CH₂)ₙOR₆ | (CH₂)ₙCOOR₆ |
| aryl | H | alkyl | (CH₂)ₙOR₆ | (CH₂)ₙOR₆ |
| aryl | H | aryl | (CH₂)ₙCOOR₆ | H |
| aryl | H | aryl | (CH₂)ₙCOOR₆ | alkyl |
| aryl | H | aryl | (CH₂)ₙCOOR₆ | aryl |
| aryl | H | aryl | (CH₂)ₙCOOR₆ | (CH₂)ₙCOOR₆ |
| aryl | H | aryl | (CH₂)ₙCOOR₆ | (CH₂)ₙOR₆ |
| aryl | H | aryl | (CH₂)ₙOR₆ | H |
| aryl | H | aryl | (CH₂)ₙOR₆ | alkyl |
| aryl | H | aryl | (CH₂)ₙOR₆ | aryl |
| aryl . | H | aryl | (CH₂)ₙOR₆ | (CH₂)ₙCOOR₆ |
| aryl | H | aryl | (CH₂)ₙOR₆ | (CH₂)ₙOR₆ |
| aryl | COOH | H | (CH₂)ₙCOOR₆ | H |
| aryl | COOH | H | (CHZ)nCOOR6 | alkyl |
| aryl | COOH | H | (CH₂)ₙCOOR₆ | aryl |
| aryl | COOH | H | (CH₂)ₙCOOR₆ | (CH₂)ₙCOOR₆ |
| aryl | COOH | H | (CH₂)ₙCOOR₆ | (CH₂)ₙOR₆ |
| aryl | COOH | H | (CH₂)ₙOR₆ | H |
| aryl | COOH | H | (CH₂)ₙOR₆ | alkyl |
| aryl | COOH | H | (CH₂)ₙOR₆ | aryl |
| aryl | COOH | H | (CH₂)ₙOR₆ | (CH₂)ₙCOOR₆ |
| aryl | COOH | H | (CH₂)ₙOR₆ | (CH₂)ₙOR₆ |
| aryl | COOH | alkyl | (CH₂)ₙCOOR₆ | H |
| aryl | COOH | alkyl | (CH₂)ₙCOOR₆ | alkyl |
| aryl | COOH | alkyl | (CH₂)ₙCOOR₆ | aryl |
| aryl | COOH | alkyl | (CH2)ₙCOOR₆ | (CH₂)ₙCOOR₆ |
| aryl | COOH | alkyl | (CH₂)ₙCOOR₆ | (CH₂)ₙOR₆ |
| aryl | COOH | alkyl | (CH₂)ₙOR₆ | H |
| aryl | COOH | alkyl | (CH₂)ₙOR₆ | alkyl |
| aryl | COOH | alkyl | (CH₂)ₙOR₆ | aryl |
| aryl | COOH | alkyl | (CH₂)ₙOR₆ | (CH₂)ₙCOOR₆ |
| aryl | COOH | alkyl | (CH₂)ₙOR₆ | (CH₂)ₙOR₆ |
| aryl | COOH | aryl | (CH₂)ₙCOOR₆ | H |
| aryl | COOH | aryl | (CH₂)ₙCOOR₆ | alkyl |
| aryl | COOH | aryl | (CH₂)ₙCOOR₆ | aryl |
| aryl | COOH | aryl | (CH₂)ₙCOOR₆ | (CH₂)ₙCOOR₆ |
| aryl | COOH | aryl | (CH₂)ₙCOOR₆ | (CH₂)ₙOR₆ |
| aryl | COOH | aryl | (CH₂)ₙOR₆ | H |
| aryl | COOH | aryl | (CH₂)ₙOR₆ | alkyl |
| aryl | COOH | aryl | (CH₂)ₙOR₆ | aryl |
| aryl | COOH | aryl | (CH₂)ₙOR₆ | (CH₂)ₙCOOR₆ |
| aryl | COOH | aryl | (CH₂)ₙOR₆ | (CH₂)ₙOR₆ |
| aryl | NHR₆ | H | (CH₂)ₙCOOR₆ | H |
| aryl | NHR₆ | H | (CH₂)ₙCOOR₆ | alkyl |
| aryl | NHR₆ | H | (CH₂)ₙCOOR₆ | aryl |
| aryl | NHR₆ | H | (CH₂)ₙCOOR₆ | (CH₂)ₙCOOR₆ |
| aryl | NHR₆ | H | (CH₂)ₙCOOR₆ | (CH₂)ₙOR₆ |
| aryl | NHR₆ | H | (CH₂)ₙOR₆ | H |
| aryl | NHR₆ | H | (CH₂)ₙOR₆ | alkyl |
| aryl | NHR₆ | H | (CH₂)ₙOR₆ | aryl |
| aryl | NHR₆ | H | (CH₂)ₙOR₆ | (CH₂)ₙCOOR₆ |
| aryl | NHR₆ | H | (CH₂)ₙOR₆ | (CH₂)ₙOR6 |
| aryl | NHR₆ | alkyl | (CH₂)ₙCOOR₆ | H |
| aryl | NHR₆ | alkyl | (CH₂)ₙCOOR₆ | alkyl |
| aryl | NHR₆ | alkyl | (CH₂)ₙCOOR₆ | aryl |
| aryl | NHR₆ | alkyl | (CH₂)ₙCOOR₆ | (CH₂)ₙCOOR₆ |
| aryl | NHR₆ | alkyl | (CH₂)ₙCOOR₆ | (CH₂)ₙOR₆ |
| aryl | NHR₆ | alkyl | (CH₂)ₙOR₆ | H |
| aryl | NHR₆ | alkyl | (CH₂)ₙOR₆ | alkyl |
| aryl | NHR₆ | alkyl | (CH₂)ₙOR₆ | aryl |
| aryl | NHR₆ | alkyl | (CH₂)ₙOR₆ | (CH₂)ₙCOOR₆ |
| aryl | NHR₆ | alkyl | (CH₂)ₙOR₆ | (CH₂)ₙOR₆ |
| aryl | NHR₆ | aryl | (CH₂)ₙCOOR₆ | H |
| aryl | NHR₆ | aryl | (CH₂)ₙCOOR₆ | alkyl |
| aryl | NHR₆ | aryl | (CH₂)ₙCOOR₆ | aryl |
| aryl | NHR₆ | aryl | (CH₂)ₙCOOR₆ | (CH₂)ₙCOOR₆ |
| aryl | NHR₆ | aryl | (CH₂)ₙCOOR₆ | (CH₂)ₙOR₆ |
| aryl | NHR₆ | aryl | (CH₂)ₙOR₆ | H |
| aryl | NHR₆ | aryl | (CH₂)ₙOR₆ | alkyl |
| aryl | NHR₆ | aryl | (CH₂)ₙOR₆ | aryl |
| aryl | NHR₆ | aryl | (CH₂)ₙOR₆ | (CH₂)ₙCOOR₆ |
| aryl | NHR₆ | aryl | (CH₂)ₙOR₆ | (CH₂)ₙOR₆ |
| aryl | (CH₂)ₙCOOR₆ | H | (CH₂)ₙCOOR₆ | H |
| aryl | (CH₂)ₙCOOR₆ | H | (CH₂)ₙCOOR₆ | alkyl |
| aryl | (CH₂)ₙCOOR₆ | H | (CH₂)ₙCOOR₆ | aryl |
| aryl | (CH₂)ₙCOOR₆ | H | (CH₂)ₙCOOR₆ | (CH₂)ₙCOOR₆ |
| aryl | (CH₂)ₙCOOR₆ | H | (CH₂)ₙCOOR₆ | (CH₂)ₙOR₆ |
| acryl | (CH₂)ₙCOOR₆ | H | (CH₂)ₙOR₆ | H |
| aryl | (CH₂)ₙCOOR₆ | H | (CH₂)ₙOR₆ | alkyl |
| aryl | (CH₂)ₙCOOR₆ | H | (CH₂)ₙOR₆ | aryl |
| aryl | (CH₂)ₙCOOR₆ | H | (CH₂)ₙOR₆ | (CH₂)ₙCOOR₆ |
| aryl | (CH₂)ₙCOOR₆ | H | (CH₂)ₙOR₆ | (CH₂)ₙOR₆ |
| aryl | (CH₂)ₙCOOR₆ | alkyl | (CH₂)ₙCOOR₆ | H |
| aryl | (CH₂)ₙCOOR₆ | alkyl | (CH₂)ₙCOOR₆ | alkyl |
| aryl | (CH₂)ₙCOOR₆ | alkyl | (CH₂)ₙCOOR₆ | aryl |
| aryl | (CH₂)ₙCOOR₆ | alkyl | (CH₂)ₙCOOR₆ | (CH₂)ₙCOOR₆ |
| aryl | (CH₂)ₙCOOR₆ | alkyl | (CH₂)ₙCOOR₆ | (CH₂)ₙOR₆ |
| aryl | (CH₂)ₙCOOR₆ | alkyl | (CH₂)ₙOR₆ | H |
| aryl | (CH₂)ₙCOOR₆ | alkyl | (CH₂)ₙOR₆ | alkyl |
| aryl | (CH₂)ₙCOOR₆ | alkyl | (CH₂)ₙOR₆ | aryl |
| aryl | (CH₂)ₙCOOR₆ | alkyl | (CH₂)ₙOR₆ | (CH₂)ₙCOOR₆ |
| aryl | (CH₂)ₙCOOR₆ | alkyl | (CH₂)ₙOR₆ | (CH₂)ₙOR₆ |
| aryl | (CH₂)ₙCOOR₆ | aryl | (CH₂)ₙCOOR₆ | H |
| aryl | (CH₂)ₙCOOR₆ | aryl | (CH₂)ₙCOOR₆ | alkyl |
| aryl | (CH₂)ₙCOOR₆ | aryl | (CH₂)ₙCOOR₆ | aryl |
| aryl | (CH₂)ₙCOOR₆ | aryl | (CH₂)ₙCOOR₆ | (CH₂)ₙCOOR₆ |
| aryl | (CH₂)ₙCOOR₆ | aryl | (CH₂)ₙCOOR₆ | (CH₂)ₙOR₆ |
| aryl | (CH₂)ₙCOOR₆ | aryl | (CH₂)ₙOR₆ | H |
| aryl | (CH₂)ₙCOOR₆ | aryl | (CH₂)ₙOR₆ | alkyl |
| aryl | (CH₂)ₙCOOR₆ | aryl | (CH₂)ₙOR₆ | aryl |
| aryl | (CH₂)ₙCOOR₆ | aryl | (CH₂)ₙOR₆ | (CH₂)ₙCOOR₆ |
| aryl | (CH₂)ₙCOOR₆ | aryl | (CH₂)ₙOR₆ | (CH₂)ₙOR₆ |

10. Chelating agent as claimed in claim 1, wherein X is NR₄ and Y is P(R₅)₂, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R5 are according to the combinations listed in claim 9.

11. Chelating agent as claimed in any of claims 1 to 8 wherein R₆ is a biomolecule.

12. Chelating agent as claimed in claim 11, wherein the biomolecule is selected from amino acids, peptides, proteins, oligonucleotides, polynucleotides, sugars.

13. Chelating agent as claimed in claim 12, wherein the biomolecule is selected from the group consisting of antibodies, ligands of tumor receptors.

14. Chelating agent as claimed in claim 12, wherein the biomolecule is selected from the group consisting of CCK, thioglucose, glucosamine, somatostatin, neurotensin, bombesin, CCK, annexin, interleukins, growth factors, steroid hormones and molecules binding to GPIIB/IIIa receptors.

15. Chelating agent as claimed in claim 12, wherein the biomolecule is selected from the group consisting of glucose, thioglucose, neurotransmitters.

16. Chelating agent as claimed in claim 12, wherein the biomolecule is an inhibitor of the tyrosine kinase activity, such as benzothiopyranones, anilinophthalimides, quinazolines, pyridopyrimidines and pyrrolopyrimidines.

17. Chelating agent of the following formula:

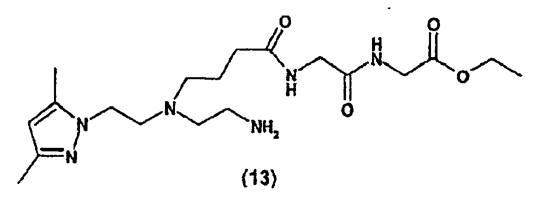

18. Chelating agent as claimed in claim 1 or claim 17, wherein the agent is complexed with a carbonyl moiety of the formula [M(CO)₃]⁺, wherein M is rhenium (Re), technetium (Tc) or manganese (Mn).

19. Chelating agent as claimed in claim 11, wherein the agent is complexed with a carbonyl moiety of the formula [M(CO)₃]⁺, wherein M is rhenium (Re) or technetium (Tc).

20. Method for the preparation of radiolabeled biomolecules comprising:
a) contacting a chelating agent as claimed in claim 1 with a carbonyl moiety of the formula [M(CO)₃]⁺, wherein M is rhenium (Re) or technetium (Tc), under conditions for forming a chelator-carbonyl complex; and
b) contacting the complex with a biomolecule for obtaining a radiolabeled biomolecule.

21. Kit suitable for performing the method as claimed in claim 20, comprising a first vial with the chelating agent of claim 1, optionally a first reaction vial for contacting the chelating agent with the carbonyl moiety of the formula [M(CO)₃]⁺, wherein M is Re or Tc, a second vial with the biomolecule and optionally a second reaction vial for reacting the biomolecule with the chelator-carbonyl complex obtained in the first step of the reaction.

22. Method for the preparation of radiolabeled biomolecules comprising:
a) contacting a chelating agent as claimed in claim 1 with a biomolecule for obtaining a chelator-biomolecule;
and
b) contacting the chelator-biomolecule with a carbonyl moiety of the formula [M(CO)₃]⁺, wherein M is rhenium (Re) or technetium (Tc), under conditions for forming a radiolabeled biomolecule.

23. Kit suitable for performing the method as claimed in claim 22, comprising a first vial with the chelating agent of claim 1, optionally a first reaction vial for reacting the chelating agent with the biomolecule, a second vial with the carbonyl moiety of the formula [M(CO)₃]⁺, wherein M is Re or Tc, and optionally a second reaction vial for reacting the chelator-biomolecule obtained in the first step of the reaction with the carbonyl.

24. Chelating agent as claimed in claim 1 or claim 17, for use in the preparation of a diagnostic or therapeutic agent for diagnosing or treating tumors.

25. Chelating agent as claimed in claim 18 for use as a diagnostic or therapeutic agent for diagnosing or treating tumors.

26. Use of a chelating agent as claimed in claim 1 or claim 17, for the preparation of a diagnostic or therapeutic agent for diagnosing or treating tumors.

27. Use of a chelating agent as claimed in claim 18, for the preparation of a diagnostic or therapeutic agent for diagnosing or treating tumours.

## Patentansprüche

1. Komplexbildner der allgemeinen Formel: 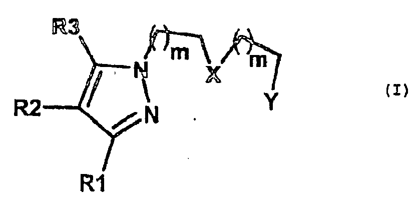 wobei m 0 oder 1 ist;
X NR₄ ist;
Y NHR₅, SR₅ oder P(R₅)₂ ist;
R₁ und R₃ gleich oder verschieden sind und aus H, Alkyl oder Aryl ausgewählt sind;
R₂ H, COOH, NHR₆ oder (CH₂)ₙCOOR₆ ist;
R₄ (CH₂)ₙCOOR₆ oder (CH₂)ₙOR₆ ist;
R₅ H, Alkyl, Aryl, (CH₂)ₙCOOR₆ oder (CH₂)ₙOR₆ ist;
R₆ H, Alkyl oder Aryl oder ein Biomolekül ist; und
n 1, 2, 3, 4, 5, 6, 7, 8, 9 oder 10 ist.

2. Komplexbildner nach Anspruch 1, wobei das Alkyl C₁-Alkyl, C₂-Alkyl, C₃-Alkyl, C₄-Alkyl, C₅-Alkyl oder C₆-Alkyl ist.

3. Komplexbildner nach Anspruch 2, wobei das Alkyl Methyl, Ethyl, *n*-Propyl, Isopropyl, *n*-Butyl, Isobutyl, *s*-Butyl, *t*-Butyl, *n*-Pentyl, Isopentyl, Neopentyl, *n-*Hexyl, Isohexyl (2-Methylpentyl), Neohexyl (2,2-Dimethylbutyl), 3-Methylpentyl, 2,3-Dimethylbutyl ist.

4. Komplexbildner nach Anspruch 1, wobei das Aryl monozyklisch, bevorzugt Phenyl oder Benzyl, oder polyzyklisch, C₁₀-C₁₈, und gegebenenfalls mit Alkyl-, Carboxy-, Oxo-, Amino-, Alkoxy- oder Aldehydgruppen substituiert ist.

5. Komplexbildner nach Anspruch 4, wobei Aryl Phenyl oder Benzyl ist.

6. Komplexbildner nach Anspruch 1, wobei n 2, 3, 4, 5 oder 6 und bevorzugt 2, 3 oder 4 ist.

7. Komplexbildner nach Anspruch 1, wobei der Komplexbildner ein Pyrazolylpolyamin der allgemeinen Formel ist: 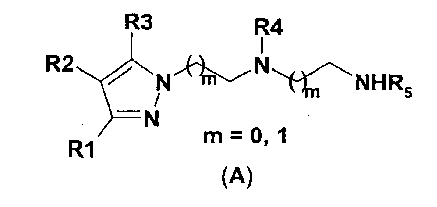 wobei R₁, R₂, R₃, R₄ und R₅ wie in Anspruch 1 definiert sind.

8. Komplexbildner nach Anspruch 1, wobei der Komplexbildner ein Pyrazolylaminophosphin der allgemeinen Formel ist: 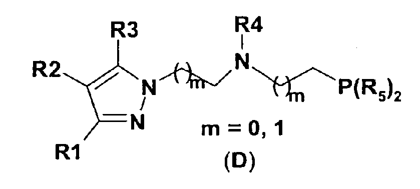 wobei R₁, R₂, R₃, R₄ und R₅ wie in Anspruch 1 definiert sind.

9. Komplexbildner nach Anspruch 1, wobei X NR₄ ist und Y NHR₅ ist, R₆ H, C₁-Alkyl, C₂-Alkyl, C₃-Alkyl, C₄-Alkyl, C₅-Alkyl oder C₆-Alkyl, Phenyl, Benzyl oder ein Biomolekül ist und R₁, R₂, R₃, R₄ und R₅ sind wie in den folgenden Kombinationen aufgelistet:
| **R1** | **R2** | **R3** | **R4** | **R5** |
|---|---|---|---|---|
| H | H | H | (CH₂)ₙCOOR₆ | H |
| H | H | H | (CH₂)ₙCOOR₆ | Alkyl |
| H | H | H | (CH₂)ₙCOOR₆ | Aryl |
| H | H | H | (CH₂)ₙCOOR₆ | (CH₂)ₙCOOR₆ |
| H | H | H | (CH₂)ₙCOOR₆ | (CH₂)ₙOR₆ |
| H | H | H | (CH₂)ₙOR₆ | H |
| H | H | H | (CH₂)ₙOR₆ | Alkyl, |
| H | H | H | (CH₂)ₙOR₆ | Aryl |
| H | H | H | (CH₂)ₙOR₆ | (CH₂)ₙCOOR₆ |
| H | H | H | (CH₂)ₙOR₆ | (CH₂)ₙOR₆ |
| H | H | Alkyl | (CH₂)ₙCOOR₆ | H |
| H | H | Alkyl | (CH₂)ₙCOOR₆ | Alkyl |
| H | H | Alkyl | (CH₂)ₙCOOR₆ | Aryl |
| H | H | Alkyl | (CH₂)ₙCOOR₆ | (CH₂)ₙCOOR₆ |
| H | H | Alkyl | (CH₂)ₙCOOR₆ | (CH₂)ₙOR₆ |
| H | H | Alkyl | (CH₂)ₙOR₆ | H |
| H | H | Alkyl | (CH₂)ₙOR₆ | Alkyl |
| H | H | Alkyl | (CH₂)ₙOR₆ | Aryl |
| H | H | Alkyl | (CH₂)ₙOR₆ | (CH₂)ₙCOOR₆ |
| H | H | Alkyl | (CH₂)ₙOR₆ | (CH₂)ₙOR₆ |
| H | H | Aryl | (CH₂)ₙCOOR₆ | H |
| H | H | Aryl | (CH₂)ₙCOOR₆ | Alkyl. |
| H | H | Aryl | (CH₂)ₙCOOR₆ | Aryl |
| H | H | Aryl | (CH₂)ₙCOOR₆ | (CH₂)ₙCOOR₆ |
| H | H | Aryl | (CH₂)ₙCOOR₆ | (CH₂)ₙOR₆ |
| H | H | Aryl | (CH₂)ₙOR₆ | H |
| H | H | Aryl | (CH₂)ₙOR₆ | Alkyl |
| H | H | Aryl | (CH₂)ₙOR₆ | Aryl |
| H | H | Aryl | (CH₂)ₙOR₆ | (CH₂)ₙCOOR₆ |
| H | H | Aryl | (CH₂)ₙOR₆ | (CH₂)ₙOR₆ |
| H | COOH | H | (CH₂)ₙCOOR₆ | H |
| H | COOH | H | (CH₂)ₙCOOR₆ | Alkyl |
| H | COOH | H | (CH₂)ₙCOOR₆ | Aryl |
| H | COOH | H | (CH₂)ₙCOOR₆ | (CH₂)ₙCOOR₆ |
| H | COOH | H | (CH₂)ₙCOOR₆ | (CH₂)ₙOR₆ |
| H | COOH | H | (CH₂)ₙOR₆ | H |
| H | COOH | H | (CH₂)ₙOR₆ | Alkyl |
| H | COOH | H | (CH₂)ₙOR₆ | Aryl |
| H | COOH | H | (CH₂)ₙOR₆ | (CH₂)ₙCOOR₆ |
| H | COOH | H | (CH₂)ₙOR₆ | (CH₂)ₙOR₆ |
| H | COOH | Alkyl | (CH₂)ₙCOOR₆ | H |
| H | COOH | Alkyl | (CH₂)ₙCOOR₆ | Alkyl. |
| H | COOH | Alkyl | (CH₂)ₙCOOR₆ | Aryl |
| H | COOH | Alkyl | (CH₂)ₙCOOR₆ | (CH₂)ₙCOOR₆ |
| H | COOH | Alkyl | (CH₂)ₙCOOR₆ | (CH₂)ₙOR₆ |
| H | COOH | Alkyl | (CH₂)ₙOR₆ | H |
| H | COOH | Alkyl | (CH₂)ₙOR₆ | Alkyl |
| H | COOH | Alkyl | (CH₂)ₙOR₆ | Aryl |
| H | COOH | Alkyl | (CH₂)ₙOR₆ | (CH₂)ₙCOOR₆ |
| H | COOH | Alkyl | (CH₂)ₙOR₆ | (CH₂)ₙOR₆ |
| H | COOH | Aryl | (CH₂)ₙCOOR₆ | H |
| H | COOH | Aryl | (CH₂)ₙCOOR₆ | Alkyl |
| H | COOH | Aryl | (CH₂)ₙCOOR₆ | Aryl |
| H | COOH | Aryl | (CH₂)ₙCOOR₆ | (CH₂)ₙCOOR₆ |
| H | COOH | Aryl | (CH₂)ₙCOOR₆ | (CH₂)ₙOR₆ |
| H | COOH | Aryl | (CH₂)ₙOR₆ | H |
| H | COOH | Aryl | (CH₂)ₙOR₆ | Alkyl |
| H | COOH | Aryl | (CH₂)ₙOR₆ | Aryl |
| H | COOH | Aryl | (CH₂)ₙOR₆ | (CH₂)ₙCOOR₆ |
| H | COOH | Aryl | (CH₂)ₙOR₆ | (CH₂)ₙOR₆ |
| H | NHR₆ | H | (CH₂)ₙCOOR₆ | H |
| H | NHR₆ | H | (CH₂)ₙCOOR₆ | Alkyl |
| H | NHR₆ | H | (CH₂)ₙCOOR₆ | Aryl |
| H | NHR₆ | H | (CH₂)ₙCOOR₆ | (CH₂)ₙCOOR₆ |
| H | NHR₆ | H | (CH₂)ₙCOOR₆ | (CH₂)ₙOR₆ |
| H | NHR₆ | H | (CH₂)ₙOR₆ | H |
| H | NHR₆ | H | (CH₂)ₙOR₆ | Alkyl |
| H | NHR₆ | H | (CH₂)ₙOR₆ | Aryl |
| H | NHR₆ | H | (CH₂)ₙOR₆ | (CH₂)ₙCOOR₆ |
| H | NHR₆ | H | (CH₂)ₙOR₆ | (CH₂)ₙOR₆ |
| H | NHR₆ | Alkyl | (CH₂)ₙCOOR₆ | H |
| H | NHR₆ | Alkyl | (CH₂)ₙCOOR₆ | Alkyl |
| H | NHR₆ | Alkyl | (CH₂)ₙCOOR₆ | Aryl |
| H | NHR₆ | Alkyl | (CH₂)ₙCOOR₆ | (CH₂)ₙCOOR₆ |
| H | NHR₆ | Alkyl | (CH₂)ₙCOOR₆ | (CH₂)ₙOR₆ |
| H | NHR₆ | Alkyl | (CH₂)ₙOR₆ | H |
| H | NHR₆ | Alkyl | (CH₂)ₙOR₆ | Alkyl |
| H | NHR₆ | Alkyl | (CH₂)ₙOR₆ | Aryl |
| H | NHR₆ | Alkyl | (CH₂)ₙOR₆ | (CH₂)ₙCOOR₆ |
| H | NHR₆ | Alkyl | (CH₂)ₙOR₆ | (CH₂)ₙOR₆ |
| H | NHR₆ | Aryl | (CH₂)ₙCOOR₆ | H |
| H | NHR₆ | Aryl | (CH₂)ₙCOOR₆ | Alkyl |
| H | NHR₆ | Aryl | (CH₂)ₙCOOR₆ | Aryl |
| H | NHR₆ | Aryl | (CH₂)ₙCOOR₆ | (CH₂)ₙCOOR₆ |
| H | NHR₆ | Aryl | (CH₂)ₙCOOR₆ | (CH₂)ₙOR₆ |
| H | NHR₆ | Aryl | (CH₂)ₙOR₆ | H |
| H | NHR₆ | Aryl | (CH₂)ₙOR₆ | Alkyl |
| H | NHR₆ | Aryl | (CH₂)ₙOR₆ | Aryl |
| H | NHR₆ | Aryl | (CH₂)ₙOR₆ | (CH₂)ₙCOOR₆ |
| H | NHR₆ | Aryl | (CH₂)ₙOR₆ | (CH₂)ₙOR₆ |
| H | (CH₂)ₙCOOR₆ | H | (CH₂)ₙCOOR₆ | H |
| H | (CH₂)ₙCOOR₆ | H | (CH₂)ₙCOOR₆ | Alkyl |
| H | (CH₂)ₙCOOR₆ | H | (CH₂)ₙCOOR₆ | Aryl |
| H | (CH₂)ₙCOOR₆ | H | (CH₂)ₙCOOR₆ | ( CH₂ ) ₙCOOR₆ |
| H | (CH₂)ₙCOOR₆ | H | (CH₂)ₙCOOR₆ | (CH₂) ₙOR₆ |
| H | (CH₂)ₙCOOR₆ | H | (CH₂)ₙOR₆ | H |
| H | (CH₂)ₙCOOR₆ | H | (CH₂)ₙOR₆ | Alkyl |
| H | (CH₂)ₙCOOR₆ | H | (CH₂)ₙOR₆ | Aryl |
| H | (CH₂)ₙCOOR₆ | H | (CH₂)ₙOR₆ | (CH₂)ₙCOOR₆ |
| H | (CH₂)ₙCOOR₆ | H | (CH₂)ₙOR₆ | (CH₂)ₙOR₆ |
| H | (CH₂)ₙCOOR₆ | Alkyl | (CH₂)ₙCOOR₆ | H |
| H | (CH₂)ₙCOOR₆ | Alkyl | (CH₂)ₙCOOR₆ | Alkyl |
| H | (CH₂)ₙCOOR₆ | Alkyl | (CH₂)ₙCOOR₆ | Aryl |
| H | (CH₂)ₙCOOR₆ | Alkyl | (CH₂)ₙCOOR₆ | (CH₂)ₙCOOR₆ |
| H | (CH₂)ₙCOOR₆ | Alkyl | (CH₂)ₙCOOR₆ | (CH₂)ₙOR₆ |
| H | (CH₂)ₙCOOR₆ | Alkyl | (CH₂)ₙOR₆ | H |
| H | (CH₂)ₙCOOR₆ | Alkyl | (CH₂)ₙOR₆ | Alkyl |
| H | (CH₂)ₙCOOR₆ | Alkyl | (CH₂)ₙOR₆ | Aryl |
| H | (CH₂)ₙCOOR₆ | Alkyl | (CH₂)ₙOR₆ | (CH₂)ₙCOOR₆ |
| H | (CH₂)ₙCOOR₆ | Alkyl | (CH₂)ₙOR₆ | (CH₂)ₙOR₆ |
| H | (CH₂)ₙCOOR₆ | Aryl | (CH₂)ₙCOOR₆ | H |
| H | (CH₂)ₙCOOR₆ | Aryl | (CH₂)ₙCOOR₆ | Alkyl |
| H | (CH₂)ₙCOOR₆ | Aryl | (CH₂)ₙCOOR₆ | Aryl |
| H | (CH₂)ₙCOOR₆ | Aryl | (CH₂)ₙCOOR₆ | (CH₂)ₙCOOR₆ |
| H | (CH₂)ₙCOOR₆ | Aryl | (CH₂)ₙCOOR₆ | (CH₂)ₙOR₆ |
| H | (CH₂)ₙCOOR₆ | Aryl | (CH₂)ₙOR₆ | H |
| H | (CH₂)ₙCOOR₆ | Aryl | (CH₂)ₙOR₆ | Alkyl |
| H | (CH₂)ₙCOOR₆ | Aryl | (CH₂)ₙOR₆ | Aryl |
| H | (CH₂)ₙCOOR₆ | Aryl | (CH₂)ₙOR₆ | (CH₂)ₙCOOR₆ |
| H | (CH₂)ₙCOOR₆ | Aryl | (CH₂)ₙOR₆ | (CH₂)ₙOR₆ |
| Alkyl | H | H | (CH₂)ₙCOOR₆ | H |
| Alkyl | H | H | (CH₂)ₙCOOR₆ | Alkyl |
| Alkyl | H | H | (CH₂)ₙCOOR₆ | Aryl |
| Alkyl | H | H | (CH₂)ₙCOOR₆ | (CH₂)ₙCOOR₆ |
| Alkyl | H | H | (CH₂)nCOOR₆ | (CH₂)ₙOR₆ |
| Alkyl | H | H | (CH₂)ₙOR₆ | H |
| Alkyl | H | H | (CH₂)ₙOR₆ | Alkyl |
| Alkyl | H | H | (CH₂)ₙOR₆ | Aryl |
| Alkyl | H | H | (CH₂)ₙOR₆ | (CH₂)ₙCOOR₆ |
| Alkyl | H | H | (CH₂)ₙOR₆ | (CH₂)ₙOR₆ |
| Alkyl | H | Alkyl | (CH₂)ₙCOOR₆ | H |
| Alkyl | H | Alkyl | (CH₂)ₙCOOR₆ | Alkyl |
| Alkyl | H | Alkyl | (CH₂)ₙCOOR₆ | Aryl |
| Alkyl | H | Alkyl | (CH₂)ₙCOOR₆ | (CH₂)ₙCOOR₆ |
| Alkyl | H | Alkyl | (CH₂)ₙCOOR₆ | (CH₂)ₙOR₆ |
| Alkyl | H | Alkyl | (CH₂)ₙOR₆ | H |
| Alkyl | H | Alkyl | (CH₂)ₙOR₆ | Alkyl |
| Alkyl | H | Alkyl | (CH₂)ₙOR₆ | Aryl |
| Alkyl | H | Alkyl | (CH₂)ₙOR₆ | (CH₂)ₙCOOR₆ |
| Alkyl | H | Alkyl | (CH₂)ₙOR₆ | (CH₂)ₙOR₆ |
| Alkyl | H | Aryl | (CH₂)ₙCOOR₆ | H |
| Alkyl | H | Aryl | (CH₂)ₙCOOR₆ | Alkyl |
| Alkyl | H | Aryl | (CH₂)ₙCOOR₆ | Aryl |
| Alkyl | H | Aryl | (CH₂)ₙCOOR₆ | (CH₂)ₙCOOR₆ |
| Alkyl | H | Aryl | (CH₂)ₙCOOR₆ | (CH₂)ₙOR₆ |
| Alkyl | H | Aryl | (CH₂)ₙOR₆ | H |
| Alkyl | H | Aryl | (CH₂)ₙOR₆ | Alkyl |
| Alkyl | H | Aryl | (CH₂)ₙOR₆ | Aryl |
| Alkyl | H | Aryl | (CH₂)ₙOR₆ | (CH₂)ₙCOOR₆ |
| Alkyl | H | Aryl | (CH₂)ₙOR₆ | (CH₂)ₙOR₆ |
| Alkyl | COOH | H | (CH₂)ₙCOOR₆ | H |
| Alkyl | COOH | H | (CH₂)ₙCOOR₆ | Alkyl |
| Alkyl | COOH | H | (CH₂)ₙCOOR₆ | Aryl |
| Alkyl | COOH | H | (CH₂)ₙCOOR₆ | (CH₂)ₙCOOR₆ |
| Alkyl | COOH | H | (CH₂)ₙCOOR₆ | (CH₂)ₙOR₆ |
| Alkyl | COOH | H | (CH₂)ₙOR₆ | H |
| Alkyl | COOH | H | (CH₂)ₙOR₆ | Alkyl |
| Alkyl | COOH | H | (CH₂)ₙOR₆ | Aryl |
| Alkyl | COOH | H | (CH₂)ₙOR₆ | (CH₂)ₙCOOR₆ |
| Alkyl | COOH | H | (CH₂)ₙOR₆ | (CH₂)ₙOR₆ |
| Alkyl | COOH | Alkyl | (CH₂)ₙCOOR₆ | H |
| Alkyl | COOH | Alkyl | (CH₂)ₙCOOR₆ | Alkyl |
| Alkyl | COOH | Alkyl | (CH₂)ₙCOOR₆ | Aryl |
| Alkyl | COOH | Alkyl | (CH₂)ₙCOOR₆ | (CH₂)ₙCOOR₆ |
| Alkyl | COOH | Alkyl | (CH₂)ₙCOOR₆ | (CH₂)ₙOR₆ |
| Alkyl | COOH | Alkyl | (CH₂)ₙOR₆ | H |
| Alkyl | COOH | Alkyl | (CH₂)ₙOR₆ | Alkyl |
| Alkyl | COOH | Alkyl | (CH₂)ₙOR₆ | Aryl |
| Alkyl | COOH | Alkyl | (CH₂)ₙOR₆ | (CH₂)ₙCOOR₆ |
| Alkyl | COOH | Alkyl | (CH₂)ₙOR₆ | (CH₂)ₙOR₆ |
| Alkyl | COOH | Aryl | (CH₂)ₙCOOR₆ | H |
| Alkyl | COOH | Aryl | (CH₂)ₙCOOR₆ | Alkyl |
| Alkyl | COOH | Aryl | (CH₂)ₙCOOR₆ | Aryl |
| Alkyl | COOH | Aryl | (CH₂)ₙCOOR₆ | (CH₂)ₙCOOR₆ |
| Alkyl | COOH | Aryl | (CH₂)ₙCOOR₆ | (CH₂)ₙOR₆ |
| Alkyl | COOH | Aryl | (CH₂)ₙOR₆ | H |
| Alkyl | COOH | Aryl | (CH₂)ₙOR₆ | Alkyl |
| Alkyl | COOH | Aryl | (CH₂)ₙOR₆ | Aryl |
| Alkyl | COOH | Aryl | (CH₂)ₙOR₆ | (CH₂)ₙCOOR₆ |
| Alkyl | COOH | Aryl | (CH₂)ₙOR₆ | (CH₂)ₙOR₆ |
| Alkyl | NHR₆ | H | (CH₂)ₙCOOR₆ | H |
| Alkyl | NHR₆ | H | (CH₂)ₙCOOR₆ | Alkyl |
| Alkyl | NHR₆ | H | (CH₂)ₙCOOR₆ | Aryl |
| Alkyl | NHR₆ | H | (CH₂)ₙCOOR₆ | (CH₂)ₙCOOR₆ |
| Alkyl | NHR₆ | H | (CH₂)ₙCOOR₆ | (CH₂)ₙOR₆ |
| Alkyl | NHR₆ | H | (CH₂)ₙOR₆ | H |
| Alkyl | NHR₆ | H | (CH₂)ₙOR₆ | Alkyl |
| Alkyl | NHR₆ | H | (CH₂)ₙOR₆ | Aryl |
| Alkyl | NHR₆ | H | (CH₂)ₙOR₆ | (CH₂)ₙCOOR₆ |
| Alkyl | NHR₆ | H | (CH₂)ₙOR₆ | (CH₂)ₙOR₆ |
| Alkyl | NHR₆ | Alkyl | (CH₂)ₙCOOR₆ | H |
| Alkyl | NHR₆ | Alkyl | (CH₂)ₙCOOR₆ | Alkyl |
| Alkyl | NHR₆ | Alkyl | (CH₂)ₙCOOR₆ | Aryl |
| Alkyl | NHR₆ | Alkyl | (CH₂)ₙCOOR₆ | (CH₂)ₙCOOR₆ |
| Alkyl | NHR₆ | Alkyl | (CH₂)ₙCOOR₆ | (CH₂)ₙOR₆ |
| Alkyl | NHR₆ | Alkyl | (CH₂)ₙOR₆ | H |
| Alkyl | NHR₆ | Alkyl | (CH₂)ₙOR₆ | Alkyl |
| Alkyl | NHR₆ | Alkyl | (CH₂)ₙOR₆ | Aryl |
| Alkyl | NHR₆ | Alkyl | (CH₂)ₙOR₆ | (CH₂)ₙCOOR₆ |
| Alkyl | NHR₆ | Alkyl | (CH₂)ₙOR₆ | (CH₂)ₙOR₆ |
| Alkyl | NHR₆ | Aryl | (CH₂)ₙCOOR₆ | H |
| Alkyl | NHR₆ | Aryl | (CH₂)ₙCOOR₆ | Alkyl |
| Alkyl | NHR₆ | Aryl | (CH₂)ₙCOOR₆ | Aryl |
| Alkyl | NHR₆ | Aryl | (CH₂)ₙCOOR₆ | (CH₂)ₙCOOR₆ |
| Alkyl | NHR₆ | Aryl | (CH₂)ₙCOOR₆ | (CH₂)ₙOR₆ |
| Alkyl | NHR₆ | Aryl | (CH₂)ₙOR₆ | H |
| Alkyl | NHR₆ | Aryl | (CH₂)ₙOR₆ | Alkyl |
| Alkyl | NHR₆ | Aryl | (CH₂)ₙOR₆ | Aryl |
| Alkyl | NHR₆ | Aryl | (CH₂)nOR₆ | (CH₂)ₙCOOR₆ |
| Alkyl | NHR₆ | Aryl | (CH₂)ₙOR₆ | (CH₂)ₙOR₆ |
| Alkyl | (CH₂)ₙCOOR₆ | H | (CH₂)ₙCOOR₆ | H |
| Alkyl | (CH₂)ₙCOOR₆ | H | (CH₂)ₙCOOR₆ | Alkyl |
| Alkyl | (CH₂)ₙCOOR₆ | H | (CH₂)ₙCOOR₆ | Aryl |
| Alkyl | (CH₂)ₙCOOR₆ | H | (CH₂)ₙCOOR₆ | (CH₂)ₙCOOR₆ |
| Alkyl | (CH₂)ₙCOOR₆ | H | (CH₂)ₙCOOR₆ | (CH₂)ₙOR₆ |
| Alkyl | (CH₂)ₙCOOR₆ | H | (CH₂)ₙOR₆ | H |
| Alkyl | (CH₂)ₙCOOR₆ | H | (CH₂)ₙOR₆ | Alkyl |
| Alkyl | (CH₂)ₙCOOR₆ | H | (CH₂)ₙOR₆ | Aryl |
| Alkyl | (CH₂)ₙCOOR₆ | H | (CH₂)ₙOR₆ | (CH₂)ₙCOOR₆ |
| Alkyl | (CH₂)ₙCOOR₆ | H | (CH₂)ₙOR₆ | (CH₂)ₙOR₆ |
| Alkyl | (CH₂)ₙCOOR₆ | Alkyl | (CH₂)ₙCOOR₆ | H |
| Alkyl | (CH₂)ₙCOOR₆ | Alkyl | (CH₂)ₙCOOR₆ | Alkyl |
| Alkyl | (CH₂)ₙCOOR₆ | Alkyl | (CH₂)ₙCOOR₆ | Aryl |
| Alkyl | (CH₂)ₙCOOR₆ | Alkyl | (CH₂)ₙCOOR₆ | (CH₂)ₙCOOR₆ |
| Alkyl | (CH₂)ₙCOOR₆ | Alkyl | (CH₂)ₙCOOR₆ | (CH₂)ₙOR₆ |
| Alkyl | (CH₂)ₙCOOR₆ | Alkyl | (CH₂)ₙOR₆ | H |
| Alkyl | (CH₂)ₙCOOR₆ | Alkyl | (CH₂)ₙOR₆ | H |
| Alkyl | (CH₂)ₙCOOR₆ | Alkyl | (CH₂)ₙOR₆ | Alkyl |
| Alkyl | (CH₂)ₙCOOR₆ | Alkyl | (CH₂)ₙOR₆ | Aryl |
| Alkyl | (CH₂)ₙCOOR₆ | Alkyl | (CH₂)ₙOR₆ | (CH₂)ₙCOOR₆ |
| Alkyl | (CH₂)ₙCOOR₆ | Alkyl | (CH₂)ₙCOR₆ | (CH₂)ₙOR₆ |
| Alkyl | (CH₂)ₙCOOR₆ | Aryl | (CH₂)ₙCOOR₆ | H |
| Alkyl | (CH₂)ₙCOOR₆ | Aryl | (CH₂)ₙCOOR₆ | Alkyl |
| Alkyl | (CH₂)ₙCOOR₆ | Aryl | (CH₂)ₙCOOR₆ | Aryl |
| Alkyl | (CH₂)ₙCOOR₆ | Aryl | (CH₂)ₙCOOR₆ | (CH₂)ₙCOOR₆ |
| Alkyl | (CH₂)ₙCOOR₆ | Aryl | (CH₂)ₙCOOR₆ | (CH₂)ₙOR₆ |
| Alkyl | (CH₂)ₙCOOR₆ | Aryl | (CH₂)ₙOR₆ | H |
| Alkyl | (CH₂)ₙCOOR₆ | Aryl | (CH₂)ₙOR₆ | Alkyl |
| Alkyl | (CH₂)ₙCOOR₆ | Aryl | (CH₂)ₙOR₆ | Aryl |
| Alkyl | (CH₂)ₙCOOR₆ | Aryl | (CH₂)ₙOR₆ | (CH₂)ₙCOOR₆ |
| Alkyl | (CH₂)ₙCOOR₆ | Aryl | (CH₂)ₙOR₆ | (CH₂)ₙOR₆ |
| Aryl | H | H | (CH₂)ₙCOOR₆ | H |
| Aryl | H | H | (CH₂)ₙCOOR₆ | Alkyl |
| Aryl | H | H | (CH₂)ₙCOOR₆ | Aryl |
| Aryl | H | H | (CH₂)ₙCOOR₆ | (CH₂)ₙCOOR₆ |
| Aryl | H | H | (CH₂)ₙCOOR₆ | (CH₂)ₙOR₆ |
| Aryl | H | H | (CH₂)ₙOR₆ | H |
| Aryl | H | H | (CH₂)ₙOR₆ | Alkyl |
| Aryl | H | H | (CH₂)ₙOR₆ | Aryl |
| Aryl | H | H | CH₂)ₙOR₆ | (CH₂)ₙCOOR₆ |
| Aryl | H | H | (CH₂)ₙOR₆ | (CH₂)ₙOR₆ |
| Aryl | H | Alkyl | (CH₂)ₙCOOR₆ | H |
| Aryl | H | Alkyl | (CH₂)ₙCOOR₆ | Alkyl |
| Aryl | H | Alkyl | (CH₂)ₙCOOR₆ | Aryl |
| Aryl | H | Alkyl | (CH₂)ₙCOOR₆ | (CH₂)ₙCOOR₆ |
| Aryl | H | Alkyl | (CH₂)ₙCOOR₆ | (CH₂)ₙOR₆ |
| Aryl | H | Alkyl | (CH₂)ₙOR₆ | H |
| Aryl | H | Alkyl | (CH₂)ₙOR₆ | Alkyl |
| Aryl | H | Alkyl | (CH₂)ₙOR₆ | Aryl |
| Aryl | H | Alkyl | (CH₂)ₙOR₆ | (CH₂)ₙCOOR₆ |
| Aryl | H | Alkyl | (CH₂)ₙOR₆ | (CH₂)ₙOR₆ |
| Aryl | H | Aryl | (CH₂)ₙCOOR₆ | H |
| Aryl | H | Aryl | (CH₂)ₙCOOR₆ | Alkyl |
| Aryl | H | Aryl | (CH₂)ₙCOOR₆ | Aryl |
| Aryl | H | Aryl | (CH₂)ₙCOOR₆ | (CH₂)COOR₆ |
| Aryl | H | Aryl | (CH₂)ₙCOOR₆ | (CH₂)ₙOR₆ |
| Aryl | H | Aryl | (CH₂)ₙOR₆ | H |
| Aryl | H | Aryl | (CH₂)ₙOR₆ | Alkyl |
| Aryl | H | Aryl | (CH₂)OR₆ | Aryl |
| Aryl . | H | Aryl | (CH₂)OR₆ | (CH₂)ₙCOOR₆ |
| Aryl | H | Aryl | (CH₂)ₙOR₆ | (CH₂)ₙOR₆ |
| Aryl | COOH | H | (CH₂)ₙCOOR₆ | H |
| Aryl | COOH | H | (CH₂)ₙCOOR₆ | Alkyl |
| Aryl | COOH | H | (CH₂)ₙCOOR₆ | Aryl |
| Aryl | COOH | H | (CH₂)ₙCOOR₆ | (CH₂)ₙCOOR₆ |
| Aryl | COOH | H | (CH₂)ₙCOOR₆ | (CH₂)ₙOR₆ |
| Aryl | COOH | H | (CH₂)ₙOR₆ | H |
| Aryl | COOH | H | (CH₂)ₙOR₆ | Alkyl |
| Aryl | COOH | H | (CH₂)ₙOR₆ | Aryl |
| Aryl | COOH | H | (CH₂)ₙOR₆ | (CH₂)ₙCOOR₆ |
| Aryl | COOH | H | (CH₂)ₙOR₆ | (CH₂)ₙOR₆ |
| Aryl | COOH | Alkyl | (CH₂)ₙCOOR₆ | H |
| Aryl | COOH | Alkyl | (CH₂)ₙCOOR₆ | Alkyl |
| Aryl | COOH | Alkyl | (CH₂)ₙCOOR₆ | Aryl |
| Aryl | COOH | Alkyl | (CH₂)ₙCOOR₆ | (CH₂)ₙCOOR₆ |
| Aryl | COOH | Alkyl | (CH₂)ₙCOOR₆ | (CH₂)ₙOR₆ |
| Aryl | COOH | Alkyl | (CH₂)ₙOR₆ | H |
| Aryl | COOH | Alkyl | (CH₂)ₙOR₆ | Alkyl |
| Aryl | COOH | Alkyl | (CH₂)ₙOR₆ | Aryl |
| Aryl | COOH | Alkyl | (CH₂)ₙOR₆ | (CH₂)ₙCOOR₆ |
| Aryl | COOH | Alkyl | (CH₂)ₙOR₆ | (CH₂)ₙOR₆ |
| Aryl | COOH | Aryl | (CH₂)ₙCOOR₆ | H |
| Aryl | COOH | Aryl | (CH₂)ₙCOOR₆ | Alkyl |
| Aryl | COOH | Aryl | (CH₂)ₙCOOR₆ | Aryl |
| Aryl | COOH | Aryl | (CH₂)ₙCOOR₆ | (CH₂)ₙCOOR₆ |
| Aryl | COOH | Aryl | (CH₂)ₙCOOR₆ | (CH₂)ₙOR₆ |
| Aryl | COOH | Aryl | (CH₂)ₙOR₆ | H |
| Aryl | COOH | Aryl | (CH₂)ₙOR₆ | Alkyl |
| Aryl | COOH | Aryl | (CH₂)ₙOR₆ | Aryl |
| Aryl | COOH | Aryl | (CH₂)ₙOR₆ | (CH₂)ₙCOOR₆ |
| Aryl | COOH | Aryl | (CH₂)ₙOR₆ | (CH₂)ₙOR₆ |
| Aryl | NHR₆ | H | (CH₂)ₙCOOR₆ | H |
| Aryl | NHR₆ | H | (CH₂)ₙCOOR₆ | Alkyl |
| Aryl | NHR₆ | H | (CH₂)ₙCOOR₆ | Aryl |
| Aryl | NHR₆ | H | (CH₂)ₙCOOR₆ | (CH₂)ₙCOOR₆ |
| Aryl | NHR₆ | H | (CH₂)ₙCOOR₆ | (CH₂)ₙOR₆ |
| Aryl | NHR₆ | H | (CH₂)ₙOR₆ | H |
| Aryl | NHR₆ | H | (CH₂)ₙOR₆ | Alkyl |
| Aryl | NHR₆ | H | (CH₂)ₙOR₆ | Aryl |
| Aryl | NHR₆ | H | (CH₂)ₙOR₆ | (CH₂)ₙCOOR₆ |
| Aryl | NHR₆ | H | (CH₂)ₙOR₆ | (CH₂)ₙOR₆ |
| Aryl | NHR₆ | Alkyl | (CH₂)COOR₆ | H |
| Aryl | NHR₆ | Alkyl | (CH₂)ₙCOOR₆ | Alkyl |
| Aryl | NHR₆ | Alkyl | (CH₂)ₙCOOR₆ | Aryl |
| Aryl | NHR₆ | Alkyl | (CH₂)ₙCOOR₆ | (CH₂)ₙCOOR₆ |
| Aryl | NHR₆ | Alkyl | (CH₂)ₙCOOR₆ | (CH₂)ₙOR₆ |
| Aryl | NHR₆ | Alkyl | (CH₂)ₙOR₆ | H |
| Aryl | NHR₆ | Alkyl | (CH₂)ₙOR₆ | Alkyl |
| Aryl | NHR₆ | Alkyl | (CH₂)ₙOR₆ | Aryl |
| Aryl | NHR₆ | Alkyl | (CH₂)ₙOR₆ | (CH₂)ₙCOOR₆ |
| Aryl | NHR₆ | Alkyl | (CH₂)ₙOR₆ | (CH₂)ₙOR₆ |
| Aryl | NHR₆ | Aryl | (CH₂)ₙCOORₑ | H |
| Aryl | NHR₆ | Aryl | (CH₂)ₙCOOR₆ | Alkyl |
| Aryl | NHR₆ | Aryl | (CH₂)ₙCOOR₆ | Aryl |
| Aryl | NHR₆ | Aryl | (CH₂)ₙCOOR₆ | (CH₂)ₙCOOR₆ |
| Aryl | NHR₆ | Aryl | (CH₂)ₙCOOR₆ | (CH₂)ₙOR₆ |
| Aryl | NHR₆ | Aryl | (CH₂)ₙOR₆ | H |
| Aryl | NHR₆ | Aryl | (CH₂)ₙOR₆ | Alkyl |
| Aryl | NHR₆ | Aryl | (CH₂)ₙOR₆ | Aryl |
| Aryl | NHR₆ | Aryl | (CH₂)ₙOR₆ | (CH₂)ₙCOOR₆ |
| Aryl | NHR₆ | Aryl | (CH₂)ₙOR₆ | (CH₂)ₙOR₆ |
| Aryl | (CH₂)ₙCOOR₆ | H | (CH₂)ₙCOOR₆ | H |
| Aryl | (CH₇)ₙCOOR₆ | H | (CH₂)ₙCOOR₆ | Alkyl |
| Aryl | (CH₂)ₙCOOR₆ | H | (CH₂)ₙCOOR₆ | Aryl |
| Aryl | (CH₂)ₙCOOR₆ | H | (CH₂)ₙCOOR₆ | (CH₂)ₙCOOR₆ |
| Aryl | (CH₂)ₙCOOR₆ | H | (CH₂)ₙCOOR₆ | (CH₂)ₙOR₆ |
| Aryl | (CH₂)ₙCOOR₆ | H | (CH₂)ₙOR₆ | H |
| Aryl | (CH₂)ₙCOOR₆ | H | (CH₂)ₙOR₆ | Alkyl |
| Aryl | (CH₂)ₙCOOR₆ | H | (CH₂)ₙOR₆ | Aryl |
| Aryl | (CH₂)ₙCOOR₆ | H | (CH₂)ₙOR₆ | (CH₂)ₙCOOR₆ |
| Aryl | (CH₂)ₙCOOR₆ | H | (CH₂)ₙOR₆ | (CH₂)ₙOR₆ |
| Aryl | (CH₂)ₙCOOR₆ | Alkyl | (CH₂)ₙCOOR₆ | H |
| Aryl | (CH₂)ₙCOOR₆ | Alkyl | (CH₂)ₙCOOR₆ | Alkyl |
| Aryl | (CH₂)ₙCOOR₆ | Alkyl | (CH₂)ₙCOOR₆ | Aryl |
| Aryl | (CH₂)ₙCOOR₆ | Alkyl | (CH₂)ₙCOOR₆ | (CH₂)ₙCOOR₆ |
| Aryl | (CH₂)ₙCOOR₆ | Alkyl | (CH₂)ₙCOOR₆ | (CH₂)ₙOR₆ |
| Aryl | (CH₂)ₙCOOR₆ | Alkyl | (CH₂)ₙOR₆ | H |
| Aryl | (CH₂)ₙCOOR₆ | Alkyl | (CH₂)ₙOR₆ | Alkyl |
| Aryl | (CH₂)ₙCOOR₆ | Alkyl | (CH₂)ₙOR₆ | Aryl |
| Aryl | (CH₂)ₙCOOR₆ | Alkyl | (CH₂)ₙOR₆ | (CH₂)ₙCOOR₆ |
| Aryl | (CH₂)ₙCOOR₆ | Alkyl | (CH₂)ₙOR₆ | (CH₂)ₙOR₆ |
| Aryl | (CH₂)ₙCOOR₆ | Aryl | (CH₂)ₙCOOR₆ | H |
| Aryl | (CH₂)ₙCOOR₆ | Aryl | (CH₂)ₙCOOR₆ | Alkyl |
| Aryl | (CH₂)ₙCOOR₆ | Aryl | (CH₂)ₙCOOR₆ | Aryl |
| Aryl | (CH₂)ₙCOOR₆ | Aryl | (CH₂)ₙCOOR₆ | (CH₂)ₙCOOR₆ |
| Aryl | (CH₂)COOR₆ | Aryl | (CH₂)COOR₆ | (CH₂)ₙOR₆ |
| Aryl | (CH₂)ₙCOOR₆ | Aryl | (CH₂)ₙOR₆ | H |
| Aryl | (CH₂)ₙCOOR₆ | Aryl | (CH₂)ₙOR₆ | Alkyl |
| Aryl | (CH₂)ₙCOOR₆ | Aryl | (CH₂)ₙOR₆ | Aryl |
| Aryl | (CH₂)ₙCOOR₆ | Aryl | (CH₂)ₙOR₆ | (CH₂)ₙCOOR₆ |
| Aryl | (CH₂)ₙCOOR₆ | Aryl | ( CH₂)ₙOR₆ | (CH₂)ₙOR₆ |

10. Komplexbildner nach Anspruch 1, wobei X NR₄ ist und Y P(R₅)₂ ist, R₆ H, C₁-Alkyl, C₂-Alkyl, C₃-Alkyl, C₄-Alkyl, C₅-Alkyl oder C₆-Alkyl, Phenyl, Benzyl oder ein Biomolekül ist und R₁, R₂, R₃, R₄ und R₅ gemäß den in Anspruch 9 aufgelisteten Kombinationen sind.

11. Komplexbildner nach einem der Ansprüche 1 bis 8, wobei R₆ ein Biomolekül ist.

12. Komplexbildner nach Anspruch 11, wobei das Biomolekül ausgewählt ist aus Aminosäuren, Peptiden, Proteinen, Oligonukleotiden, Polynukleotiden, Zuckern.

13. Komplexbildner nach Anspruch 12, wobei das Biomolekül ausgewählt ist aus der Gruppe bestehend aus Antikörpern, Liganden von Tumorrezeptoren.

14. Komplexbildner nach Anspruch 12, wobei das Biomolekül ausgewählt ist aus der Gruppe bestehend aus CCK, Thioglucose, Glucosamin, Somatostatin, Neurotensin, Bombesin, CCK, Annexin, Interleukinen, Wachstumsfaktoren, Steroidhormonen und Molekülen, die an GPIIB/IIIa-Rezeptoren binden.

15. Komplexbildner nach Anspruch 12, wobei das Biomolekül ausgewählt ist aus der Gruppe bestehend aus Glucose, Thioglucose, Neurotransmittern.

16. Komplexbildner nach Anspruch 12, wobei das Biomolekül ein Inhibitor der Tyrosinkinaseaktivität ist, wie zum Beispiel Benzothiopyranone, Anilinophthalimide, Chinazoline, Pyridopyrimidine und Pyrrolopyrimidine.

17. Komplexbildner der folgenden Formel:

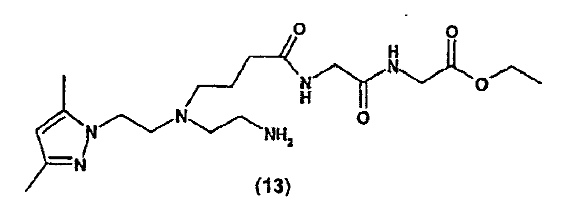

18. Komplexbildner nach Anspruch 1 oder Anspruch 17, wobei der Komplexbildner mit einer Carbonyleinheit der Formel [M(CO)₃]⁺ komplexiert ist, wobei M Rhenium (Re), Technetium (Tc) oder Mangan (Mn) ist.

19. Komplexbildner nach Anspruch 11, wobei der Komplexbildner mit einer Carbonyleinheit der Formel [M(CO)₃]⁺ komplexiert ist, wobei M Rhenium (Re) oder Technetium (Tc) ist.

20. Verfahren zur Herstellung von radiomarkierten Biomolekülen, umfassend:
a) In-Kontakt-bringen eines Komplexbildners nach Anspruch 1 mit einer Carbonyleinheit der Formel [M(CO)₃]⁺, wobei M Rhenium (Re) oder Technetium (Tc) ist, unter Bedingungen zum Bilden eines Chelator-Carbonyl-Komplexes, und
b) In-Kontakt-bringen des Komplexes mit einem Biomolekül, um ein radiomarkiertes Biomolekül zu erhalten.

21. Kit, geeignet, das Verfahren nach Anspruch 20 durchzuführen, umfassend ein erstes Gefäß mit dem Komplexbildner nach Anspruch 1, gegebenenfalls ein erstes Reaktionsgefäß zum In-Kontakt-bringen des Komplexbildners mit der Carbonyleinheit der Formel [M(CO)₃]⁺, wobei M Re oder Tc ist, ein zweites Gefäß mit dem Biomolekül und gegebenenfalls ein zweites Reaktionsgefäß zum Reagieren lassen des Biomoleküls mit dem im ersten Schritt der Reaktion erhaltenen Chelator-Carbonyl-Komplex.

22. Verfahren zur Herstellung von radiomarkierten Biomolekülen, umfassend:
a) In-Kontakt-bringen eines Komplexbildners nach Anspruch 1 mit einem Biomolekül, um ein Chelator-Biomolekül zu erhalten;
und
b) In-Kontakt-bringen des Chelator-Biomoleküls mit einer Carbonyleinheit der Formel [M(CO)₃]⁺, wobei M Rhenium (Re) oder Technetium (Tc) ist, unter Bedingungen zum Bilden eines radiomarkierten Biomoleküls.

23. Kit, geeignet, das Verfahren nach Anspruch 22 durchzuführen, umfassend ein erstes Gefäß mit dem Komplexbildner nach Anspruch 1, gegebenenfalls ein erstes Reaktionsgefäß zum Reagieren lassen des Komplexbildners mit dem Biomolekül, ein zweites Gefäß mit der Carbonyleinheit der Formel [M(CO)₃]⁺, wobei M Re oder Tc ist, und gegebenenfalls ein zweites Reaktionsgefäß zum Reagieren lassen des Chelator-Biomoleküls, das im ersten Schritt der Reaktion mit dem Carbonyl erhalten wurde.

24. Komplexbildner nach Anspruch 1 oder Anspruch 17, zur Verwendung bei der Herstellung eines diagnostischen oder therapeutischen Mittels zum Diagnostizieren oder Behandeln von Tumoren.

25. Komplexbildner nach Anspruch 18, zur Verwendung als diagnostisches oder therapeutisches Mittel zum Diagnostizieren oder Behandeln von Tumoren.

26. Verwendung eines Komplexbildners nach Anspruch 1 oder Anspruch 17 zur Herstellung eines diagnostischen oder therapeutischen Mittels zum Diagnostizieren oder Behandeln von Tumoren.

27. Verwendung eines Komplexbildners nach Anspruch 18 zur Herstellung eines diagnostischen oder therapeutischen Mittels zum Diagnostizieren oder Behandeln von Tumoren.

## Revendications

1. Agent chélateur de formule générale : 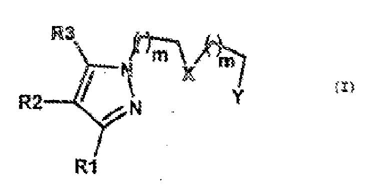 dans laquelle m vaut 0 ou 1,
X est NR₄ ;
Y est NHR₅, SR₅ ou P(R₅)₂;
R₁ et R₃ sont identiques ou différents et sont choisis parmi un atome d'hydrogène, un groupe alkyl ou aryle ;
R₂ est un atome d'hydrogène, COOH, NHR₆ ou (CH₂)ₙCOOR₆ ;
R₄ est (CH₂)ₙCOOR₆ ou (CH₂)ₙOR₆;
R₅ est un atome d'hydrogène, un groupe alkyl, aryl, (CH₂)ₙCOOR₆ ou (CH₂)ₙOR₆ ;
R₆ est un atome d'hydrogène, un groupe alkyl ou aryl ou une biomolécule ; et
n vaut 1, 2, 3, 4, 5, 6, 7, 8, 9 ou 10.

2. Agent chélateur selon la revendication 1, dans lequel le groupe alkyl est un groupe alkyl en C₁, alkyl en C₂, alkyl en C₃, alkyl en C₄, alkyl en C₅ ou alkyl en C₆.

3. Agent chélateur selon la revendication 2, dans lequel le groupe alkyl est un groupe méthyle, éthyle, n-propyle, isopropyle, n-butyle, isobutyle, s-butyle, t-butyle, n-pentyle, isopentyle, néopentyle, n-hexyle, isohexyl(2-méthylpentyl), néohexyl(2,2-diméthylbutyl), 3-méthylpentyle, 2,3-diméthylbutyle.

4. Agent chélateur selon la revendication 1, dans lequel le groupe aryl est un groupe monocyclique, de préférence, un groupe phényle ou benzyl, ou polycyclique en C₁₀ à C₁₈ et éventuellement substitué par un groupe alkyl, carboxy, oxo, amino, alcoxy ou aldéhyde.

5. Agent chélateur selon la revendication 4, dans lequel le groupe aryl est un groupe phényle ou benzyl.

6. Agent chélateur selon la revendication 1, dans lequel n vaut 2, 3, 4, 5 ou 6 et de préférence, 2, 3 ou 4.

7. Agent chélateur selon la revendication 1, ledit agent étant une pyrazolyl-polyamine de formule générale : 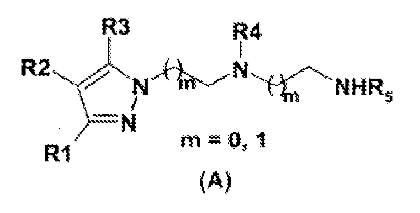 dans laquelle R₁, R₂, R₃, R₄ et R₅ sont tels que définis à la revendication 1.

8. Agent chélateur selon la revendication 1, ledit agent étant une pyrazolyl-aminophosphine de formule générale : 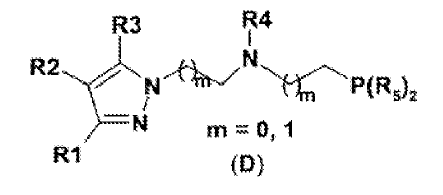 dans laquelle R₁, R₂, R₃, R₄ et R₅ sont tels que définis à la revendication 1.

9. Agent chélateur selon la revendication 1, dans lequel X est NR₄ et Y est NHR₅, R₆ est H, un groupe alkyl en C₁, alkyl en C₂, alkyl en C₃, alkyl en C₄, alkyl en C₅ ou alkyl en C₆, phényle, benzyl ou une biomolécule et R₁, R₂, R₃, R₄ et R₅ sont tels que dans les combinaisons suivantes :
| **R1** | **R2** | **R3** | **R4** | **R5** |
|---|---|---|---|---|
| H | H | H | (CH₂)ₙCOOR₆ | H |
| H | H | H | (CH₂)ₙCOOR₆ | alkyl |
| H | H | H | (CH₂)ₙCOOR₆ | aryl |
| H | H | H | (CH₂)ₙCOOR₆ | (CH₂)ₙCOOR₆ |
| H | H | H | (CH₂)ₙCOOR₆ | (CH₂)ₙOR₆ |
| H | H | H | (CH₂)ₙOR₆ | H |
| H | H | H | (CH₂)ₙOR₆ | alkyl |
| H | H | H | (CH₂)ₙOR₆ | aryl |
| H | H | H | CH₂)ₙOR₆ | (CH₂)ₙOOR₆ |
| H | H | H | (CH₂)ₙOR₆ | (CH₂)ₙOR₆ |
| H | H | alkyl | (CH₂)ₙCOOR₆ | H |
| H | H | alkyl | (CH₂)ₙCOOR₆ | alkyl |
| H | H | alkyl | (CH₂)ₙ COOR₆ | aryl |
| H | H | alkyl | (CH₂)ₙCOOR₆ | (CH₂)ₙCOOR₆ |
| H | H | alkyl | (CH₂)ₙCOOR₆ | (CH₂)ₙOR₆ |
| H | H | alkyl | (CH₂)ₙOR₆ | alkyl |
| H | H | alkyl | (CH₃)ₙOR₆ | aryl |
| H | H | alkyl | (CH₂)ₙOR₆ | (CH₂)ₙCOOR₆ |
| H | H | Alkyl | (CH₂)ₙOR₆ | (CH₂)ₙOR₆ |
| H | H | aryl | (CH₂)ₙCOOR₆ | H |
| H | H | aryl | (CH₂)ₙCOOR₆ | alkyl |
| H | H | aryl | (CH₂)ₙCOOR₆ | aryl |
| H | H | aryl | (CH₂)ₙCOOR₆ | (CH₂)ₙCOOR₆ |
| H | H | aryl | (CH₂)ₙCOOR₆ | (CH₃)ₙOR₆ |
| H | H | aryl | (CH₂)ₙOR₆ | H |
| H | H | aryl | CH₃)ₙOR₆ | alkyl |
| H | H | aryl | (CH₂)ₙOR₆ | aryl |
| H | H | aryl | (CH₂)ₙOR₆ | (CH₂)ₙCOOR₆ |
| H | H | aryl | (CH₂)ₙOR₆ | (CH₂)ₙOR₆ |
| H | COOH | H | (CH₂)ₙCOOR₆ | H |
| H | COOH | H | (CH₂)ₙCOOR₆ | alkyl |
| H | COOH | H | (CH₂)ₙCOOR₆ | aryl |
| H | COOH | H | (CH₂)ₙCOOR₆ | (CH₂)ₙCOOR₆ |
| H | COOH | H | (CH₂)ₙCOOR₆ | (CH₂)ₙOR₆ |
| H | COOH | H | (CH₂)ₙOR₆ | H |
| H | COOH | H | (CH₂)ₙOR₆ | alkyl |
| H | COOH | H | (CH₂)ₙOR₆ | aryl |
| H | COOH | H | (CH₂)ₙOR₆ | (CH₂)ₙCOOR₆ |
| H | COOH | H | (CH₂)ₙOR₆ | (CH₂)ₙOR₆ |
| H | COOH | alkyl | (CH₂)ₙCOOR₆ | H |
| H | COOH | alkyl | (CH₂)ₙCOOR₆ | alkyl |
| H | COOH | alkyl | (CH₂)ₙCOOR₆ | aryl |
| H | COOH | alkyl | (CH₂)ₙCOOR₆ | (CH₂)ₙCOOR₆ |
| H | COOH | alkyl | (CH₂)ₙCOOR₆ | (CH₂)ₙOH₆ |
| H | COOH | alkyl | (CH₂)ₙOR₆ | H |
| H | COOH | alkyl | (CH₂)ₙOR₆ | alkyl |
| H | COOH | alkyl | (CH₂)ₙOR₆ | aryl |
| H | COOH | alkyl | (CH₂)ₙOR₆ | (CH₂)ₙCOOR₆ |
| H | COOH | alkyl | (CH₂)ₙCOOR₆ | (CH₂)ₙOR₆ |
| H | COOH | aryl | (CH₂)ₙCOOR₆ | H |
| H | COOH | aryl | (CH₂)ₙCOOR₆ | alkyl |
| H | COOH | aryl | (CH₃)ₙCOOR₆ | aryl |
| H | COOH | aryl | (CH₂)ₙCOOR₆ | (CH₂)ₙCOOR₆ |
| H | COOH | aryl | (CH₂)ₙCOOR₆ | (CH₂)ₙOR₆ |
| H | COOH | aryl | (CH₃)ₙOR₆ | H |
| H | COOH | aryl | (CH₂)ₙOR₆ | alkyl |
| H | COOH | aryl | (CH₂)ₙOR₈ | alkyl |
| H | COOH | aryl | (CH₃)ₙOR₆ | (CH₂)ₙCOOR₆ |
| H | COOH | aryl | (CH₂)ₙOR₆ | (CH₂)ₙOR₆ |
| H | NHR₅ | H | (CH₂)ₙCOOR₆ | H |
| H | NHR₆ | H | (CH₃)ₙCODR₆ | alkyl |
| H | NHR₆ | H | (CH₂)ₙCOOR₆ | aryl |
| H | NHR₆ | H | (CH₃)ₙCOOR₆ | (CH2)ₙCOOR₆ |
| H | NHR₆ | H | (CH₃)ₙCOOR₆ | (CH₂)ₙOR₆ |
| H | NHR₆ | H | (CH₂)ₙOR₈ | H |
| H | NHR₆ | H | (CH₂)ₙOR₈ | alkyl |
| H | NHR₆ | H | (CH₃)ₙOR₆ | aryl |
| H | NHR₆ | H | (CH₂)ₙOR₆ | (CH₂)ₙCOOR₆ |
| H | NHR₆ | H | (CH₃)ₙOR₆ | (CH2)ₙOR₆ |
| H | NHR₆ | alkyl | (CH₂)ₙCOOR₆ | H |
| H | NHR₆ | alkyl | (CH₃)ₙCOOR₆ | alkyl |
| H | NHR₆ | alkyl | (CH₃)ₙCOOR₆ | aryl |
| H | NHR₆ | alkyl | (CH₃)ₙCOOR₆ | (CH₂)ₙCOOR₆ |
| H | NHR₆ | alkyl | (CH₃)ₙCOOR₆ | (CH₂)ₙOR₆ |
| H | NHR₆ | alkyl | (CH₂)ₙOR₆ | H |
| H | NHR₆ | alkyl | (CH₂)ₙOR₆ | alkyl |
| H | NHR₆ | alkyl | (CH₂)ₙOR₆ | aryl |
| H | NHR₆ | alkyl | (CH₂)ₙOR₆ | (CH₂)ₙCOOR₆ |
| H | NHR₆ | alkyl | (CH₂)ₙOR₆ | (CH₃)ₙOR₆ |
| H | NHR₆ | aryl | (CH₂)ₙCOOR₆ | H |
| H | NHR₆ | aryl | (CH₂)ₙCOOR₆ | alkyl |
| H | NHR₆ | aryl | (CH₂)nCOOR₆ | aryl |
| H | NHR₆ | aryl | (CH₂)ₙCOOR₆ | (CH2)ₙCOOR₆ |
| H | NHR₆ | aryl | (CH₂)ₙCOOR₆ | (CH₂)ₙOR₆ |
| H | NHR₆ | aryl | (CH₂)ₙOR₆ | H |
| H | NHR₆ | aryl | (CH₂)ₙOR₆ | alkyl |
| H | NHR₆ | aryl | (CH₂)ₙOR₆ | aryl |
| H | NHR₆ | aryl | (CH₂)ₙOR₆ | (CH₂)ₙCOOR₆ |
| H | NHR₆ | aryl | (CH₂)ₙOR₆ | (OH₂)ₙOR₆ |
| H | (CH₂)ₙCOOR₆ | H | (CH₂)ₙCOOR₆ | H |
| H | (CH₂)ₙCOOR₆ | H | (CH₂)ₙCOOR₆ | alkyl |
| H | (CH₂)ₙCOOR₆ | H | (CH₂)ₙCOOR₆ | aryl |
| H | (CH₂)ₙCOOR₆ | H | (CH₂)ₙCOOR₆ | (CH₂)ₙCOOR₆ |
| H | (CH₂)ₙCOOR₆ | H | (CH₂)ₙCOOR₆ | (CH₂)ₙOR₆ |
| H | (CH₂)ₙCOOR₆ | H | (CH₂)ₙOR₆ | H |
| H | (CH₂)ₙCOOR₆ | H | (CH₂)ₙOR₆ | alkyl |
| H | (CH₂)ₙCOOR₆ | H | (CH₂)ₙOR₆ | aryl |
| H | (CH₂)ₙCOOR₆ | H | (CH₂)ₙOR₆ | (CH₂)ₙCOOR₆ |
| H | (CH₂)ₙCOOR₆ | H | (CH₂)ₙOR₆ | (CH₂)ₙOR₆ |
| H | (CH₂)ₙCOOR₆ | alkyl | (CH₂)ₙCOOR₆ | H |
| H | (CH₂)ₙCOOR₆ | alkyl | (CH₂)ₙCOOR₆ | alkyl |
| H | (CH₂)ₙCOOR₆ | alkyl | (CH₂)ₙCOOR₆ | aryl |
| H | (CH₂)ₙCOOR₆ | alkyl | (CH₂)ₙCOOR₆ | (CH₂)ₙCOOR₆ |
| H | (CH₂)ₙCOOR₆ | alkyl | (CH2)ₙCOOR₆ | (CH₂)ₙOR₆ |
| H | (CH₂)ₙCOOR₆ | alkyl | (CH₂)ₙOR₆ | H |
| H | (CH₂)ₙCOOR₆ | alkyl | (CH₂)ₙOR₆ | alkyl |
| H | (CH₂)ₙCOOR₆ | alkyl | (CH₂)ₙOR₆ | aryl |
| H | (CH₂)ₙCOOR₆ | alkyl | (CH₂)ₙOR₆ | (CH2)ₙCOOR₆ |
| H | (CH₂)ₙCOOR₆ | alkyl | (CH₂)ₙOR₆ | (CH₂)ₙOR₆ |
| H | (CH₂)ₙCOOR₆ | aryl | (CH₂)ₙCOOR₆ | H |
| H | (CH₂)ₙCOOR₆ | aryl | (CH₂)ₙCOOR₆ | alkyl |
| H | (CH₂)ₙCOOR₆ | aryl | (CH₂)ₙCOOR₆ | aryl |
| H | (CH₂)ₙCOOR₆ | aryl | (CH₂)ₙCOOR₆ | (CH₂)ₙCOOR₆ |
| H | (CH₂)ₙCOOR₆ | aryl | (CH₂)ₙCOOR₆ | (CH₂)ₙOR₆ |
| H | (CH₂)ₙCOOR₆ | aryl | (CH₂)ₙOR₆ | H |
| H | (CH₂)ₙCOOR₆ | aryl | (CH₂)ₙOR₆ | alkyl |
| H | (CH₂)ₙCOOR₆ | aryl | (CH₂)ₙOR₆ | aryl |
| H | (CH₂)ₙCOOR₆ | aryl | (CH₂)ₙOR₆ | alkyl |
| H | (CH₂)ₙCOOR₆ | aryl | (CH₂)ₙOR₆ | aryl |
| H | (CH₂)ₙCOOR₆ | aryl | (CH₂)ₙOR₆ | (CH₂)ₙCOOR₆ |
| H | (CH₂)ₙCOOR₆ | aryl | (CH₂)ₙOR₆ | (CH₂)ₙOR₄ |
| H | (CH₂)ₙCOOR₆ | aryl | (CH₂)ₙCOO₆ | H |
| alkyl | H | H | (CH₂)ₙCOOR₆ | alkyl |
| alkyl | H | H | CH₂)ₙCOOR₆ | aryl |
| alkyl | H | H | (CH₂)ₙCOOR₆ | |
| alkyl | H | H | (CH₂)ₙCOOR₆ | (CH₂)ₙCOOR₆ |
| alkyl | H | H | (CH₂)ₙCOOR₆ | (CH₂)ₙOR₆ |
| alkyl | H | H | (CH₂)ₙOR₆ | H |
| alkyl | H | H | (CH₂)ₙOR₆ | alkyl |
| alkyl | H | H | CH₂)ₙOR₆ | aryl |
| alkyl | H | H | (CH₂)ₙOR₆ | (CH₂)ₙCOOR₆ |
| alkyl | H | H | (CH₂)ₙOR₆ | (CH₂)ₙOR₆ |
| alkyl | H | H | (CH₂)ₙCOOR₆ | alkyl |
| alkyl | H | H | (CH₂)ₙCOOR₆ | alkyl |
| alkyl | H | alkyl | (CH₂)ₙCOOR₆ | aryl |
| alkyl | H | alkyl | (CH₂)ₙCOOR₆ | (CH₂)ₙCOOR₆ |
| alkyl | H | alkyl | (CH₂)ₙCOOR₆ | (CH₂)ₙOR₆ |
| alkyl | H | alkyl | (CH₂)ₙOR₆ | H |
| alkyl | H | alkyl | (CH₂)ₙOR₆ | alkyl |
| alkyl | H | alkyl | (CH₂)ₙOR₆ | aryl |
| alkyl | H | alkyl | (CH₂)ₙOR₆ | (CH₂)ₙCOOR₆ |
| alkyl | H | alkyl | (CH₂)ₙOR₆ | (CH₂)ₙOR₆ |
| alkyl | H | aryl | (CH₂)ₙCOOR₆ | H |
| alkyl | H | alkyl | (CH₂)ₙCOOR₆ | alkyl |
| alkyl | H | alkyl | (CH₂)ₙCOOR₆ | alkyl |
| alkyl | H | alkyl | (CH₂)ₙCOOR₆ | (CH₂)ₙCOOR₆ |
| alkyl | H | alkyl | (CH₂)ₙCOOR₆ | (CH₂)ₙOR₆ |
| alkyl | H | aryl | (CH₂)ₙOR₆ | H |
| alkyl | H | aryl | (CH₂)ₙOR₆ | alkyl |
| alkyl | H | alkyl | (CH₂)ₙOR₆ | aryl |
| alkyl | H | aryl | (CH₂)ₙOR₆ | (CH₂)ₙCOOR₆ |
| alkyl | H | aryl | (CH₂)ₙOR₆ | (CH₂)ₙOR₆ |
| alkyl | COOH | H | (CH₂)ₙCOOR₆ | H |
| alkyl | COOH | H | (CH₂)ₙCOOR₆ | aryl |
| alkyl | COOH | H | (CH₂)ₙCOOR₆ | aryl |
| alkyl | COOR | H | (CH₂)ₙCOOR₆ | (CH₂)ₙCOOR₆ |
| alkyl | COOH | H | (CH₂)ₙCOOR₆ | (CH₂)ₙOR₆ |
| alkyl | COOH | H | (CH₂)ₙOR₆ | H |
| alkyl | COOH | H | (CH₂)ₙOR₆ | alkyl |
| alkyl | COOH | H | (CH₂)ₙOR₆ | aryl |
| alkyl | COOH | H | (CH₂)ₙOR₆ | (CH₂)ₙCOOR₆ |
| alkyl | COOH | H | (CH₂)ₙOR₆ | (CH₂)ₙOR₆ |
| alkyl | COOH | alkyl | (CH₂)ₙCOOR₆ | H |
| alkyl | COOH | alkyl | (CH₂)ₙCOOR₆ | alkyl |
| alkyl | COOH | alkyl | (CH₂)ₙCOOR₆ | aryl |
| alkyl | COOH | alkyl | (CH₂)ₙCOOR₆ | (CH₂)ₙCOOR₆ |
| alkyl | COOH | alkyl | (CH₂)ₙCOOR₆ | (CH₂)ₙOR₆ |
| alkyl | COOH | alkyl | (CH₂)ₙOR₆ | H |
| alkyl | COOH | alkyl | (CH₂)ₙOR₆ | alkyl |
| alkyl | COOH | alkyl | (CH₂)ₙOR₆ | aryl |
| alkyl | COOH | alkyl | (CH₂)ₙOR₆ | (CH₂)ₙCOOR₆ |
| alkyl | COOH | alkyl | (CH₂)ₙOR₆ | (CH₂)ₙOR₆ |
| alkyl | COOH | aryl | (CH₂)ₙCOOR₆ | H |
| alkyl | COOH | aryl | (CH₂)ₙCOOR₆ | alkyl |
| alkyl | COOH | aryl | (CH₂)ₙCOOR₆ | aryl |
| alkyl | CCOH | alkyl | (CH₂)ₙCOOR₆ | (CH₂)ₙCOOR₆ |
| alkyl | COOH | aryl | (CH₂)ₙCOOR₆ | (CH₂)ₙOR₆ |
| alkyl | COOH | alkyl | (CH₂)ₙOR₆ | H |
| alkyl | COOH | aryl | (CH₂)ₙOR₆ | alkyl |
| alkyl | COOH | aryl | (CH₂)ₙOR₆ | aryl |
| alkyl | COOH | aryl | (CH₂)ₙOR₆ | (CH₂)ₙCOOR₆ |
| alkyl | COOH | aryl | (CH₂)ₙOR₆ | (CH₂)ₙOR₆ H |
| alkyl | NHR₆ | H | (CH₂)ₙCOOR₆ | H |
| alkyl | NHR₆ | H | (CH₂)ₙCOOR₆ | alkyl |
| alkyl | NHR₆ | H | (CH₂)ₙCOOR₆ | aryl |
| alkyl | NHR₆ | H | (CH₂)ₙCOOR₆ | (CH₂)ₙCOOR₆ |
| alkyl | NHR₆ | H | (CH₂)ₙCOOH₆ | (CH₃)ₙOR₆ |
| alkyl | NHR₆ | H | (CH₂)ₙOR₆ | H |
| alkyl | NHR₆ | H | (CH₂)ₙOR₆ | alkyl |
| alkyl | NHR₆ | H | (CH₂)ₙOR₆ | aryl |
| alkyl | NHR₆ | H | (CH₂)ₙOR₆ | (CH₂)ₙCOOR₆ |
| alkyl | NHR₆ | H | (CH₂)ₙOR₆ | (CH₂)ₙOR₆ |
| alkyl | NHR₆ | alkyl | (CH₂)ₙCOOR₆ | H |
| alkyl | NHR₆ | alkyl | (CH₂)ₙCOOR₆ | alkyl |
| alkyl | NHR₆ | alkyl | (CH₂)ₙCOOR₆ | aryl |
| alkyl | NHR₆ | alkyl | (CH₂)ₙCOOR₆ | (CH₂)ₙCOOR₆ |
| alkyl | NHR₆ | alkyl | (CH₂)ₙCOOR₆ | (CH₂)ₙOR₆ |
| alkyl | NHR₆ | alkyl | (CH₂)ₙOR₆H | H |
| alkyl | NHR₆ | alkyl | (CH₂)ₙOR₆ | alkyl |
| alkyl | NHR₆ | alkyl | (CH₂)ₙOR₆ | aryl |
| alkyl | NHR₆ | alkyl | (CH₂)ₙOR₆ | (CH₂)ₙCOOR₆ |
| alkyl | NHR₆ | alkyl | (CH₂)ₙOR₆ | (CH₂)ₙOR₆ |
| alkyl | NHR₆ | aryl | (CH₂)ₙCOOR₆ | H |
| alkyl | NHR₆ | aryl | (CH₂)ₙCOOR₆ | alkyl |
| alkyl | NHR₆ | aryl | (CR₂)ₙCOOR₆ | aryl |
| alkyl | NHR₆ | aryl | (CH₂)ₙCOOR₆ | (CH₂)ₙCOOR₆ |
| alkyl | NHR₆ | aryl | (CH₂)ₙCOOR₆ | (CH₂)ₙOR₆ |
| alkyl | NHR₆ | aryl | (CH₂)ₙOR₆ | H |
| alkyl | NHR₆ | aryl | (CH₂)ₙOR₆ | alkyl |
| alkyl | NHR₆ | aryl | (CH₂)ₙOR₆ | aryl |
| alkyl | NHR₆ | aryl | (CH₂)ₙOR₆ | (CH₂)ₙOR₆ |
| alkyl | NHR₆ | aryl | (CH₂)ₙCOOR₆ | (CH₂)ₙOR₆ |
| alkyl | (CH₂)ₙCOOR₆ | H | (CH₂)ₙCOOR₆ | H |
| alkyl | (CH₂)ₙCOOR₆ | H | (CH₂)ₙCOOR₆ | alkyl |
| alkyl | (CH₂)ₙCOOR₆ | H | (CH₂)ₙCOOR₆ | aryl |
| alkyl | (CH₂)ₙCOOR₆ | H | (CH₂)ₙCOOR₆ | (CH₂)ₙCOOR₆ |
| alkyl | (CH₂)ₙCOOR₆ | H | (CH₂)ₙCOOR₆ | (CH₂)ₙOR₆ |
| alkyl | (CH₂)ₙCOOR₆ | H | (CH₂)ₙOR₆ | H |
| alkyl | (CH₂)ₙCOOR₆ | H | (CH₂)ₙOR₆ | alkyl |
| alkyl | (CH₂)ₙCOOR₆ | H | (CH₂)ₙOR₆ | aryl |
| alkyl | (CH₂)ₙCOOR₆ | H | (CH₂)ₙOR₆ | (CH₂)ₙCOOR₆ |
| alkyl | (CH₂)ₙCOOR₆ | H | (CH₂)ₙOR₆ | (CH₂)ₙOR₆ |
| alkyl | (CH₂)ₙCOOR₆ | alkyl | (CH₂)ₙCOOR₆ | H |
| alkyl | (CH₂)ₙCOOR₆ | alkyl | (CH₂)ₙCOOR₆ | alkyl |
| alkyl | (CH₂)ₙCOOR₆ | alkyl | (CH₂)ₙCOOR₆ | aryl |
| alkyl | (CH₂)ₙCOOR₆ | alkyl | (CH₂)ₙCOOR₆ | (CH₂)ₙCOOR₆ |
| alkyl | (CH₂)ₙCOOR₆ | alkyl | (CH₂)ₙCOOR₆ | (CH₂)ₙOR₆ |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | alkyl |
| alkyl | (CH₂)ₙCOOR₆ | alkyl | (CH₂)ₙOR₆ | aryl |
| alkyl | (CH₂)ₙCOOR₆ | alkyl | (CH₂)ₙOR₆ | (CH₂)ₙCOOR₆ |
| alkyl | (CH₂)ₙCOOR₆ | alkyl | (CH₂)ₙCOR₆ | (CH₂)ₙOR₆ |
| alkyl | (CH₂)ₙCOOR₆ | aryl | (CH₂)ₙCOOR₆ | H |
| alkyl | (CH₂)ₙCOOR₆ | aryl | (CH₂)ₙCOOR₆ | alkyl |
| alkyl | (CH₂)ₙCOOR₆ | aryl | (CH₂)ₙCOOR₆ | aryl |
| alkyl | (CH₂)ₙCOOR₆ | aryl | (CH₂)ₙCOOR₆ | (CH₂)ₙCOOR₆ |
| alkyl | (CH₃)ₙCOOR₆ | aryl | (CH₂)ₙCOOR₆ | (CH₂)ₙOR₆ |
| alkyl | (CH₂)ₙCOOR₆ | aryl | (CH₂)ₙOR₆ | H |
| alkyl | (CH₂)ₙCOOR₆ | aryl | (CH₂)ₙOR₆ | alkyl |
| alkyl | (CH₂)ₙCOOR₆ | aryl | (CH₂)nOR₆ | aryl |
| alkyl | (CH₂)ₙCOOR₆ | aryl | (CH₂)ₙOR₆ | (CH₂)ₙCOOR₆ |
| alkyl | (CH₂)ₙCOOR₆ | aryl | (CH₂)ₙOR₆ | (CH₂)ₙOR₆ |
| aryl | H | H | (CH₂)ₙCOOR₆ | H |
| aryl | H | H | (CH₂)ₙCOOR₆ | alkyl |
| aryl | H | H | (CH₂)ₙCOOR₆ | aryl |
| aryl | H | H | (CH₂)ₙCOOR₆ | (CH₂)ₙCOOR₆ |
| aryl | H | H | (CH₂)ₙCOOR | (CH₂)ₙOR₆ |
| aryl | H | H | (CH₂)ₙOR₆ | H |
| aryl | H | H | (CH₂)ₙOR₆ | alkyl |
| aryl | H | H | (CH₂)ₙOR₆ | aryl |
| aryl | H | H | (CH₂)ₙOR₆ | (CH₂)ₙCOOR₆ |
| aryl | H | H | (CH₂)ₙOR₆ | (CH₂)ₙOR₆ |
| aryl | H | alkyl | (CH₂)ₙCOOR₆ | H |
| aryl | H | alkyl | (CH₂)ₙCOOR₆ | alkyl |
| aryl | H | alkyl | (CH₂)ₙCOOR₆ | aryl |
| aryl | H | alkyl | (CH₂)ₙCOOR₆ | (CH₂)ₙCOOR₆ |
| aryl | H | alkyl | (CH₂)ₙCOOR₆ | (CH₂)ₙOR₆ |
| aryl | H | alkyl | (CH₂)ₙOR₆ | H |
| aryl | H | alkyl | (CH₂)ₙOR₆ | alkyl |
| alkyl | H | alkyl | (CH₂)ₙCH₆ | aryl |
| aryl | H | alkyl | (CH₂)ₙOR₆ | (CH₂)ₙCOOR₆ |
| aryl | H | alkyl | (CH₂)ₙOR₆ | (CH₂)ₙOR₆ |
| aryl | H | aryl | (CH₂)ₙCOOR₆ | H |
| aryl | H | aryl | (CH₂)ₙCOOR₆ | alkyl |
| aryl | H | aryl | (CH2)ₙCORR₆ | aryl |
| aryl | H | aryl | (CH₂)ₙCOOR₆ | (CH₂)ₙCOOR₆ |
| aryl | H | aryl | (CH₂)ₙCOOR₆ | (CH₂)ₙOR₆ |
| aryl | H | aryl | (CH₂)ₙOR₆ | H |
| aryl | H | aryl | (CH₂)ₙOR₆ | alkyl |
| aryl | H | aryl | (CH₂)ₙOR₆ | aryl |
| aryl | H | aryl | (CH₂)ₙOR₆ | (CH₂)ₙCOOR₆ |
| aryl | H | aryl | (CH₂)ₙOR₆ | (CH₂)ₙOR₆ |
| aryl | COOH | H | (CH₂)ₙCOOR₆ | H |
| aryl | COOH | H | (CH₂)ₙCOOR₆ | alkyl |
| aryl | COOH | H | (CH₂)ₙCOOR₆ | aryl |
| aryl | COOH | H | (CH₂)ₙCOOR₆ | (CH₂)ₙCOOR₆ |
| aryl | COOH | H | (CH₂)ₙCOOR₆ | (CH₃)ₙOR₆ |
| aryl | H | H | (CH₂)ₙCH₆ | (CH₂)ₙCH₆ |
| aryl | COOH | H | (CH₂)ₙCR₆ | alkyl |
| aryl | COOH | H | (CH₂)ₙOR₆ | aryl |
| aryl | COOH | H | (CH₂)ₙOR₆ | (CH₂)ₙCOOR₆ |
| aryl | COOH | H | (CH₂)ₙOR₆ | (CH₂)ₙOR₆ |
| aryl | COOH | alkyl | (CH₂)ₙCOOR₆ | H |
| aryl | COOH | alkyl | (CH₂)ₙCOOR₆ | alkyl |
| aryl | COOH | alkyl | (CH₂)ₙCOOR₆ | aryl |
| aryl | COOH | alkyl | (CH₂)ₙCOOR₆ | (CH₂)ₙCOOR₆ |
| aryl | COOH | alkyl | (CH₂)ₙCOOR₆ | (CH₂)ₙCOOR₆ |
| aryl | COOH | alkyl | (CH₂)ₙOR₆ | H |
| aryl | COOH | alkyl | (CH₂)ₙCR₆ | alkyl |
| aryl | COOH | alkyl | (CH₂)ₙCR₆ | aryl |
| aryl | COOH | alkyl | (CH₂)ₙOR₆ | (CH₂)ₙCOOR₆ |
| aryl | COOH | alkyl | (CH₂)ₙOR₆ | (CH₂)ₙOR₆ |
| aryl | COOH | aryl | (CH₂)ₙCOOR₆ | H |
| aryl | COOH | aryl | (CH₂)ₙCOOR₆ | alkyl |
| aryl | COOH | aryl | (CH₂)ₙCOOR₆ | aryl |
| aryl | COOH | aryl | (CH₂)ₙCOOR₆ | (CH₂)ₙCOOR₆ |
| aryl | COOH | aryl | (CH₂)ₙCOOR₆ | (CH₂)ₙCOOR₆ |
| aryl | COOH | aryl | (CH₂)ₙCOOR₆ | H |
| aryl | COOH | aryl | (CH₂)ₙOR₆ | alkyl |
| aryl | COOH | aryl | (CH₂)ₙOR₆ | aryl |
| aryl | COOH | aryl | (CH₂)ₙOR₆ | (CH₂)ₙCOOR₆ |
| aryl | COOH | aryl | (CH₂)ₙOR₆ | (CH₂)ₙOR₆ |
| aryl | NHR₆ | H | (CH₂)ₙCOOR₆ | H |
| aryl | NHR₆ | H | (CH₂)ₙCOOR₆ | alkyl |
| aryl | NHR₆ | H | (CH₂)ₙCOOR₆ | aryl |
| aryl | NHR₆ | H | (CH₂)ₙCOOR₆ | (CH₂)ₙCOOR₆ |
| aryl | NHR₆ | H | (CH₂)ₙCOOR₆ | (CH₂)ₙOR₆ |
| aryl | NHR₆ | H | (CH₂)ₙOR₆ | H |
| aryl | NHR₆ | H | (CH₂)ₙOR₆ | alkyl |
| aryl | NHR₆ | H | (CH₂)ₙOR₆ | aryl |
| aryl | NHR₆ | H | (CH₂)ₙOR₆ | (CH₂)ₙCOOR₆ |
| aryl | NHR₆ | H | (CH₂)ₙOR₆ | (CH₂)ₙOR₆ |
| aryl | NHR₆ | alkyl | (CH₂)ₙCOOR₆ | H |
| aryl | NHR₆ | alkyl | (CH₂)ₙCOOR₆ | alkyl |
| aryl | NHR₆ | alkyl | (CH₂)ₙCOOR₆ | aryl |
| aryl | NHR₆ | alkyl | (CH₂)ₙCOOR₆ | (CH₂)ₙCOOR₆ |
| aryl | NHR₆ | alkyl | (CH₂)ₙCOOR₆ | (CH₂)ₙCOOR₆ |
| aryl | NHR₆ | alkyl | (CH₂)ₙCOOR₆ | (CH₂)ₙOR₆ |
| aryl | NHR₆ | alkyl | (CH₂)ₙOR₆ | H |
| aryl | NHR₆ | alkyl | (CH₂)ₙOR₆ | alkyl |
| aryl | NHR₆ | alkyl | (CH₂)ₙOR₆ | aryl |
| aryl | NHR₆ | alkyl | (CH₂)ₙOR₆ | (CH₂)ₙCOOR₆ |
| aryl | NHR₆ | alkyl | (CH₂)ₙOR₆ | (CH₂)ₙOR₆ |
| aryl | NHR₆ | aryl | (CH₂)ₙCOOR₆ | H |
| aryl | NHR₆ | aryl | (CH₂)ₙCOOR₆ | alkyl |
| aryl | NHR₆ | aryl | (CH₂)ₙCOOR₆ | aryl |
| aryl | NHR₆ | aryl | (CH₂)ₙCOOR₆ | (CH₂)ₙCOOR₆ |
| aryl | NHR₆ | aryl | (CH₂)ₙCOOR₆ | (CH₂)ₙOR₆ |
| aryl | NHR₆ | aryl | (CH₂)ₙOR₆ | H |
| aryl | NHR₆ | aryl | (CH₂)ₙOR₆ | alkyl |
| aryl | NHR₆ | aryl | (CH₂)ₙOR₆ | aryl |
| aryl | NHR₆ | aryl | (CH₂)ₙOR₆ | (CH₂)ₙCOOR₆ |
| aryl | NHR₆ | aryl | (CH₂)ₙOR₆ | (CH₂)ₙOR₆ |
| aryl | (CH₂)ₙCOOR₆ | H | (CH₂)ₙCOOR₆ | H |
| aryl | (CH₂)ₙCOOR₆ | H | (CH₂)ₙCOOR₆ | alkyl |
| aryl | (CH₂)ₙCOOR₆ | H | (CH₂)ₙCOOR₆ | aryl |
| aryl | (CH₂)ₙCOOR₆ | H | (CH₂)ₙCOOR₆ | (CH₂)ₙCOOR₆ |
| aryl | (CH₂)ₙCOOR₆ | H | (CH₂)ₙCOOR₆ | (CH₂)ₙOR₆ |
| aryl | (CH₂)ₙCOOR₆ | H | (CH₂)ₙOR₆ | H |
| aryl | (CH₂)ₙCOOR₆ | H | (CH₂)ₙOR₆ | alkyl |
| aryl | (CH₂)ₙCOOR₆ | H | (CH₂)ₙOR₆ | aryl |
| aryl | (CH₂)ₙCOOR₆ | H | (CH₂)ₙOR₆ | (CH₂)ₙCOOR₆ |
| aryl | (CH₂)ₙCOOR₆ | H | (CH₂)ₙOR₆ | (CH₂)ₙOR₆ |
| aryl | (CH₂)ₙCOOR₆ | alkyl | (CH₂)ₙCOOR₆ | H |
| aryl | (CH₂)ₙCOOR₆ | alkyl | (CH₂)ₙCOOR₆ | alkyl |
| aryl | (CH₂)ₙCOOR₆ | alkyl | (CH₂)ₙCOOR₆ | aryl |
| aryl | (CH₂)ₙCOOR₆ | alkyl | (CH₂)ₙCOOR₆ | (CH₂)ₙCOOR₆ |
| aryl | (CH₂)ₙCOOR₆ | alkyl | (CH₂)ₙCOOR₆ | (CH₂)ₙOR₆ |
| aryl | (CH₂)ₙCOOR₆ | alkyl | (CH₂)ₙOR₆ | H |
| aryl | (CH₂)ₙCOOR₆ | alkyl | (CH₂)ₙOR₆ | alkyl |
| aryl | (CH₂)ₙCOOR₆ | alkyl | (CH₂)ₙCOOR₆ | aryl |
| aryl | (CH₂)ₙOR₆ | alkyl | (CH₂)ₙCOOR₆ | (CH₂)ₙOR₆ |
| aryl | (CH₂)ₙCOOR₆ | alkyl | (CH₂)ₙOR₆ | (CH₂)ₙOR₆ |
| aryl | (CH₂)ₙCOOR₆ | aryl | (CH₂)ₙCOOR₆ | H |
| aryl | (CH₂)ₙCOOR₆ | aryl | (CH₂)ₙCOOR₆ | alkyl |
| aryl | (CH₂)ₙCOOR₆ | aryl | (CH₂)ₙCOOR₆ | aryl |
| aryl | (CH₂)ₙCOOR₆ | aryl | (CH₂)ₙCOOR₆ | (CH₂)ₙCOOR₆ |
| aryl | (CH₂)ₙCOOR₆ | aryl | (CH₂)ₙCOOR₆ | (CH₂)ₙOR₆ |
| aryl | (CH₂)ₙCOOR₆ | aryl | (CH₂)ₙOR₆ | H |
| aryl | (CH₂)ₙCOOR₆ | aryl | (CH₂)ₙOR₆ | alkyl |
| aryl | (CH₂)ₙCOOR₆ | aryl | (CH₂)ₙCOOR₆ | aryl |
| aryl | (CH₂)ₙCOOR₆ | aryl | (CH₃)ₙOR₆ | (CH₂)ₙCOOR₆ |
| aryl | (CH₂)ₙCOOR₆ | aryl | (CH₂)ₙOR₆ ( | CH₂)ₙOR₆ |

10. Agent chélateur selon la revendication 1, dans lequel X est NR₄ et Y est P(R₅)₂, R₆ est un atome d'hydrogène, un groupe alkyl en C₁, alkyl en C₂, alkyl en C₃, alkyl en C₄, alkyl en C₅ ou alkyl en C₆, phényle, benzyl ou une biomolécule et R₁, R₂, R₃, R₄ et R₅ sont selon les combinaisons énumérées à la revendication 9.

11. Agent chélateur selon l'une quelconque des revendications 1 à 8, dans lequel R₆ est une biomolécule.

12. Agent chélateur selon la revendication 11, dans lequel la biomolécule est choisie parmi les acides aminés, les peptides, les protéines, les oligonucléotides, les polynucléotides, les sucres.

13. Agent chélateur selon la revendication 12, dans lequel la biomolécule est choisie dans le groupe consistant en anticorps, les ligands de récepteurs tumoraux.

14. Agent chélateur selon la revendication 12, dans lequel la biomolécule est choisie dans le groupe consistant en CCK, le thioglucose, la glucosamine, la somatostatine, la neurotensine, la bombésine, CCK, l'annexine, les interleukines, les facteurs de croissance, les hormones stéroïdiennes et les molécules se liant aux récepteurs GPIIB/IIIa.

15. Agent chélateur selon la revendication 12, dans lequel la biomolécule est choisie dans le groupe consistant en le glucose, le thioglucose, les neurotransmetteurs.

16. Agent chélateur selon la revendication 12, dans lequel la biomolécule est un inhibiteur de l'activité de la thyrosine kinase tel que les benzothiopyranones, les anilinophtalimides, les quinazolines, les pyridopyrimidines et les pyrrolopyrimidines.

17. Agent chélateur de formule suivante :

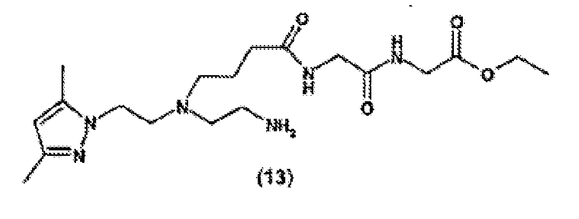

18. Agent chélateur selon la revendication 1 ou la revendication 17, l'agent étant complexé avec un fragment carbonyle de formule [M (CO)₃]⁺, où M est le rhénium (Re), le technétium (Tc) ou le manganèse (Mn).

19. Agent chélateur selon la revendication 11, l'agent étant complexé à un fragment carbonyle de formule [M(CO)₃]⁺, M étant le rhénium (Re) ou le technétium (Tc).

20. Procédé de préparation de biomolécules radiomarquées comprenant :
a) la mise en contact d'un agent chélateur tel que défini à la revendication 1, comportant un fragment carbonyle de formule [M(CO)₃]⁺, où M est le rhénium (Re) ou le technétium (Tc) dans des conditions permettant de former un complexe chélateur-carbonyle ; et
b) la mise en contact du complexe avec une biomolécule pour obtenir une biomolécule radiomarquée.

21. Kit approprié pour réaliser le procédé tel que défini à la revendication 20, comprenant un premier flacon contenant l'agent chélateur tel que défini à la revendication 1, optionnellement, un premier flacon réactionnel pour mettre en contact l'agent chélateur avec le fragment carbonyle de formule [M(CO)₃]⁺, où M est Re ou Tc, un deuxième flacon contenant la biomolécule et optionnellement, un deuxième flacon réactionnel pour faire réagir la biomolécule avec le complexe chélateur-carbonyle obtenu à la première étape de la réaction.

22. Procédé de préparation de biomolécules radiomarquées comprenant :
a) la mise en contact d'un agent chélateur tel que défini à la revendication 1, avec une biomolécule pour obtenir un chélateur-biomolécule ;
et
b) la mise en contact du chélateur-biomolécule avec un fragment carbonyle de formule [M(CO)₃]⁺, où M est le rhénium (Re) ou le technétium (Tc), dans des conditions permettant de former une biomolécule radiomarquée.

23. Kit approprié pour réaliser le procédé tel que défini à la revendication 22, comprenant un premier flacon contenant l'agent chélateur tel que défini à la revendication 1, optionnellement, un premier flacon réactionnel pour faire réagir l'agent chélateur avec la biomolécule, un deuxième flacon contenant le fragment carbonyle de formule [M(CO)₃]⁺, où M est Re ou Tc, et optionnellement, un deuxième flacon réactionnel pour faire réagir le chélateur-biomolécule obtenu à la première étape de la réaction avec le carbonyle.

24. Agent chélateur selon la revendication 1 ou la revendication 17, pour son utilisation dans la préparation d'un agent de diagnostic ou d'un agent thérapeutique pour diagnostiquer ou traiter des tumeurs.

25. Agent chélateur selon la revendication 18, pour son utilisation comme agent de diagnostic ou agent thérapeutique pour diagnostiquer ou traiter des tumeurs.

26. Utilisation d'un agent chélateur selon la revendication 1 ou la revendication 17, pour la préparation d'un agent de diagnostic ou d'un agent thérapeutique pour diagnostiquer ou traiter des tumeurs.

27. Utilisation d'un agent chélateur selon la revendication 18, pour la préparation d'un agent de diagnostic ou d'un agent thérapeutique pour diagnostiquer ou traiter des tumeurs.
